# FASCICULE DE BREVET EUROPEEN

(11) **EP 0 662 134 B1**
(45) Date de publication et mention de la délivrance du brevet: **28.12.2005**
(21) Numéro de dépôt: 93920919.3
(22) Date de dépôt: 25.09.1993
(51) Int. Cl.: C12N 15/52, C12N 15/76, C12N 15/54, C12N 1/21, C12N 9/10, C12N 9/00, C12N 15/53, C12N 9/06, C12P 21/04, C12P 17/18, C07K 7/06, C07K 5/06

(54) **POLYPEPTIDES IMPLIQUES DANS LA BIOSYNTHESE DES STREPTOGRAMINES, SEQUENCES NUCLEOTIDIQUES CODANT POUR CES POLYPEPTIDES ET LEUR UTILISATION**
AN DER STREPTOGRAMIN BIOSYNTHESE BETEILIGTE POLYPEPTIDE, IHRE KODIERENDEN NUKLEOTIDSEQUENZEN UND IHRE VERWENDUNG
POLYPEPTIDES INVOLVED IN STREPTOGRAMIN BIOSYNTHESIS, NUCLEOTIDE SEQUENCES CODING FOR SAID POLYPEPTIDES AND USE THEREOF

(30) Priorité: 25.09.1992 FR 9211441
(43) Date de publication de la demande: 12.07.1995
(73) Titulaire: Aventis Pharma S.A., 92160 Antony (FR)
(72) Inventeur: BLANC, Véronique, F-75012 Paris (FR); BLANCHE, Francis, F-75019 Paris (FR); CROUZET, Joel, F-75012 Paris (FR); JACQUES, Nathalie, F-75015 Paris (FR); LACROIX, Patricia, F-94360 Bry-sur-Marne (FR); THIBAUT, Denis, F-75013 Paris (FR); ZAGOREC, Monique, F-75002 Paris (FR); Crecy-Lagarde, Valérie de, F-78490 Grosrouvre (FR); Debussche, Laurent, F-91200 Athis-Mons (FR)
(74) Mandataire: Le Pennec, Magali
(86) Numéro de dépôt international: PCT/FR1993/000923
(87) Numéro de publication internationale: WO 1994/008014

(56) Documents cités:
- BIOTECHNOLOGY vol. 8 , Février 1990 , NEW YORK US pages 115 - 121 KEITH F. CHATER ET AL 'The improving prospects for yield increase by genetic engineering in antibiotic-producing Streptomyces'
- GENE. vol. 74 , 1988 , AMSTERDAM NL pages 305 - 320 S. E. HALLAM ET AL 'Nucleotide sequence , transcription and deduced function of a gene involved in polyketide antibiotic synthesis in Streptomyces coelicolor' cité dans la demande
- CHEMICAL ABSTRACTS, vol. 115, no. 23, 9 Décembre 1991, Columbus, Ohio, US; abstract no. 251978n, FUNANE , KAZUMI ET AL 'Isolation and properties of IMfactor (an inducer of secondary metabolite production)deficient mutants of Streptomyces virgineae MAFF 10-06014' page 453 ;colonne R ; & SHOKUHIN SOGO KENKYU HOKOKU vol. 55 , 1991 pages 37 - 44
- DRUGS AND THE PHARMACEUTICAL SCIENCES vol. 22 , 1984 pages 695 - 720 A. M. BIOT 'Virginiamycin : properties , biosynthesis and fermentation'
- BIOTECHNOLOGY LETTERS vol. 14, no. 11 , Novembre 1992 pages 1065 - 1070 V. PAQUET ET AL 'Induction of pristinamycins production in Streptomyces pristinaespiralis'
- DATABASE WPI Section Ch, Week 8420, 4 Avril 1984 Derwent Publications Ltd., London, GB; Class B02, AN 84-123313 'Selective production of neoviridogrisein II from the antibiotic producing microorganism prolyl-hydroxylase activity-defect variant' & JP,A,59 059 198 (SANRAKU OCEAN) 4 Avril 1984
- FOLIA MICROBIOLOGICA vol. 35, no. 6 , 1990 , PRAGUE, TCHECOSLOVAQUIE. page 494 M. BLUMAUEROVA ET AL 'Physiological and genetic aspects of Virginiamycin production'
- BIOTECHNOLOGY AND BIOINDUSTRY vol. 2 , 1988 pages 20 - 22 V.PRYKRYLOVA ET AL 'Strain developement in Streptomyces virginiae, a producer of Virginiamycin'

### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION : POLYPEPTIDES IMPLIQUES DANS LA BIOSYNTHESE DES STREPTOGRAMINES, SEQUENCES NUCLEOTIDIQUES CODANT POUR CES POLYPEPTIDES ET UTILISATIONS.
   (iii) NOMBRE DE SEQUENCES: 16
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1 :
   (i) CARACTERISTIQUES DE LA SEQUENCE:
      (A) LONGUEUR: 5392 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 1:
   GGATCCTGGC GTCCGCCGTC AAGAACTGAA CCGAGGAGAC ACCCACCATG ACCGCACCCC   60
   GCCGGCGCAT CACCCTCGCC GGCATCATCG ACGGCCCCGG CGGCCATGTG GCCGCCTGGC  120
   GCCACCCGGC GACCAAGGCG GACGCCCAGC TCGACTTCGA ATTCCACCGC GACAACGCCC  180
   GCACCCTCGA ACGCGGCCTG TTCGACGCCG TGTTCATCGC GGACATCGTC GCCGTGTGGG  240
   GCACCCGCCT GGACTCCCTG TGCCGCACCT CGCGCACCGA GCACTTCGAA CCGCTCACCC  300
   TGCTCGCCGC CTACGCCGCG GTCACCGAGC ACATCGGCCT GTGCGCCACC GCCACCACCA  360
   CGTACAACGA ACCGGCGCAC ATCGCCGCCC GCTTCGCCTC CCTCGACCAC CTCAGCGGCG  420
   GCCGGGCCGG CTGGAACGTC GTCACCTCCG CCGCACCGTG GGAGTCCGCC AACTTCGGCT  480
   TCCCCGAGCA CCTGGAGCAC GGCAAACGCT ACGAGCGGGC CGAGGAGTTC ATCGACGTCG  540
   TCAAAAAACT GTGGGACAGC GACGGCCGCC CCGTCGACCA CCGCGGCACC CACTTCGAGG 600
   CCCCCGGCCC GCTCGGGATC GCCCGCCCCC CGCAGGGCCG CCCCGTCATC ATCCAGGCCG 660
   GCTCCTCGCC GGTGGGACGC GAGTTCGCCG CCCGGCACGC CGAGGTCATC TTCACCCGGC 720
   ACAACCGGCT CTCCGACGCC CAGGACTTCT ACGGCGACCT CAAGGCACGC GTCGCCCGGC 780
   ACGGCCGCGA CCCCGAGAAG GTCCTCGTGT GGCCGACCCT CGCGCCGATC GTCGCCGCCA 840
   CCGACACCGA GGCGAAGCAG CGCCTGCAGG AACTGCAGGA CCTCACCCAC GACCATGTCG 900
   CCCTGCGCAC CCTTCAGGAC CACCTCGGCG ACGTCGACCT GAGCGCGTAC CCGATCGACG 960
   GGCCCGTCCC CGACATCCCG TACACCAACC AGTCCCAGTC GACGACCGAG CGGCTGATCG 1020
   GCCTGGCCAG GCGCGAGAAC CTCAGCATCC GCGAGCTGGC CCTGCGGCTG ATGGGCGACA 1080
   TCGTCGTCGG CACACCGGAG CAGCTCGCCG ACCACATGGA GAGCTGGTTC ACCGGCCGCG 1140
   GCGCCGACGG CTTCAACATC GACTTCCCGT ACCTGCCGGG CTCCGCCGAC GACTTCGTCG 1200
   ACCACGTGGT GCCCGAACTG CAGCGCCGCG GCCTGTACCG CTCGGGCTAC GAGGGCACCA 1260
   CCCTGCGGGC CAACCTCGGC ATCGACGCCC CCCGGAAGGC AGGTGCAGCG GCTTGACTTC 1320
   CGTCCTAAAG GCGGGGGATT CCAGCGGTCG CCCGCTGGGG TTCCTGCTTC ACCGACGACC 1380
   GCCCCGTCCG GGAGGACTCC CGTTGAGGTC TTATACCGTC TCCACAGGCC GACGCCGCCA 1440
   GCCCGGCGGC CAGGATGTTG CGTGCCGCAT TCACGTCGCG GTCATGCACA GCGCCGCAGT 1500
   CGCACGTCCA CTCCCGGACG TTCAGCGGCA GCTTCCCGCG GACCGTGCCG CAGGTTCCGC 1560
   ACAGCTTGGA GCTGGGGAAC CAGCGGTCGA TCACGACGAG TTCGCGCCCA TACCAGGCGC 1620
   ACTTGTACTC CAGCATGGAG CGCAGTTCCG TCCAGGCCGC GTCGGAGATG GCGCGCGCGA 1680
   GCTTGCCGTT CTTCAGCAGG TTGCGGACGG TGAGGTCCTC GATCACGACC GTTTGGTTCT 1740
   CACGGACGAG TCGAGTCGAC AGCTTGTGGA GGAAGTCGCA GCGCCGGTCG GTGATCCGGG 1800
   CGTGGACGCG GGCGACCTTG CGGCGGGCTT TCTTCCGGTT CGCCGACCCC TTCGCCTTGC 1860
   GCGACACGTC CCGCTGAGCC TTCGCGAGGC GGGCGCGGTC ACGGCGCTCG TGCTTGGGGT 1920
   TGGTGATCTT CTCCCCGGTG GACAGGGTCA CCAGGGAGGT GATCCCGGCG TCGATGCCGA 1980
   CGGCCGCCGT GGTGGCGGGC GCGGGGGTGA TGGTGTCCTC GCACAGCAGG GACACGAACC 2040
   AGCGGCCCGC ACGGTCGCGG GACACGGTCA CCGTCGTCGG CTCCGCCCCT TCGGGAAGGG 2100
   GACGGGACCA GCGGATGTCC AGGGGCTCCG CGGTCTTCGC CAGCGTGAGC TGTCCGTTAC 2160
   GCCACGTGAA GGCGCTGCGG GTGTACTCGG CCGACGCCCT GGACTTTTTC CGCGACTTGT 2220
   ACCGCGGGTA CTTCGACCGC TTGGCGAAGA AGTTGGCGAA CGCCGTCTGC AAGTGCCGCA 2280
   GCGCCTGCTG GAGCGGGACG GAGGACACCT CCGAGAGGAA GGCGAGTTCT TCGGTCTTCT 2340
   TCCACTCCGT CAGCGCGGCG GACGACTGCA CGTAGGAGAC CCGGCGCTGC TCGCCGTACC 2400
   AGGCTCGCGT GCGCCCCTCA AGCGCCTTGT TGTACACGAG GCGGACACAG CCGAACGTGC 2460
   GGGACAGCTC AGCCGCCTGC TCGTCCGTGG GATAAAAGCG GTACTTGAAA GCCCGCTTGA 2520
   CCTGCTGCAT CACGCCTCAC ACGCTATCAG TTCCCGTGTG AGCGGCGGGT GTCTGCCGGT 2580
   GGTTGCAGAC GCCGAACCGC CCTGGCGGCG ATTCGCCCAT CCCTGCCCTG CTCCGCAAGA 2640
   GCTTCGTCTC CTCCCCGGTC TGAAGGCCGG GGTATCCACG AAGGAATTCT GATGACCGCG 2700
   CCCATCCTCG TCGCCACCCT CGACACCCGC GGCCCCGCCG CCACCCTCGG CACGATCACC 2760
   CGCGCCGTGC GGGCCGCGGA GGCCGCCGGA TTCGACGCCG TCCTGATCGA CGACCGGGCC 2820
   GCCGCCGGCG TCCAGGGCCG GTTCGAGACG ACGACGCTGA CCGCCGCGCT GGCCGCCGTC 2880
   ACCGAGCACA TCGGCCTGAT CACCGCCCCG CTCCCGGCCG ACCAGGCCCC CTACCACGTG 2940
   TCCCGGATCA CCGCCTCGCT CGACCACCTC GCCCACGGCC GCACCGGCTG GCTCGCGAGC 3000
   ACGGACACCA CCGACCCCGA GGGCCGCACC GGCGAACTCA TCGACGTCGT CCGCGGCCTG 3060
   TGGGACAGCT TCGACGACGA CGCCTTCGTC CACGACCGCG CCGACGGCCT GTACTGGCGG 3120
   CTGCCCGCCG TCCACCAACT CGACCACCAG GGCAGGCACT TCGACGTGGC CGGCCCCCTC 3180
   AACGTCGCCC GCCCGCCGCA GGGCCACCCC GTCGTCGCCG TCACCGGCCC CGCCCTCGCC 3240
   GCGGCCGCCG ACCTCGTCCT GCTCGACGAG GCGGCCGACG CCGCCTCGGT GAAGCAGCAG 3300
   GCACCGCACG CCAAGATCCT CCTGCCGCTG CCCGGCCCGG CCGCCGAACT GCCCGCCGAC 3360
   AGCCCCGCGG ACGGCTTCAC GGTGGCGCTC ACCGGCTCCG ACGACCCGGT CCTGGCCGCG 3420
   CTCGCCGCCC GGCCCGGCCG CCCGGACCGC ACCGCGGCCA CCACCCTGCG CGAACGCCTG 3480
   GGCCTGGCCC GCCCCGAGAG CCGCCACGCC CTCACCACCG CCTGACGACC CGTCCGCCCG 3540
   CTGCTTCCTG GAGAGTCATG TCCCGTCGCC TGTTCACCTC GGAGTCCGTG ACCGAGGGCC 3600
   ACCCCGACAA GATCGCCGAC CAGATCAGTG ACACCGTCCT CGACGCCCTG CTGCGCGAGG 3660
   ACCCCGCCTC ACGCGTCGCG GTCGAGACCC TGATCACCAC CGGCCAGGTC CACATCGCCG 3720
   GCGAGGTCAC CACCAAGGCG TACGCGCCCA TCGCCCAACT GGTCCGCGAC ACGATCCTGG 3780
   CCATCGGCTA CGACTCGTCC GCCAAGGGCT TCGACGGCGC CTCCTGCGGC GTCTCCGTCT 3840
   CCATCGGCGC GCAGTCCCCG GACATCGCCC AGGGCGTCGA CAGCGCCTAC GAGACCCGCG 3900
   TCGAGGGCGA GGACGACGAG CTCGACCAGC AGGGCGCCGG CGACCAGGGC CTGATGTTCG 3960
   GCTACGCCAC CGACGAGACC CCCTCGCTGA TGCCGCTGCC CATCGAGCTC GCCCACCGCC 4020
   TCTCGCGCCG GCTCACCGAG GTCCGCAAGG ACGGCACCGT CCCCTACCTG CGCCCCGACG 4080
   GCAAGACCCA GGTCACCATC GAGTACCAGG GCAGCCGCCC GGTGCGCCTG GACACCGTCG 4140
   TCGTCTCCTC CCAGCACGCC GCCGACATCG ACCTCGGCTC CCTGCTCACC CCCGACATCC 4200
   GCGAGCACGT CGTCGAGCAC GTCCTCGCCG CACTCGCCGA GGACGGCATC AAGCTCGAGA 4260
   CGGACAACTA CCGCCTGCTG GTCAACCCGA CCGGCCGTTT CGAGATCGGC GGCCCGATGG 4320
   GCGACGCCGG CCTGACCGGC CGCAAGATCA TCATCGACAC GTACGGCGGC ATGGCCCGCC   4380
   ACGGCGGTGG CGCGTTCTCC GGCAAGGACC CGTCCAAGGT CGACCGTTCC GCCGCGTACG   4440
   CGATGCGCTG GGTCGCCAAG AACGTCGTCG CCGCGGGCCT CGCCTCCCGC TGCGAGGTCC   4500
   AGGTCGCCTA CGCCATCGGC AAGGCCGAGC CGGTCGGCCT GTTCGTCGAG ACGTTCGGCA   4560
   CCGGCACCGT CGCCCAGGAG CGCATCGAGA AGGCCATCAC CGAGGTCTTC GACCTGCGCC   4620
   CCGCGGCCAT CATCCGCGAC CTCGACCTGC TGCGGCCCAT CTACGCCGCC ACCGCCGCCT   4680
   ACGGCCACTT CGGCCGCGAA CTGCCCGACT TCACCTGGGA GCGGACCGAC CGCGCCCACC   4740
   GGCTCAAGGC CGCGGCCGGT CTCTGAGCCG GCCGGACCTG TGAGGAGACC TGACGTGCGC   4800
   ATCGCTGTCA CCGGTTCCAT CGCCACCGAC CATCTGATGG TCTTCCCCGG CCGGTTCGCG   4860
   GATCAGCTGA TCCCCGACCA GCTCGCTCAT GTCTCGCTCT CCTTCCTGGT CGACGCACTC   4920
   GAGGTGCGCC GGGGCGGAGT GGCGGACAAC GTCGCCTTCG GCCTCGGCGG CCTCGGCCTC   4980
   ACCCCCCAGC TGGTCGGCGC CGTGGGCAGC GACTTCGCCG AGTACGAGGT CTGGCTCAAG   5040
   GAACACGGCG TCGACACCGG CCCCGTCCTG GTCTCCACCG AGCGGCAGAC CGCCCGGTTC   5100
   ATGTGCATCA CCGACCAGGA CTCCAACCAG ATCGCCTCCT TCTACGCGGG CGCCATGCAA   5160
   GAGGCCCGCG ACATCGACCT GTGGCACCTG ACCACCGGCA GCGTCCGCCC CGACCTCGTC   5220
   CTGGTCTGCC CGAACGACCC GGCGGCGATG CTGCGCCACA CGGGGAGTGC CGCGAAACTG   5280
   GGCCTGCCGT TCGCCGCCGA CCCCTCCCAG CAGCTCGCCC GCCTGGAGGG AGGGAGGTAC   5340
   GCGAACTCGG TCGACGGGGC CCGTTGGTTT TTCACCAACG AAGTACGAGG CC           5392
(2) INFORMATION POUR LA SEQ ID NO: 2:
   (i) CARACTERISTIQUES DE LA SEQUENCE:
      (A) LONGUEUR: 1269 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (ix) CARACTERISTIQUE ADDITIONELLE:
      (A) NOM/CLE: CDS
      (B) EMPLACEMENT: 1..1269
   (xi) DESCRIPTION DE LA SEQUENCE: SEQ ID NO: 2:
(2) INFORMATION POUR LA SEQ ID NO: 3:
   (i) CARACTERISTIQUES DE LA SEQUENCE:
      (A) LONGUEUR: 834 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (ix) CARACTERISTIQUE ADDITIONELLE:
      (A) NOM/CLE: CDS
      (B) EMPLACEMENT: 1..834
   (xi) DESCRIPTION DE LA SEQUENCE: SEQ ID NO: 3:
(2) INFORMATION POUR LA SEQ ID NO: 4:
   (i) CARACTERISTIQUES DE LA SEQUENCE:
      (A) LONGUEUR: 1209 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (ix) CARACTERISTIQUE ADDITIONELLE:
      (A) NOM/CLE: CDS
      (B) EMPLACEMENT: 1..1209
   (xi) DESCRIPTION DE LA SEQUENCE: SEQ ID NO: 4:
(2) INFORMATION POUR LA SEQ ID NO: 5:
   (i) CARACTERISTIQUES DE LA SEQUENCE:
      (A) LONGUEUR: 1879 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (ix) CARACTERISTIQUE ADDITIONELLE:
      (A) NOM/CLE: CDS
      (B) EMPLACEMENT: 110..1858
   (xi) DESCRIPTION DE LA SEQUENCE: SEQ ID NO: 5:
   GATCGGCTCC TGACGGAGCG GCGGCGCGCG GGCGCGGCGC ATCAGCGGCG TGTCAACGGC    60
   GCTGCCGACA CTGGGCGCGA CGCGAGGACG AAGCCGGAAA GGACCAACG ATG CTG       115
                                                         Met Leu
                                                           1
   GAC GGA TGC GTT CCC TGG CCC GAG GAT GTG GCC GCG AAG TAC CGG GCG     163
   Asp Gly Cys Val Pro Trp Pro Glu Asp Val Ala Ala Lys Tyr Arg Ala
             5                  10                  15
   GCC GGC TAC TGG CGG GGC GAG CCG CTG GGC ATG CTG CTG GGC CGC TGG     211
   Ala Gly Tyr Trp Arg Gly Glu Pro Leu Gly Met Leu Leu Gly Arg Trp
        20                  25                  30
   GCG GAG CAG TAC GGC GAG CGG GAG GCG CTG GTC GGC GCG GAC GGG TGC     259
   Ala Glu Gln Tyr Gly Glu Arg Glu Ala Leu Val Gly Ala Asp Gly Cys
    35                  40                  45                  50
   TCC CGT GTC ACC TAC CGT GCC CTG GAC CGC TGG TGC GAC CGG CTG GCG     307
   Ser Arg Val Thr Tyr Arg Ala Leu Asp Arg Trp Cys Asp Arg Leu Ala
                    55                  60                  65
   GCG GGG TTC GCG GCG CGC GGG ATC GGC GCC GGC GAG CGG GTG CTG GTG     355
   Ala Gly Phe Ala Ala Arg Gly Ile Gly Ala Gly Glu Arg Val Leu Val
                   70                  75                  80
   CAG CTG CCG AAC ACG CCC GAG TTC GTC GCG GTG TGC TTC GCG CTG TTC     403
   Gln Leu Pro Asn Thr Pro Glu Phe Val Ala Val Cys Phe Ala Leu Phe
           85                  90                  95
   CGT CTG GGC GCG CTG CCG GTG TTC GCG CTG CCC GCG CAC CGT GCC GCC     451
   Arg Leu Gly Ala Leu Pro Val Phe Ala Leu Pro Ala His Arg Ala Ala
       100                 105                 110
   GAG GTG GGG CAC CTG CTC GAG CTG TCC GGC GCC GTC GCC CAC ATC CTG     499
   Glu Val Gly His Leu Leu Glu Leu Ser Gly Ala Val Ala His Ile Leu
   115                 120                 125                 130
   CCG GGC ACC GGC ACC GGC TAC GAC CAT GTC GCG GCG GCC GTG GAG GCC     547
   Pro Gly Thr Gly Thr Gly Tyr Asp His Val Ala Ala Ala Val Glu Ala
                   135                 140                 145
   CGT GCC CGC CGT GCC CGC CCG GTG CAG GTG TTC GTG GCG GGC GAG GCG     595
   Arg Ala Arg Arg Ala Arg Pro Val Gln Val Phe Val Ala Gly Glu Ala
               150                 155                 160
   CCC GCG GTG CTG CCC GAG GGG TTC ACC GCG CTG GCC GAC GTG GAC GGC     643
   Pro Ala Val Leu Pro Glu Gly Phe Thr Ala Leu Ala Asp Val Asp Gly
           165                 170                 175
   GAC CCG GTG GCG CCG GCG GAC GTG GAC GCC TTC CGA CGT GGC GTC TTC     691
   Asp Pro Val Ala Pro Ala Asp Val Asp Ala Phe Arg Arg Gly Val Phe
       180                 185                 190
   CTG CTG TCC GGG GGG ACG ACC GCG CTG CCG AAG CTG ATC CCG CGC ACC     739
   Leu Leu Ser Gly Gly Thr Thr Ala Leu Pro Lys Leu Ile Pro Arg Thr
   195                 200                 205                 210
   CAC GAC GAC TAC GCC TAC CAG TGC CGG GTC ACG GCC GGT ATC TGC GGC     787
   His Asp Asp Tyr Ala Tyr Gln Cys Arg Val Thr Ala Gly Ile Cys Gly
                   215                 220                 225
   CTG GAC GCG GAC AGT GTC TAT CTG GCG GTG CTG CCG GCC GAG TTC AAC     835
   Leu Asp Ala Asp Ser Val Tyr Leu Ala Val Leu Pro Ala Glu Phe Asn
               230                 235                 240
   TTC CCC TTC GGC TGC CCG GGC ATC CTG GGC ACC CTG CAC GCC GGC GGG     883
   Phe Pro Phe Gly Cys Pro Gly Ile Leu Gly Thr Leu His Ala Gly Gly
           245                 250                 255
   CGG GTG GTG TTC GCG CTG TCA CCG CAG CCC GAG GAG TGC TTC GCG CTG     931
   Arg Val Val Phe Ala Leu Ser Pro Gln Pro Glu Glu Cys Phe Ala Leu
       260                 265                 270
   ATC GAA CGC GAA CAC GTC ACC TTC ACC TCC GTC ATC CCC ACG ATC GTG     979
   Ile Glu Arg Glu His Val Thr Phe Thr Ser Val Ile Pro Thr Ile Val
   275                 280                 285                 290
   CAC CTG TGG CTG GCG GCC GCC GCA CAA GGC CAC GGC CGC GAC CTG GGC    1027
   His Leu Trp Leu Ala Ala Ala Ala Gln Gly His Gly Arg Asp Leu Gly
                   295                 300                 305
   AGC CTT CAG CTG CTG CAG GTC GGC AGC GCC AAA CTC CAC GAG GAG CTC    1075
   Ser Leu Gln Leu Leu Gln Val Gly Ser Ala Lys Leu His Glu Glu Leu
                   310                      315                      320
   GCC GCC CGG ATC GGC CCC GAA CTG GGG GTG CGG CTG CAG CAG GTG TTC    1123
   Ala Ala Arg Ile Gly Pro Glu Leu Gly Val Arg Leu Gln Gln Val Phe
               325                      330                      335
   GGC ATG GCC GAG GGA CTG CTG ACC TTC ACC CGC GAC GAC GAC CCG GCG    1171
   Gly Met Ala Glu Gly Leu Leu Thr Phe Thr Arg Asp Asp Asp Pro Ala
           340                      345                      350
   GAC GTG GTG CTG CGC ACC CAG GGC CGG CCG GTG TCC GAG GCC GAC GAG    1219
   Asp Val Val Leu Arg Thr Gln Gly Arg Pro Val Ser Glu Ala Asp Glu
   355                      360                      365                 370
   ATA CGC GTC GCC GAC CCC GAC GGC CGG CCC GTG CCC CGC GGT GAG ACC    1267
   Ile Arg Val Ala Asp Pro Asp Gly Arg Pro Val Pro Arg Gly Glu Thr
                   375                      380                      385
   GGT GAA CTG CTC ACC CGC GGC CCC TAC ACG CTG CGC GGC TAC TAC CGG    1315
   Gly Glu Leu Leu Thr Arg Gly Pro Tyr Thr Leu Arg Gly Tyr Tyr Arg
               390                      395                      400
   GCC CCC GAG CAC AAC GCC CGC GCG TTC ACC GAG GAC GGC TTC TAC CGC    1363
   Ala Pro Glu His Asn Ala Arg Ala Phe Thr Glu Asp Gly Phe Tyr Arg
               405                      410                      415
   AGC GGC GAT CTG GTG CGG CTC ACC GCC GAC GGG CAG TTG GTG GTG GAG    1411
   Ser Gly Asp Leu Val Arg Leu Thr Ala Asp Gly Gln Leu Val Val Glu
           420                      425                      430
   GGC AGG ATC AAG GAC GTC GTC ATC CGC GGC GGC GAC AAG GTC TCC GCG    1459
   Gly Arg Ile Lys Asp Val Val Ile Arg Gly Gly Asp Lys Val Ser Ala
   435                      440                      445                 450
   ACC GAG GTC GAG GGC CAC CTG GGC GCC CAC CCC GAC GTC CAG CAG GCC    1507
   Thr Glu Val Glu Gly His Leu Gly Ala His Pro Asp Val Gln Gln Ala
                   455                      460                      465
   GCC GTC GTC GCC ATG CCC GAC CCG GTG TGG GGC GAG AAG GTC TGC GCC    1555
   Ala Val Val Ala Met Pro Asp Pro Val Trp Gly Glu Lys Val Cys Ala
               470                      475                      480
   TAC ATC GTG CCC GCA CCC GGC CGT CCC GCA CCG CCG ATG GCG GCG CTG    1603
   Tyr Ile Val Pro Ala Pro Gly Arg Pro Ala Pro Pro Met Ala Ala Leu
           485                      490                      495
   CGC CGG CTG CTG CGC GCG CGG GGA CTG GCC GAC TAC AAG CTT CCC GAC    1651
   Arg Arg Leu Leu Arg Ala Arg Gly Leu Ala Asp Tyr Lys Leu Pro Asp
           500                      505                      510
   CGG GTG GAG GTC GTC GAC GCG TTC CCG CTG ACC GGC CTC AAC AAG GTC    1699
   Arg Val Glu Val Val Asp Ala Phe Pro Leu Thr Gly Leu Asn Lys Val
   515                      520                      525                 530
   GAC AAG AAG GCC CTG GCG GCC GAC ATC GCC GCC AAG ACC GCC CCC ACC    1747
   Asp Lys Lys Ala Leu Ala Ala Asp Ile Ala Ala Lys Thr Ala Pro Thr
                   535                      540                      545
   CGC CCC ACC ACC GCC GGC CAC GGC CCG ACC ACG GAC GGC GAT ACG GCC    1795
   Arg Pro Thr Thr Ala Gly His Gly Pro Thr Thr Asp Gly Asp Thr Ala
               550                      555                      560
   GGT GGG GGT GGG TCC GCG GGC GGG GTG ACG GCC GCC GGT GGC GGG CGG    1843
   Gly Gly Gly Gly Ser Ala Gly Gly Val Thr Ala Ala Gly Gly Gly Arg
           565                 570                 575
   GAG GAG GCG GCG TGAGCGGGCC CGGGCCCGAG GGCG                    1879
   Glu Glu Ala Ala
       580
(2) INFORMATION POUR LA SEQ ID NO: 6:
   (i) CARACTERISTIQUES DE LA SEQUENCE:
      (A) LONGUEUR: 1833 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (ix) CARACTERISTIQUE ADDITIONELLE:
      (A) NOM/CLE: CDS
      (B) EMPLACEMENT: 103..1689
   (xi) DESCRIPTION DE LA SEQUENCE: SEQ ID NO: 6:
   GGATCCCCTC GCCCAGGGCC CTGGCGGGCC CGCCGGGCCG TGGGGGAGGT GCGGGGGCCG    60
   CGGGCCCCGG CACCGCACGA ACAGAACAAC CGCTCCGGGC CC ATG CGG ACT TCA      114
                                                 Met Arg Thr Ser
                                                   1
   CGG TCC CAC GAC CAG CGG GCC CCT ACC CCC TGG AGA CAT CCC TTG CAC     162
   Arg Ser His Asp Gln Arg Ala Pro Thr Pro Trp Arg His Pro Leu His
     5                  10                  15                  20
   AGC ACC CGG CCC GCG CCC GCG GCC GAC CGT GAC CCC AGG CGC TGG GTC     210
   Ser Thr Arg Pro Ala Pro Ala Ala Asp Arg Asp Pro Arg Arg Trp Val
                    25                  30                  35
   ATC CTC GGC GTG ATC TGC CTG GCC CAA CTC GTC GTC CTG CTC GAC AAC     258
   Ile Leu Gly Val Ile Cys Leu Ala Gln Leu Val Val Leu Leu Asp Asn
                40                  45                  50
   ACC GTC CTC AAC GTC GCC ATC CCG GTG CTC ACC ACC GAC CTG GGC GCC     306
   Thr Val Leu Asn Val Ala Ile Pro Val Leu Thr Thr Asp Leu Gly Ala
            55                  60                  65
   AGC ACC GCC GAC ATC CAG TGG ATG ATC AAC GCC TAC GCG CTC GTG CAG     354
   Ser Thr Ala Asp Ile Gln Trp Met Ile Asn Ala Tyr Ala Leu Val Gln
        70                  75                  80
   TCC GGG CTG CTG CTC ACC GCG GGC AGC CTC GCG GAC CGC TAC GGC CGC     402
   Ser Gly Leu Leu Leu Thr Ala Gly Ser Leu Ala Asp Arg Tyr Gly Arg
    85                  90                  95                 100
   AAA CGG CTG CTG ATG CTC GGA CTG GTG CTC TTC GGC GCC GGG TCC GCC     450
   Lys Arg Leu Leu Met Leu Gly Leu Val Leu Phe Gly Ala Gly Ser Ala
                   105                 110                 115
   TGG GCG GCC TTC GCC CAG GAC TCC GCC CAA CTC ATC GCC GCC CGG GCC      498
   Trp Ala Ala Phe Ala Gln Asp Ser Ala Gln Leu Ile Ala Ala Arg Ala
               120                 125                 130
   GGC ATG GGC GTG GGC GGG GCG CTG CTG GCG ACC ACC ACC CTC GCC GTC      546
   Gly Met Gly Val Gly Gly Ala Leu Leu Ala Thr Thr Thr Leu Ala Val
           135                 140                 145
   ATC ATG CAG GTC TTC GAC GAC GAC GAA CGC CCC CGG GCG ATC GGC CTG      594
   Ile Met Gln Val Phe Asp Asp Asp Glu Arg Pro Arg Ala Ile Gly Leu
       150                 155                 160
   TGG GGA GCG GCC AGC TCA CTG GGC TTC GCG GCC GGC CCG CTG CTC GGC      642
   Trp Gly Ala Ala Ser Ser Leu Gly Phe Ala Ala Gly Pro Leu Leu Gly
   165                 170                 175                 180
   GGC GCC CTC CTC GAC CAC TTC TGG TGG GGC TCC ATC TTC CTG ATC AAC      690
   Gly Ala Leu Leu Asp His Phe Trp Trp Gly Ser Ile Phe Leu Ile Asn
                   185                 190                 195
   CTG CCC GTC GCG CTG CTG GGC CTG CTG GCC GTC GCC CGC CTG GTG CCC      738
   Leu Pro Val Ala Leu Leu Gly Leu Leu Ala Val Ala Arg Leu Val Pro
               200                 205                 210
   GAG ACG AAG AAC CCC GAA GGC CGG CGC CCC GAC CTG CTC GGC GCC GTG      786
   Glu Thr Lys Asn Pro Glu Gly Arg Arg Pro Asp Leu Leu Gly Ala Val
           215                 220                 225
   CTC TCC ACC CTC GGC ATG GTC GGC GTC GTC TAC GCC ATC ATC TCC GGC      834
   Leu Ser Thr Leu Gly Met Val Gly Val Val Tyr Ala Ile Ile Ser Gly
       230                 235                 240
   CCC GAA CAC GGC TGG ACG GCC CCG CAG GTC CTC CTG CCG GCC GCC GTC      882
   Pro Glu His Gly Trp Thr Ala Pro Gln Val Leu Leu Pro Ala Ala Val
   245                 250                 255                 260
   GCG GCC GCC GCG CTC ACC GCG TTC GTC CGC TGG GAA CTG CAC ACC CCC      930
   Ala Ala Ala Ala Leu Thr Ala Phe Val Arg Trp Glu Leu His Thr Pro
                   265                 270                 275
   CAC CCC ATG CTC GAC ATG GGC TTC TTC ACC GAC CGG CGC TTC AAC GGG      978
   His Pro Met Leu Asp Met Gly Phe Phe Thr Asp Arg Arg Phe Asn Gly
               280                 285                 290
   CCG TCG CCG GCG GAG TGC TCG TCG TTC GGC ATG GCC GGC TCG CTC TTC     1026
   Pro Ser Pro Ala Glu Cys Ser Ser Phe Gly Met Ala Gly Ser Leu Phe
           295                 300                 305
   CTG CTC ACC CAG CAC CTC CAA CTC GTC CTC GGC TAC GAC GCC CTG CAG     1074
   Leu Leu Thr Gln His Leu Gln Leu Val Leu Gly Tyr Asp Ala Leu Gln
       310                 315                 320
   GCC GGC CTG CGC ACC GCG CCA CTG GCT TTG ACG ATC GTC GCC CTC AAC     1122
   Ala Gly Leu Arg Thr Ala Pro Leu Ala Leu Thr Ile Val Ala Leu Asn
   325                 330                 335                 340
   CTG GCC GGC CTC GGC GCG AAA CTC CTC GCC GCG CTC GGC ACC GCC CGC     1170
   Leu Ala Gly Leu Gly Ala Lys Leu Leu Ala Ala Leu Gly Thr Ala Arg
                   345                 350                 355
   AGC ATC GCC CTG GGC ATG ACA CTG CTG GCC GCC GGC CTC AGC GCG GTG   1218
   Ser Ile Ala Leu Gly Met Thr Leu Leu Ala Ala Gly Leu Ser Ala Val
               360                 365                 370
   GCC GTC GGC GGA TCG GGC CCC GAC GCC GGC TAC GGC GGC ATG CTC GCC   1266
   Ala Val Gly Gly Ser Gly Pro Asp Ala Gly Tyr Gly Gly Met Leu Ala
           375                 380                 385
   GGC CTG CTC CTC ATG GGC GCG GGC ATC GCA CTG GCC ATG CCC GCC ATG   1314
   Gly Leu Leu Leu Met Gly Ala Gly Ile Ala Leu Ala Met Pro Ala Met
       390                 395                 400
   GCC ACC GCC GTG ATG TCC TCC ATC CCG CCC GCC AAG GCC GGG GCC GGA   1362
   Ala Thr Ala Val Met Ser Ser Ile Pro Pro Ala Lys Ala Gly Ala Gly
   405                 410                 415                 420
   GCG GGC GTG CAG GGC ACC CTG ACC GAG TTC GGC GGC GGA CTG GGA GTG   1410
   Ala Gly Val Gln Gly Thr Leu Thr Glu Phe Gly Gly Gly Leu Gly Val
                   425                 430                 435
   GCG ATC CTC GGC GCC GTC CTC GGC TCC CGC TTC GCC TCC CAA CTG CCC   1458
   Ala Ile Leu Gly Ala Val Leu Gly Ser Arg Phe Ala Ser Gln Leu Pro
               440                 445                 450
   GCC GCC ATC ACC GGC ACC GGC TCC CTC GAC GAG GCA CTG CGC GAC GCC   1506
   Ala Ala Ile Thr Gly Thr Gly Ser Leu Asp Glu Ala Leu Arg Asp Ala
           455                 460                 465
   ACA CCC CAA CAG GCC GGG CAG GTC CAC GAC GCG TTC GCC GAC GCG GTG   1554
   Thr Pro Gln Gln Ala Gly Gln Val His Asp Ala Phe Ala Asp Ala Val
       470                 475                 480
   AAC ACC AGC CAA CTC ATC GGC GCC GCC GCC GTG TTC ACC GGC GGC CTG   1602
   Asn Thr Ser Gln Leu Ile Gly Ala Ala Ala Val Phe Thr Gly Gly Leu
   485                 490                 495                 500
   CTC GCC GCG CTG CTG CTG CAC CGC GCC GAC CGC AAG GCC GCC CCC CAG   1650
   Leu Ala Ala Leu Leu Leu His Arg Ala Asp Arg Lys Ala Ala Pro Gln
                   505                 510                 515
   CCC ACC GCC CCC ACC CCC GAA CCC ACC ACC ACC GCC TGACCCCCGG        1696
   Pro Thr Ala Pro Thr Pro Glu Pro Thr Thr Thr Ala
               520                 525
   CCCGCCGGGC ACCACACAAC CCACGGCCCC ACCCCTGCGG CTCCCCACCG GGACCCACAG 1756
   GGGCGGGGCC GTGCCGCTGC CCTGCCCACA CACACAGCCC CCACACACAC AGCCCCCGCA 1816
   CGGCCGACAG CGCCGGG                                                1833
(2) INFORMATION POUR LA SEQ ID NO: 7:
   (i) CARACTERISTIQUES DE LA SEQUENCE:
      (A) LONGUEUR: 695 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 212..695
      (D) AUTRES RENSEIGNEMENTS: /product= "Gene SnaC"
   (xi) DESCRIPTION DE LA SEQUENCE: SEQ ID NO: 7:
   CTCGAGCCGC GCCCCCAGGT GCTGGTGTCG CTCGCCGTGG AGAAGGGCGC CGACGGCACC      60
   GCGCCGCCGG ACCGGCTGCT GATCCACGAC GGCTTCCCCT GGGGCCGCGC CGCCCCGCGC     120
   GAAGCGGAGC TGCCCACCGG GCACCGCGCC CTGCCGGCCC TGGCCGGCGC CGCCCGCTGA     180
   GGCGCGGCAA CCACCAACAG AAGGAGCCCC C GTG ACA GGA GCC GAC GAC CCG       232
                                     Val Thr Gly Ala Asp Asp Pro
                                      1               5
   GCA AGG CCC GCG GTC GGC CCG CAG AGT TTC CGA GAC GCG ATG GCG CAG      280
   Ala Arg Pro Ala Val Gly Pro Gln Ser Phe Arg Asp Ala Met Ala Gln
            10                  15                  20
   CTG GCG TCG CCC GTC ACC GTC GTA ACC GTC CTC GAC GCG GCC GGA CGC      328
   Leu Ala Ser Pro Val Thr Val Val Thr Val Leu Asp Ala Ala Gly Arg
        25                  30                  35
   CGC CAC GGC TTC ACG GCC GGC TCG GTG GTC TCT GTG TCG CTG GAC CCG      376
   Arg His Gly Phe Thr Ala Gly Ser Val Val Ser Val Ser Leu Asp Pro
    40                  45                  50                  55
   CCG CTG GTG ATG GTC GGC ATC GCG CTC ACC TCC AGC TGC CAC ACG GCG      424
   Pro Leu Val Met Val Gly Ile Ala Leu Thr Ser Ser Cys His Thr Ala
                    60                  65                  70
   ATG GCC GCC GCC GCC GAG TTC TGC GTC AGC ATC CTC GGC GAG GAC CAG      472
   Met Ala Ala Ala Ala Glu Phe Cys Val Ser Ile Leu Gly Glu Asp Gln
                75                  80                  85
   CGC GCC GTC GCG AAG CGG TGC GCG ACG CAC GGC GCC GAC CGG TTC GCG      520
   Arg Ala Val Ala Lys Arg Cys Ala Thr His Gly Ala Asp Arg Phe Ala
            90                  95                 100
   GGC GGC GAG TTC GCC GCC TGG GAC GGT ACG GGG GTG CCC TAC CTG CCG      568
   Gly Gly Glu Phe Ala Ala Trp Asp Gly Thr Gly Val Pro Tyr Leu Pro
       105                 110                 115
   GAC GCC AAG GTC GTC CTG CGC TGC CGC ACC ACG GAC GTG GTG CGC GCC      616
   Asp Ala Lys Val Val Leu Arg Cys Arg Thr Thr Asp Val Val Arg Ala
   120                 125                 130                 135
   GGC GAC CAC GAC CTG GTG CTC GGC ACG CCC GTG GAG ATC CGC ACG GGC      664
   Gly Asp His Asp Leu Val Leu Gly Thr Pro Val Glu Ile Arg Thr Gly
                   140                 145                 150
   GAC CCG GCG AAG CCA CCC CTG CTG TGG TAC C                            695
   Asp Pro Ala Lys Pro Pro Leu Leu Trp Tyr
               155                 160
(2) INFORMATION POUR LA SEQ ID NO: 8:
   (i) CARACTERISTIQUES DE LA SEQUENCE:
      (A) LONGUEUR: 640 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 1..640
      (D) AUTRES RENSEIGNEMENTS: /product= "gene SnaD"
   (xi) DESCRIPTION DE LA SEQUENCE: SEQ ID NO: 8:
   GCG ACC GCC CGG CTC ATC GGC CCG CTG CCG CGC CGG CTG GGC CTC CAG     48
   Ala Thr Ala Arg Leu Ile Gly Pro Leu Pro Arg Arg Leu Gly Leu Gln
    1               5                  10                  15
   GTG CAC CAG GTG ATG ACG GGC GCG TTC GCG CAG GCC CTC GCC CGC TGG     96
   Val His Gln Val Met Thr Gly Ala Phe Ala Gln Ala Leu Ala Arg Trp
               20                  25                  30
   CGG GGC AGC CGC GCC GTC ACC TTC GAC GTG GAG ACC CAC GGA CGG CAC    144
   Arg Gly Ser Arg Ala Val Thr Phe Asp Val Glu Thr His Gly Arg His
           35                  40                  45
   GGC CGC GAC GAA CTG TTC CGT ACC GTC GGC TGG TTC ACC TCC ATC CAC    192
   Gly Arg Asp Glu Leu Phe Arg Thr Val Gly Trp Phe Thr Ser Ile His
       50                  55                  60
   CCC GTC GTC CTG GGC GCG GAC CGC TCC GTG CAC CCC GAG CAG TAC CTC    240
   Pro Val Val Leu Gly Ala Asp Arg Ser Val His Pro Glu Gln Tyr Leu
   65                  70                  75                  80
   GCC CAG ATC GGC GCG GCG CTG ACC GCC GTA CCG GAC GGC GGC GTC GGC    288
   Ala Gln Ile Gly Ala Ala Leu Thr Ala Val Pro Asp Gly Gly Val Gly
                   85                  90                  95
   TTC GGC GCC TGC CGC GAG TTC TCC CCG GAC GCC GGG CTG CGC ACT CTG    336
   Phe Gly Ala Cys Arg Glu Phe Ser Pro Asp Ala Gly Leu Arg Thr Leu
               100                 105                 110
   CTG CGT GAC CTG CCG CCC GCC CTG GTG TGC TTC AAC TAC TAC GGT CAG    384
   Leu Arg Asp Leu Pro Pro Ala Leu Val Cys Phe Asn Tyr Tyr Gly Gln
           115                 120                 125
   GCC GAC CAG TTG AGC CCG AAC GGC GGT TTC CGT ATG TCG GGC CGT CCC    432
   Ala Asp Gln Leu Ser Pro Asn Gly Gly Phe Arg Met Ser Gly Arg Pro
       130                 135                 140
   ATC CCG CGC GAG CAC TCC GCC CGC TGC GAG CGC GTC TAC GGC ATC GAG    480
   Ile Pro Arg Glu His Ser Ala Arg Cys Glu Arg Val Tyr Gly Ile Glu
   145                 150                 155                 160
   GTG TAC GGC ATC GTC CAC GGC GGC CGC CTG CGC ATG GGC CTG ACC TGG    528
   Val Tyr Gly Ile Val His Gly Gly Arg Leu Arg Met Gly Leu Thr Trp
               165                 170                 175
   GTG CCG AGC CCG GCG GAC GGT GTG GAC GAG GCC GGC GTC GAC GCG CTC    576
   Val Pro Ser Pro Ala Asp Gly Val Asp Glu Ala Gly Val Asp Ala Leu
           180                 185                 190
   GTG GAG CAG ATG AGC TGG GTG CTG GCC ACG CTC GCG GGC GCC GAC CCG    624
   Val Glu Gln Met Ser Trp Val Leu Ala Thr Leu Ala Gly Ala Asp Pro
       195                 200                 205
   CAC GCC GTG ACC CCG G                                              640
   His Ala Val Thr Pro
       210
(2) INFORMATION POUR LA SEQ ID NO: 9:
   (i) CARACTERISTIQUES DE LA SEQUENCE:
      (A) LONGUEUR: 645 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 61..645
      (D) AUTRES RENSEIGNEMENTS: /product= "gene papA"
   (xi) DESCRIPTION DE LA SEQUENCE: SEQ ID NO: 9:
   GGCGTCAAGA ACCTGCCGCT GACCGTACGG CGCGGCTGAC ACAGACAAGG GGGCCACCTG     60
   GTG CGC ACC GTG CGA ACC CTG CTG ATC GAC AAC TAC GAC TCG TTC ACC      108
   Val Arg Thr Val Arg Thr Leu Leu Ile Asp Asn Tyr Asp Ser Phe Thr
     1               5                  10                  15
   TAC AAC CTC TTC CAG ATG CTG GCC GAG GTG AAC GGC GCC GCT CCG CTC      156
   Tyr Asn Leu Phe Gln Met Leu Ala Glu Val Asn Gly Ala Ala Pro Leu
                20                  25                  30
   GTC GTC CGC AAC GAC GAC ACC CGC ACC TGG CAG GCC CTG GCG CCG GGC      204
   Val Val Arg Asn Asp Asp Thr Arg Thr Trp Gln Ala Leu Ala Pro Gly
            35                  40                  45
   GAC TTC GAC AAC GTC GTC GTC TCA CCC GGC CCC GGC CAC CCC GCC ACC      252
   Asp Phe Asp Asn Val Val Val Ser Pro Gly Pro Gly His Pro Ala Thr
        50                  55                  60
   GAC ACC GAC CTG GGC CTC AGC CGC CGG GTG ATC ACC GAA TGG GAC CTG      300
   Asp Thr Asp Leu Gly Leu Ser Arg Arg Val Ile Thr Glu Trp Asp Leu
    65                  70                  75                  80
   CCG CTG CTC GGG GTG TGC CTG GGC CAC CAG GCC CTG TGC CTG CTC GCC      348
   Pro Leu Leu Gly Val Cys Leu Gly His Gln Ala Leu Cys Leu Leu Ala
                    85                  90                  95
   GGC GCC GCC GTC GTC CAC GCA CCC GAA CCC TTT CAC GGC CGC ACC AGC      396
   Gly Ala Ala Val Val His Ala Pro Glu Pro Phe His Gly Arg Thr Ser
               100                 105                 110
   GAC ATC CGC CAC GAC GGG CAG GGC CTG TTC GCG AAC ATC CCC TCC CCG      444
   Asp Ile Arg His Asp Gly Gln Gly Leu Phe Ala Asn Ile Pro Ser Pro
           115                 120                 125
   CTG ACC GTG GTC CGC TAC CAC TCG CTG ACC GTC CGG CAA CTG CCC GCC      492
   Leu Thr Val Val Arg Tyr His Ser Leu Thr Val Arg Gln Leu Pro Ala
       130                 135                 140
   GAC CTG CGC GCC ACC GCC CAC ACC GCC GAC GGG CAG CTG ATG GCC GTC      540
   Asp Leu Arg Ala Thr Ala His Thr Ala Asp Gly Gln Leu Met Ala Val
   145                 150                 155                 160
   GCC CAC CGC CAC CTG CCC CGC TTC GGC GTG CAG TTC CAC CCC GAA TCG      588
   Ala His Arg His Leu Pro Arg Phe Gly Val Gln Phe His Pro Glu Ser
                   165                 170                 175
   ATC AGC AGC GAA CAC GGC CAC CGG ATG CTC GCC AAC TTC CGC GAC CTG      636
   Ile Ser Ser Glu His Gly His Arg Met Leu Ala Asn Phe Arg Asp Leu
               180                 185                 190
   TCC CTG CGC                                                          645
   Ser Leu Arg
           195
(2) INFORMATION POUR LA SEQ ID NO: 10:
   (i) CARACTERISTIQUES DE LA SEQUENCE:
      (A) LONGUEUR: 1052 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 84..962
      (D) AUTRES RENSEIGNEMENTS: /product= "Gene PapM"
   (xi) DESCRIPTION DE LA SEQUENCE: SEQ ID NO: 10:
   CTCGAGGACG AGTGGATCGC CTCCGGCGGC GCCCCCGTCC CCACGCCCGT GCACGCGTCC      60
   GCGTCCGCGC GGGGGGCCGT GTC GTG ACC GCC GCC GCA CCC ACC CTC GCC        110
                             Val Thr Ala Ala Ala Pro Thr Leu Ala
                               1               5
   CAG GCG CTG GAC GAG GCC ACC GGG CAG CTG ACC GGC GCC GGG ATC ACC      158
   Gln Ala Leu Asp Glu Ala Thr Gly Gln Leu Thr Gly Ala Gly Ile Thr
    10                  15                  20                  25
   GCC GAC GCC GCC CGG GCC GAC ACC CGG CTG CTG GCC GCC CAC GCC TGC      206
   Ala Asp Ala Ala Arg Ala Asp Thr Arg Leu Leu Ala Ala His Ala Cys
                    30                  35                  40
   CAG GTC GCC CCG GGG GAC CTC GAC ACC TGC CTG GCC GGC CCG GTG CCG      254
   Gln Val Ala Pro Gly Asp Leu Asp Thr Cys Leu Ala Gly Pro Val Pro
                45                  50                  55
   CCC CGG TTC TGG CAC TAC GTC CGG CGC CGT CTG ACC CGC GAA CCC GCC      302
   Pro Arg Phe Trp His Tyr Val Arg Arg Arg Leu Thr Arg Glu Pro Ala
            60                  65                  70
   GAA CGC ATC GTC GGC CAC GCC TAC TTC ATG GGC CAC CGC TTC GAC CTG      350
   Glu Arg Ile Val Gly His Ala Tyr Phe Met Gly His Arg Phe Asp Leu
        75                  80                  85
   GCC CCC GGC GTC TTC GTC CCC AAA CCC GAG ACC GAG GAG ATC ACC CGG      398
   Ala Pro Gly Val Phe Val Pro Lys Pro Glu Thr Glu Glu Ile Thr Arg
    90                  95                 100                 105
   GAC GCC ATC GCC CGC CTG GAG GCC CTC GTC CGC CGC GGC ACC ACC GCA      446
   Asp Ala Ile Ala Arg Leu Glu Ala Leu Val Arg Arg Gly Thr Thr Ala
                   110                 115                 120
   CCC CTG GTC GTC GAC CTG TGC GCC GGA CCG GGC ACC ATG GCC GTC ACC      494
   Pro Leu Val Val Asp Leu Cys Ala Gly Pro Gly Thr Met Ala Val Thr
               125                 130                 135
   CTG GCC CGC CAC GTA CCG GCC GCC CGC GTC CTG GGC ATC GAA CTC TCC      542
   Leu Ala Arg His Val Pro Ala Ala Arg Val Leu Gly Ile Glu Leu Ser
           140                 145                 150
   CAG GCC GCC GCC CGC GCC GCC CGG CGC AAC GCC CGC GGC ACC GGC GCC      590
   Gln Ala Ala Ala Arg Ala Ala Arg Arg Asn Ala Arg Gly Thr Gly Ala
       155                 160                 165
   CGC ATC GTG CAG GGC GAC GCC CGC GAC GCC TTC CCC GAA CTG AGC GGC      638
   Arg Ile Val Gln Gly Asp Ala Arg Asp Ala Phe Pro Glu Leu Ser Gly
   170                 175                 180                 185
   ACC GTC GAC CTC GTC GTC ACC AAC CCG CCC TAC ATC CCC ATC GGA CTG      686
   Thr Val Asp Leu Val Val Thr Asn Pro Pro Tyr Ile Pro Ile Gly Leu
                   190                 195                 200
   CGC ACC TCC GCA CCC GAA GTG CTC GAG CAC GAC CCG CCG CTG GCC CTG      734
   Arg Thr Ser Ala Pro Glu Val Leu Glu His Asp Pro Pro Leu Ala Leu
               205                 210                 215
   TGG GCC GGG GAG GAG GGC CTC GGC ATG ATC CGC GCC ATG GAA CGC ACC      782
   Trp Ala Gly Glu Glu Gly Leu Gly Met Ile Arg Ala Met Glu Arg Thr
            220                      225                      230
   GCG GCC CGG CTG CTG GCC CCC GGC GGC GTC CTG CTC CTC GAA CAC GGC      830
   Ala Ala Arg Leu Leu Ala Pro Gly Gly Val Leu Leu Leu Glu His Gly
       235                 240                 245
   TCC TAC CAA CTC GCC TCC GTG CCC GCC CTG TTC CGC GCA ACC GGC CGC      878
   Ser Tyr Gln Leu Ala Ser Val Pro Ala Leu Phe Arg Ala Thr Gly Arg
   250                 255                 260                 265
   TGG AGC CAC GCC TCG TCC CGT CCC ACC TGC AAC GAC GGC TGC CTG ACC      926
   Trp Ser His Ala Ser Ser Arg Pro Thr Cys Asn Asp Gly Cys Leu Thr
                   270                 275                 280
   GCC GTA CGC AAC CAC ACC TGC GCA CCG CCC GCC TGACACGGCG TCACGGCACG    979
   Ala Val Arg Asn His Thr Cys Ala Pro Pro Ala
               285                 290
   GCCGGCCTGT CGGCAACGAC CCTACGCCAT TGACAAACCG ACCGTGCCGT TTTTTTAATG   1039
   TCGGGGTGGC GGA                                                      1052
(2) INFORMATION POUR LA SEQ ID NO: 11:
   (i) CARACTERISTIQUES DE LA SEQUENCE:
      (A) LONGUEUR: 227 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 3..227
      (D) AUTRES RENSEIGNEMENTS: /product= "Partie du gene SnbC"
   (xi) DESCRIPTION DE LA SEQUENCE: SEQ ID NO: 11:
   AG ATC TTC GAG CAC AAG ACC GTC GCC CAG CTC GCA CCC GTC GCC GAG       47
      Ile Phe Glu His Lys Thr Val Ala Gln Leu Ala Pro Val Ala Glu
       1               5                  10                  15
   ACG CTC GCC GAC ACC ACC CGC GAG GAA CCC GCC GCC GTC GCC GCG ACC      95
   Thr Leu Ala Asp Thr Thr Arg Glu Glu Pro Ala Ala Val Ala Ala Thr
                   20                  25                  30
   GGC GAC GTA CCG CTC ACC CCG ATC ATG CAC TGG CTG CGC GAA CGC GGC     143
   Gly Asp Val Pro Leu Thr Pro Ile Met His Trp Leu Arg Glu Arg Gly
               35                  40                  45
   GGC CCC GTC GAC GCG TTC AGC CAG ACG ATG GCC GTC ACC GTC CCC GCC     191
   Gly Pro Val Asp Ala Phe Ser Gln Thr Met Ala Val Thr Val Pro Ala
           50                  55                  60
   GGC CTG GAC CGG GAA CGG CTC GTG GCC GCC CTG CAG                     227
   Gly Leu Asp Arg Glu Arg Leu Val Ala Ala Leu Gln
       65                  70                  75
(2) INFORMATION POUR LA SEQ ID NO: 12:
   (i) CARACTERISTIQUES DE LA SEQUENCE:
      (A) LONGUEUR: 247 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 1..247
      (D) AUTRES RENSEIGNEMENTS: /product = "Partie du gene SnbC"
   (xi) DESCRIPTION DE LA SEQUENCE: SEQ ID NO: 12:
   CTC GAG TAC GAC ACC GCC CTG TAC GAG CGG GCC ACC GCC GAA GCC CTC      48
   Leu Glu Tyr Asp Thr Ala Leu Tyr Glu Arg Ala Thr Ala Glu Ala Leu
    1               5                  10                  15
   ACC GGC CGG CTG CTG CGG CTC CTC GAC GCC GTC GTC ACC GAC CCG CAG      96
   Thr Gly Arg Leu Leu Arg Leu Leu Asp Ala Val Val Thr Asp Pro Gln
               20                  25                  30
   GCG CCG GTC GGC TCC CAC GAC CTC CTC GAA GAG GCC GAA CAC GCC CGC     144
   Ala Pro Val Gly Ser His Asp Leu Leu Glu Glu Ala Glu His Ala Arg
           35                  40                  45
   CTG GCA GCC TTC AAC GAC ACC GCC CGG CCC GTG CCG CGA GCC GGC CTC     192
   Leu Ala Ala Phe Asn Asp Thr Ala Arg Pro Val Pro Arg Ala Gly Leu
        50                  55                  60
   GCC GAA CTC TTC ACC GCC CAG GCC CGC CGC ACC GCC GAT GCG GTC GCC     240
   Ala Glu Leu Phe Thr Ala Gln Ala Arg Arg Thr Ala Asp Ala Val Ala
    65                  70                  75                  80
   GTC GTC G                                                           247
   Val Val
(2) INFORMATION POUR LA SEQ ID NO: 13:
   (i) CARACTERISTIQUES DE LA SEQUENCE:
      (A) LONGUEUR: 192 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 3..192
      (D) AUTRES RENSEIGNEMENTS: /product= "Partie du gene SnbD"
   (xi) DESCRIPTION DE LA SEQUENCE: SEQ ID NO: 13:
   GC ATG CCC CCC GTC ACC CCC TAC CGC GCC TAC CTG GCC CAC CTC GCC      47
      Met Pro Pro Val Thr Pro Tyr Arg Ala Tyr Leu Ala His Leu Ala
       1               5                  10                  15
   GGC CGT GAC GAC GAC GCC GCC CGC GCC GCG TGG CGG ACC GCC CTC GCG     95
   Gly Arg Asp Asp Asp Ala Ala Arg Ala Ala Trp Arg Thr Ala Leu Ala
                   20                  25                  30
   GAC CTG GAG GAG CCG AGC CTC GTC GCG GGC GCC GGA GCA GGC CGC GGC    143
   Asp Leu Glu Glu Pro Ser Leu Val Ala Gly Ala Gly Ala Gly Arg Gly
               35                  40                  45
   GCC GCC GAC GGC TCC GCC CTG CCC GGC CAG ATC CCC GGT TAC CGA GCT C  192
   Ala Ala Asp Gly Ser Ala Leu Pro Gly Gln Ile Pro Gly Tyr Arg Ala
           50                  55                  60
(2) INFORMATION POUR LA SEQ ID NO: 14:
   (i) CARACTERISTIQUES DE LA SEQUENCE:
      (A) LONGUEUR: 474 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 1..474
      (D) AUTRES RENSEIGNEMENTS: /product= "Partie du gene SnbD"
   (xi) DESCRIPTION DE LA SEQUENCE: SEQ ID NO: 14:
   CTG CAG GTC GAG GGC CGG CCC GCG CAC CTG GAA CTG CCC TGC GAC CAC     48
   Leu Gln Val Glu Gly Arg Pro Ala His Leu Glu Leu Pro Cys Asp His
                       1               5                   10                  15
   CCC CGG CCC GCC GTC GCC ACC CAC CGC GGC GCC ACC GTG CCC TTC CAC     96
   Pro Arg Pro Ala Val Ala Thr His Arg Gly Ala Thr Val Pro Phe His
               20                  25                  30
   ATC GAC GCC GGC CTC CAC GAG AAG CTG ACC GCG CTC TCC AAG GCC TGC    144
   Ile Asp Ala Gly Leu His Glu Lys Leu Thr Ala Leu Ser Lys Ala Cys
           35                  40                  45
   GAC AGC AGC CTG TTC ATG GTG CTC CAG GCC GCG GTC GCC GCC CTG CTC    192
   Asp Ser Ser Leu Phe Met Val Leu Gln Ala Ala Val Ala Ala Leu Leu
       50                  55                  60
   ACC CGG CAC GGC GCC GGC ACC GAC ATC CCC GTC GGC AGC CCC GTC GCC    240
   Thr Arg His Gly Ala Gly Thr Asp Ile Pro Val Gly Ser Pro Val Ala
   65                  70                  75                  80
   GGC CGC ACC GAC GAC GCC CTC GAC GAC CTG GTG GGC TTC TTC GTC AAC    288
   Gly Arg Thr Asp Asp Ala Leu Asp Asp Leu Val Gly Phe Phe Val Asn
                   85                  90                  95
   ACC CTC GTC CTG CGC ACC GAC ACC TCC GGC GAC CCC ACC TTC CGC GAA    336
   Thr Leu Val Leu Arg Thr Asp Thr Ser Gly Asp Pro Thr Phe Arg Glu
               100                 105                 110
   CTC GTC GCA CGC GTG CGG CAG TTC GAC CTC GCC GCC TAC ACG CAC CAG    384
   Leu Val Ala Arg Val Arg Gln Phe Asp Leu Ala Ala Tyr Thr His Gln
           115                 120                 125
   GAC ATG CCG TTC GAA AAG CTC GTC GAA GAG GTC AAC CCC GAG CGC TCC    432
   Asp Met Pro Phe Glu Lys Leu Val Glu Glu Val Asn Pro Glu Arg Ser
       130                 135                 140
   CTG GCC CGC AAC CCG CTC TTC CAG GTC GTC CTG GCG CTG CAG            474
   Leu Ala Arg Asn Pro Leu Phe Gln Val Val Leu Ala Leu Gln
   145                 150                 155
(2) INFORMATION POUR LA SEQ ID NO: 15:
   (i) CARACTERISTIQUES DE LA SEQUENCE:
      (A) LONGUEUR: 485 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 3..485
      (D) AUTRES RENSEIGNEMENTS: /product= "Partie du gene SnbE"
   (xi) DESCRIPTION DE LA SEQUENCE: SEQ ID NO: 15:
   GC ATG CCG CGC TCC CTC GAC CTG TAC GTC GCA CTG CTC GCC GTC CTC        47
      Met Pro Arg Ser Leu Asp Leu Tyr Val Ala Leu Leu Ala Val Leu
       1               5                  10                  15
   AAG ACC GGC GCC GCC TAC CTG CCC GTC GAC ATC TCC TAC CCG GCC GAA       95
   Lys Thr Gly Ala Ala Tyr Leu Pro Val Asp Ile Ser Tyr Pro Ala Glu
                   20                  25                  30
   CGC ATC GCG TTC ATG ATC GAG GAC GCC CGC CCG GTG ACC GTC CTC GAC      143
   Arg Ile Ala Phe Met Ile Glu Asp Ala Arg Pro Val Thr Val Leu Asp
               35                  40                  45
   CGC CTG CCC GAC GAC CTG GGC GCC TAC CGG GAC ACC GAC CTC ACC GAC      191
   Arg Leu Pro Asp Asp Leu Gly Ala Tyr Arg Asp Thr Asp Leu Thr Asp
           50                  55                  60
   GCC GAC CGC ACG GCG CCG CTA CGG CCC GAA CAC CCG GCG TAC GTC ATC      239
   Ala Asp Arg Thr Ala Pro Leu Arg Pro Glu His Pro Ala Tyr Val Ile
       65                  70                  75
   CAC ACC TCC GGC TCC ACC GGC ACC CCC AAG GCC GTC GTC ATG CCC CAC      287
   His Thr Ser Gly Ser Thr Gly Thr Pro Lys Ala Val Val Met Pro His
   80                  85                  90                  95
   GCC GGC CTG GTC AAC CTG CTG ACC TGG CAC GCC CGC CGC TTC CCC GGC      335
   Ala Gly Leu Val Asn Leu Leu Thr Trp His Ala Arg Arg Phe Pro Gly
                   100                 105                 110
   GGC ACC GGG GTG CGC ACC GCC CAG TTC ACC GCC ATC GGC TTC GAC TTC      383
   Gly Thr Gly Val Arg Thr Ala Gln Phe Thr Ala Ile Gly Phe Asp Phe
               115                 120                 125
   TCG GTG CAG GAG ATC CTC TCC CCG CTC GTC ATG GGC AAG ACC CTC GCC      431
   Ser Val Gln Glu Ile Leu Ser Pro Leu Val Met Gly Lys Thr Leu Ala
           130                 135                 140
   GTG CCC TCG GAA GAG GTC CGC CAC AGC GCC GAA CTG CTG GCC GGC TGG      479
   Val Pro Ser Glu Glu Val Arg His Ser Ala Glu Leu Leu Ala Gly Trp
       145                 150                 155
   CTC GAG                                                              485
   Leu Glu
   160
(2) INFORMATION POUR LA SEQ ID NO: 16:
   (i) CARACTERISTIQUES DE LA SEQUENCE:
      (A) LONGUEUR: 291 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: S.pristinaespiralis
   (ix) CARACTERISTIQUE ADDITIONELLE:
      (A) NOM/CLE: CDS
      (B) EMPLACEMENT: 1..291
      (D) AUTRES RENSEIGNEMENTS: /product= "Partie du gene SnbE"
   (xi) DESCRIPTION DE LA SEQUENCE: SEQ ID NO: 16:
   CTG CAG GCC GAG GGC GCC GAA GTG AGC CTG CTG GCC GTC CTC GAC GGC        48
   Leu Gln Ala Glu Gly Ala Glu Val Ser Leu Leu Ala Val Leu Asp Gly
     1               5                  10                  15
   TAC CCC GAC GCC TAC GAC GGC ACC GAG CAC GAG GTC GGC GAG GAA CAG        96
   Tyr Pro Asp Ala Tyr Asp Gly Thr Glu His Glu Val Gly Glu Glu Gln
                20                  25                  30
   GTC CTG GCG ATC CTC CTC AAC GCC GCC GGC GTC GAC CGG GCC CAG GCC       144
   Val Leu Ala Ile Leu Leu Asn Ala Ala Gly Val Asp Arg Ala Gln Ala
            35                  40                  45
   TTC GGC GAC GCC CCC CTC CAA CGG GCC GCC GTG CTC GAG AAG CTG CGC       192
   Phe Gly Asp Ala Pro Leu Gln Arg Ala Ala Val Leu Glu Lys Leu Arg
        50                  55                  60
   GAC AGC GGC AGC GCC CTG GGC AAC CTC GAC GAC GAC GCG GTC GGC CGC       240
   Asp Ser Gly Ser Ala Leu Gly Asn Leu Asp Asp Asp Ala Val Gly Arg
    65                  70                  75                  80
   ATG GTC ACC GTC TTC CTC AAC AAC ACG CGC CTC ATC CAG AAC TTC CGG       288
   Met Val Thr Val Phe Leu Asn Asn Thr Arg Leu Ile Gln Asn Phe Arg
                    85                  90                  95
   CCC                                                                   291
   Pro

## Description

La présente invention concerne de nouveaux polypeptides impliqués dans la biosynthèse des Streptogramines et comprend également l'isolement et l'identification de gènes de biosynthèse des composants A et B des Streptogramines, l'expression de ces gènes dans le but d'augmenter les taux de production et leur utilisation pour la construction de mutants bloqués pouvant conduire à la synthèse de nouveaux antibiotiques ou à des formes dérivées de Streptogramines.

Les Streptogramines forment un groupe homogène d'antibiotiques constitués d'une association de deux types de molécules chimiquement différentes; d'une part des macrolactones polyinsaturées (composants du groupe A, dont deux exemples de structures sont présentées figure 1) et d'autre part, des depsipeptides (composants du groupe B, dont trois exemples de structure sont présentés sur la figure 2). Ce groupe comprend de nombreux antibiotiques (cf. tableau 1 et figure 3) connus sous différents noms en fonction de leur origine dont les Pristinamycines, les Mikamycines, les Virginiamycines (pour une revue, voir Cocito 1979, 1983).

Les composants A et B ont une activité antibactérienne synergique qui peut atteindre 100 fois celle des composants séparés et qui, contrairement à celle de chaque composant, est bactéricide (Cocito 1979). Cette activité est plus particulièrement efficace contre les bactéries Gram-positives, comme les Staphylocoques et Streptocoques (Cocito 1979, Videau 1982). Les composants A et B inhibent la synthèse protéique en se fixant à la sous-unité 50S du ribosome (Cocito 1979 ; pour une revue, voir Di Giambattista et al. 1989).

Les Streptogramines sont essentiellement produites par des Actinomycètes dont de nombreux Streptomycètes, présentés dans le tableau 1. En outre, les Streptogramines sont aussi synthétisées par des eucaryotes tel que Micromonospora qui synthétise les Vernamycines. Les Actinomycètes constituent un groupe de microorganismes très intéressant du fait de la quantité importante de métabolites secondaires qu'ils produisent, parmis lesquels de nombreux antibiotiques (Beta-lactames, Tétracyclines, Macrolides, Aminoglycosides, Polyacétates, etc), des herbicides, des anti-cancéreux, des antifongiques, des immunomodulateurs, des inhibiteurs d'enzymes. De nombreuses voies de biosynthèse, concernant des antibiotiques appartenant à des classes variées ainsi que d'autres métabolites secondaires tels que des pigments (pour une revue, Chater 1990), ont à ce jour déjà été étudiées chez les Actinomycètes. Un aspect important de ce groupe de bactéries, est que les gènes impliqués dans une même voie de biosynthèse, gènes de structure, mais également, gène(s) de résistance et gène(s) de régulation, sont groupés physiquement sur le chromosome, constituant des clusters, pouvant atteindre plus de 100 kb (Hopwood et al.1986a, Hopwood et al. 1986b, Hallam et al. 1988, Anzai et al. 1987, Ohnuki et al. 1985). A ce jour aucun exemple n'est venu contredire cette constatation. Une telle organisation structurale présente un intérêt important dans le développement des stratégies de clonage des gènes de biosynthèse. En effet, il est possible, à partir d'un seul gène préalablement cloné par des techniques diverses, gène de biosynthèse, de résistance ou de régulation, de marcher le long du chromosome et d'isoler ainsi l'ensemble des gènes du cluster de biosynthèse.

La connaissance des voies de biosynthèse de chacun des composants des Streptogramines n'est que très partielle à ce jour, mais l'origine des différentes parties de chaque molécule a été identifiée par marquage radioactif (Kingston et al. 1983). Ainsi, les composants de type A sont formés de deux régions provenant de la condensation d'acétates et de plusieurs acides aminés tels que la sérine, la glycine, par exemple. En ce qui concerne les composants de type B, des études ont montré que tous les acides aminés présents dans la chaîne peptidique dérivent des acides aminés naturels (Hook et Vining 1973). Toutefois, aucun polypeptide impliqué dans ces voies n'a été purifié en quantité suffisante. à ce jour pour permettre sa caractérisation moléculaire, et aucun gène de biosynthèse n'a été décrit. Dans le processus de biosynthèse des composants de type B deux parties peuvent être distinguées:
1) Synthèse des précurseurs, ou de leurs analogues, du macrocycle: acide 3-hydroxypicolinique, acide L-2-aminobutyrique, p-diméthylamino-L-phénylalanine, acide 4-oxo-L-pipecolique, L-phénylglycine.
2) Formation du macrocycle à partir des précurseurs cités ci-dessus, de la L-threonine et de la L-proline, ou de leurs analogues, avec éventuellement modification de ces précurseurs ou N-methylation peptidique.

A ce jour seule l'origine métabolique probable des précurseurs du macrocycle des composants de type B a été déterminée par des études utilisant des isotopes marqués (Reed et al., 1986, Molinero et al., 1989, Reed et al., 1989).

La présente invention résulte de la purification' de polypeptides intervenant dans la biosynthèse des Streptogramines ainsi que du clonage de gènes dont le produit intervient dans la biosynthèse des Streptogramines. Il est entendu que le terme biosynthèse des Streptogramines comprend les gènes régulateurs et les gènes conférant la résistance aux microorganismes producteurs. La présente invention permet ainsi d'augmenter les taux de production de ces métabolites grâce aux techniques d'ADN recombinant. Un autre intérêt de la présente invention réside dans la possibilité, par construction de mutants bloqués dans les différentes étapes de cette biosynthèse, de produire des intermédiaires de synthèse de chacun des deux composants. Ces intermédiaires peuvent servir de substrats à de nouvelles modifications, par voie chimique, biochimique, enzymatique ou microbiologique. De même l'isolement des gènes de biosynthèse permet, par transfert des gènes entre souches productrices, de fabriquer des antibiotiques hybrides ayant des propriétés pharmacologiquernent intéressantes (Hopwood et al. 1985a, Hopwood et al. 1985b, Hutchinson et al. 1989). Un autre intérêt de la présente invention réside dans le fait qu'elle apporte une meilleure connaissance des voies de biosynthèse des métabolites classés comme des Streptogramines. En effet, l'invention permet de construire des souches bactériennes ou fongiques dans lesquelles on exprime, sous le contrôle de signaux d'expression appropriés, une ou plusieurs protéines intervenant dans la biosynthèse des Streptogramines. De telles souches peuvent alors être utilisées pour réaliser des bioconversions. Ces bioconversions peuvent se faire soit à l'aide de cellules entières, soit à l'aide d'extraits acellulaires des dites cellules. Ces bioconversions peuvent permettre de transformer une Streptogramine en une forme dérivée, avec une enzyme d'une voie de biosynthèse. Par exemple, la Pristinamycine IIB peut être, de cette manière, transformée en Pristinamycine IIA. Le même raisonnement peut être appliqué à tout intermédiaire de biosynthèse.

Un premier objet de l'invention concerne donc une séquence nucléotidique codant pour un polypeptide impliqué dans la biosynthèse des Streptogramines.

Plus particulièrement, plusieurs gènes dont le produit intervient dans la biosynthèse des Streptogramines ont été isolés à partir de Streptomyces pristinaespiralis. Les Streptogramines produites par cette souche étant plus communément désignées par le terme Pristinamycines (Cf tableau 1), dans ce qui suit, référence sera faite dans certains cas aux gènes de biosynthèse des Pristinamycines. Mais il est clair que les résultats obtenus s'appliquent à l'ensemble des Streptogramines. Les Pristinamycines I et II correspondent respectivement aux composants B et A des Streptogramines. Les molécules de la famille des Pristinamycines II et de la famille des Pristinamycines I, désignent donc dans ce qui suit les composants A et B des Streptogramines respectivement.

La présente invention décrit en particulier l'isolement et la caractérisation des gènes snaA, snaB, snaC, snaD, papA, papM, samS, snbA, snbC, snbD, snbE et snbR. Ces gènes ont été isolés à partir d'une banque d'ADN génomique de S. pristinaespiralis. Cette banque a été obtenue par digestion partielle de l'ADN génomique de S. pristinaespiralis, par l'enzyme de restriction Sau3A. De larges fragments d'ADN, de 40 à 50 kb en moyenne, ont été clonés dans le cosmide pHC79 (Hohn, B., and Collins, J. F., 1980). Après encapsidation in vitro, les souches d'E.coli HB101 (Boyer et Roulland-Dussoix, 1969) et DH1 (Low, 1968) ont été transfectées. La banque d'ADN de S.pristinaespiralis se trouve ainsi dans deux souches différentes d'E.coli.

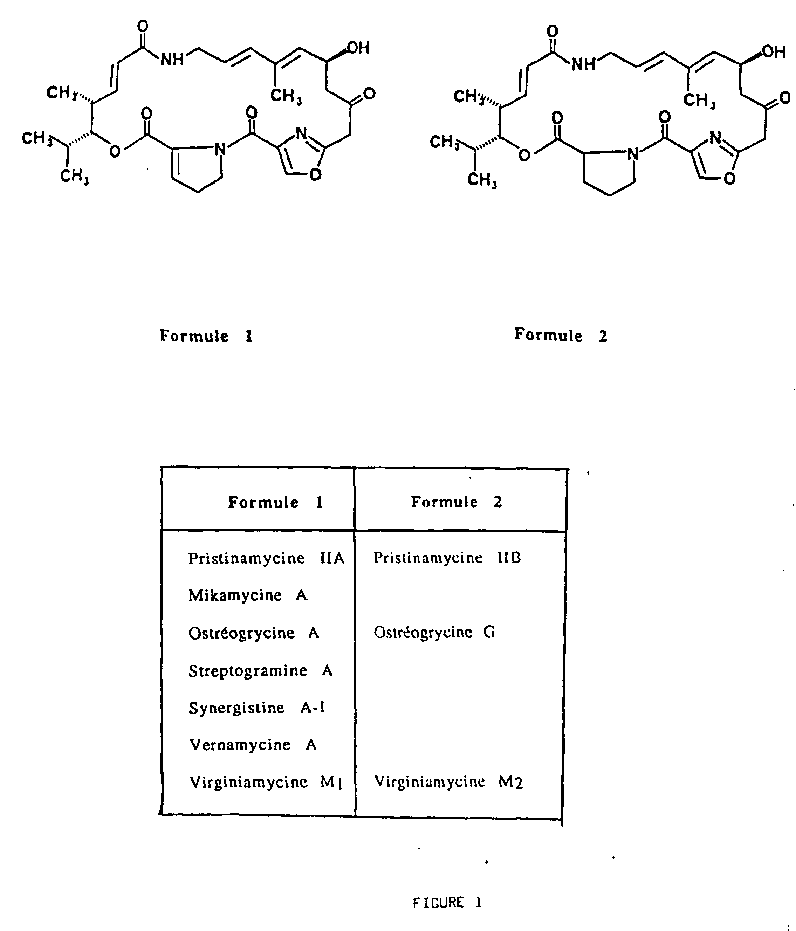

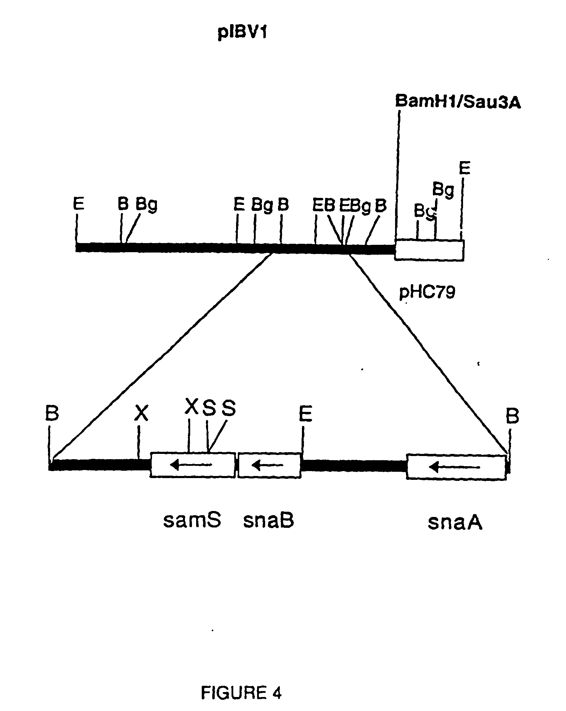

Les gènes snaA, snaB et samS (initialement désigné snaC) sont présents sur le cosmide pIBV1 (figure 4). Le produit des gènes snaA et snaB, correspondant aux polypeptides SnaA et SnaB, intervient dans la dernière étape de biosynthèse du composant II des Pristinamycines (conversion de la Pristinamycine IIB en Pristinamycine IIA) correspondant à l'oxydation de la liaison 2,3 de la D-proline. Ces deux polypeptides constituent les deux sous-unités de la Pristinamycine IIA synthase dont la purification est décrite dans la présente invention. Le produit du gène samS interviendrait dans la synthèse de la SAM (donneur de groupements méthyles) à partir d'ATP et de méthionine. Le composant A de la plupart des Streptogramines est en effet méthylé en C-4 (figure 1), et il a été décrit (Kingston et al., 1983) que ce méthyle dérive du méthyle de la méthionine, très probablement via une réaction de méthylation avec la SAM. Le gène samS coderait donc pour une SAM synthase (SamS ; EC. 2.5.1.6) spécifique de la voie de biosynthèse des Pristinamycines.

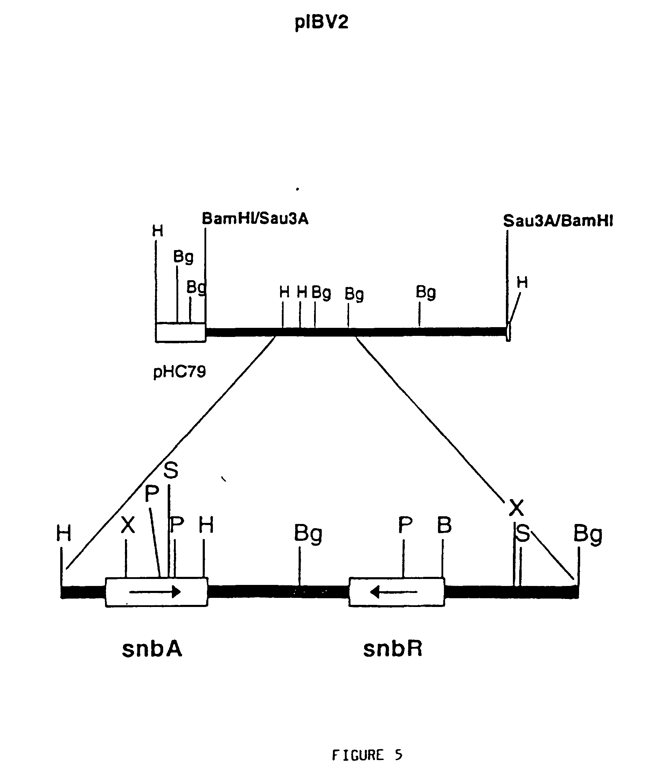

Les gènes snbA, snbR, papA et papM sont présents sur le cosmide pIBV2 (figure 5). Le gène snbA correspond, d'après les études biochimiques présentées dans l'exemple 5, à la première étape de la synthèse des Pristinamycines I. Il s'agit de l'activation du premier acide de la chaîne, l'acide 3-hydroxypicolinique, par adénylation. Le gène snbR pourrait intervenir dans le transport des molécules de la famille des Pristinamycines I (voire des Pristinamycines II) hors de la cellule après synthèse, conférant ainsi à la souche productrice une résistance à ce composant. Le gène papA correspond, d'après les analyses de séquence (exemple 8.8) et l'étude d'un mutant disrupté dans ce gène (exemple 9.3.), à un gène de biosynthèse de la paraaminophénylalanine à partir du chorismate. La paraaminophénylalanine est ensuite diméthylée par le produit du gène papM, une N-méthyltransférase décrite dans la présente invention, pour former la paradiméthylaminophénylalanine, qui est ensuite incorporée dans la Pristinamycine IA. Les gènes papA et papM interviennent donc dans la synthèse d'un des précurseurs de la Pristinamycine IA.

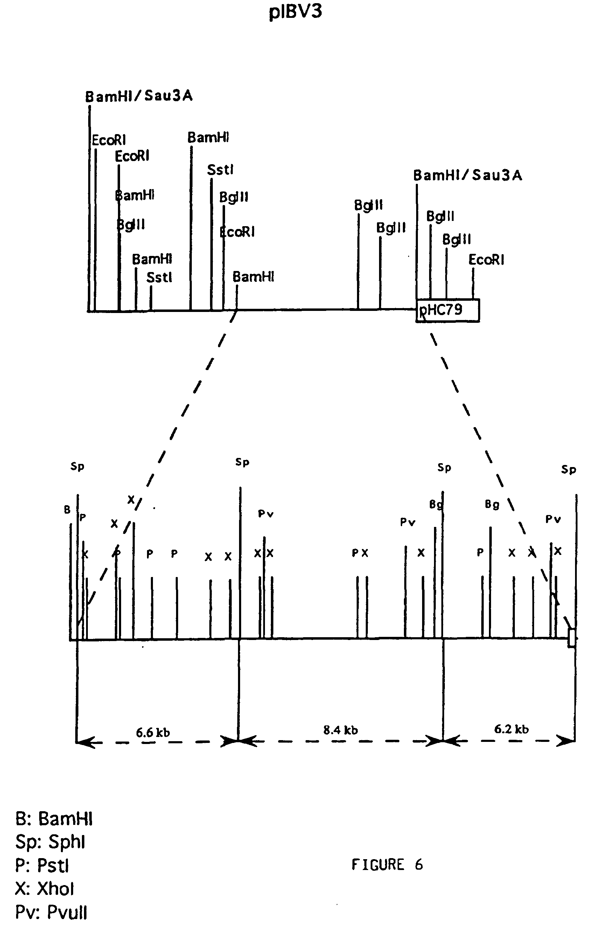

Les gènes snaA, snaD, snbC, snbD et snbE sont présents sur le cosmide pIBV3 (figure 6) qui jouxte donc le cosmide pIBV1 sur lequel le gène snaA est déjà présent. Le gène snaD code, d'après l'analyse de sa séquence (exemple 8.9.) et l'étude d'un mutant disrupté dans ce gène (exemple 9.5.), pour une peptide synthase impliquée dans la biosynthèse de la Pristinamycine II. Le gène snbC, dont le produit est décrit dans la présente invention, intervient dans l'incorporation des résidus thréonine et acide aminobutyrique dans la chaine peptidique de la Pristinamycine IA. Le gène snbD, dont le produit est aussi décrit dans la présente invention, est impliqué dans l'incorporation des résidus proline et paradiméthylaminophénylalanine dans la chaine peptidique de la Pristinamycine IA. Il gouverne aussi la N-méthylation de la liaison peptidique entre ces 2 résidus. Enfin, le gène snbE, dont le produit est aussi décrit dans la présente invention, intervient dans l'incorporation des deux derniers résidus de la Pristinamycine IA, à savoir la phénylglycine et l'acide 4-oxopipécolique.

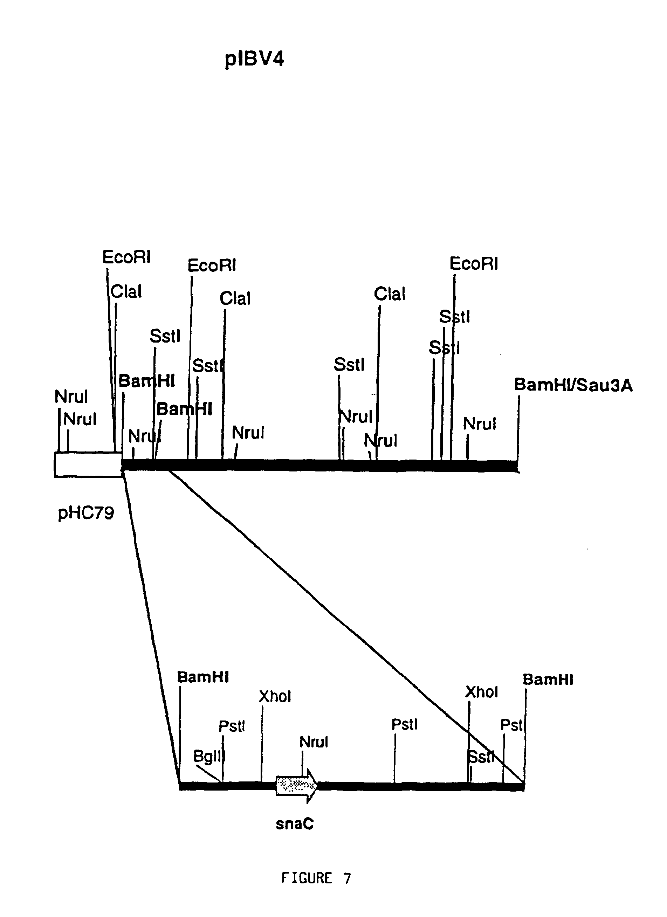

Le gène snaC est présent sur le cosmide pIBV4 (figure 7). Il code pour une FMN:NADH oxydoréductase, désignée également FMN réductase, décrite dans la présente invention, et qui fournit à la Pristinamycine IIA synthase le FMNH2 à partir du FMN et du NADH. Le gène snaC intervient donc dans l'étape finale de la biosynthèse de la Pristinamycine IIA.

Ces différents gènes ont été sous-clonés à partir de leur cosmide d'origine et leurs séquences nucléiques ont été déterminées. Les gènes snaA, snaB et samS, ont été sous-clonés sur un fragment BamHI-BamHI de 6 kb, dont une partie a été séquencée (SEQ ID n° 1). Le gène snbA a été sous cloné dans un fragment EcoRI-BglII de 5.5 kb, dont une partie a été séquencée (SEQ ID n° 5). Le gène snbR a été sous cloné dans un fragment BglII-BglII de 4.6 kb, dont une partie a été séquencée (SEQ ID n° 6). Une partie du gène papA a été sous clonée dans un fragment XhoI-XhoI de 3,4 kb dont une partie a été séquencée (SEQ ID n° 9). Le gène papM a été sous cloné dans un fragment PstI-PstI de 4,1 kb dont une partie a été séquencée (SEQ ID n° 10). Une partie du gène snaD a été sous clonée dans un fragment BamHI-SstI de 1,5 kb dont une partie a été séquencée (SEQ ID n° 8). Une partie du gène snbC a été sous clonée sur un fragment SphI-SphI de 6,2 kb dont 2 régions ont été séquencées (SEQ ID n° 11 et 12). Une partie du gène snbD a été sous clonée sur un fragment SphI-SphI de 8,4 kb dont 2 régions ont été séquencées (SEQ ID n° 13 et 14). Une partie du gène snbE a été sous clonée sur un fragment SphI-SphI de 6,6 kb dont 2 régions ont été séquencées (SEQ ID n° 15 et 16). Le gène snaC- a été sous cloné dans un fragment BamHI-BamHI de 4 kb dont une partie a été séquencée (SEQ ID n° 7).

La proximité des gènes snaA, snaB, snaD, samS, snbC, snbD et snbE d'une part, ainsi que des gènes snbA, snbR, papA et papM confirme la localisation en clusters des gènes de biosynthèse des composants A et B des Streptogramines. De plus, les 4 cosmides décrits dans la présente invention sont regroupés dans une région du chromosome d'une taille estimée à 200 kb par électrophorèse à champ pulsé, soit 3% du génome total (7500 kb) de Streptomyces pristinaespiralis (exemple 13). Il est donc évident que les régions qui entourent les gènes identifiés dans la présente invention (snaA, snaB, snaD, samS, snbC, snbD et snbE; snbA, snbR, papA et papM; snaC), contiennent les autres gènes du cluster de biosynthèse des Pristinamycines, et que ces gènes peuvent être utilisés pour localiser les autres gènes de biosynthèse des Streptogramines.

Préférentiellement, l'invention a pour objet une séquence nucléotidique choisie parmi :
(a) tout ou partie des gènes snaA (SEQ ID n° 2), snaB (SEQ ID n° 3), snaC (SEQ ID n° 7), snaD (SEQ ID n° 8), papA (SEQ ID n° 9), papM (SEQ ID n° 10), samS (SEQ ID n° 4), snbA (SEQ ID n° 5), snbC (SEQ ID n° 11 et 12), snbD (SEQ ID n° 13 et 14), snbE (SEQ ID n° 15 et 16) et snbR (SEQ ID n° 6),
(b) les séquences adjacentes aux gènes (a) constituant les clusters de biosynthèse et codant pour les polypeptides impliqués dans la biosynthèse des Streptogramines,
(c) les séquences hybridant avec tout ou partie des gènes (a) ou (b) et codant pour un polypeptide impliqué dans la biosynthèse des Streptogramines, et,
(d) les séquences dérivées des séquences (a), (b) et (c) en raison de la dégénérescence du code génétique.

Encore plus préférentiellement, l'invention a pour objet les séquences nucléotidiques représentées par les gènes snaA (SEQ ID n° 2), snaB (SEQ ID n° 3), snaC (SEQ ID n° 7), snaD (SEQ ID n° 8), papA (SEQ ID n° 9), papM (SEQ ID n° 10), samS (SEQ ID n° 4), snbA (SEQ ID n° 5), snbC (SEQ ID n° 11 et 12), snbD (SEQ ID n° 13 et 14), snbE (SEQ ID n° 15 et 16) et snbR (SEQ ID n° 6).

Un autre objet de l'invention concerne tout ADN recombinant comprenant un gène de biosynthèse des Streptogramines. Plus préférentiellement, il s'agit d'un ADN recombinant comprenant tout ou partie des cosmides pIBV1, pIBV2, pIBV3 ou pIBV4 tels que représentés sur les figures 4 à 7 ou tout ou partie de séquences hybridant avec les cosmides pIBV1 à pIBV4 ou avec des fragments de ceux-ci.

Dans un mode préféré de l'invention, les séquences nucléotidiques définies ci-dessus font partie d'un vecteur d'expression, qui peut être à réplication autonome ou intégratif.

Comme indiqué plus haut, bien que l'invention soit plus particulièrement illustrée avec les gènes de biosynthèse de la Pristinamycine, il est clair que les résultats obtenus s'appliquent à l'ensemble des Streptogramines.

Plus particulièrement, les techniques développées dans la présente invention pour purifier des protéines ou cloner des gènes de biosynthèse des Streptogramines à partir de S. pristinaespiralis peuvent être appliquées aux autres microorganismes producteurs de Streptogramines (Cf tableau 1).

Ainsi, la purification d'une activité enzymatique à partir de S. pristinaespiralis rend possible la purification de la même activité à partir d'une autre souche productrice de Streptogramine. La présente invention peut donc être appliquée au clonage de gènes de biosynthèse de Streptogramines à partir de tout microorganisme producteur par purification d'une protéine intervenant dans la biosynthèse puis, à l'aide de la séquence NH₂-terminale de celle-ci, synthèse d'une sonde oligonucléotidique qui permet de cloner le gène correspondant. Ensuite une marche sur le chromosome permet d'identifier le cluster de biosynthèse en entier.

De plus, à partir des gènes identifiés dans la présente demande, il est possible, par hybridation, de cloner directement les gènes de biosynthèse des Streptogramines à partir de l'ADN d'un autre microorganisme producteur. En effet, les gènes de biosynthèse des Pristinamycines hybrident fortement à ceux des autres Streptogramines. Il est ainsi possible de cloner par hybridation les gènes de biosynthèse des Streptogramines en utilisant comme sonde les gènes sna, snb ou pap, ou des fragments de ceux-ci, ou les fragments adjacents à ceux-ci contenant, comme il est montré dans la présente invention, d'autres gènes sna et snb. Ceci résulte du fait que: 1) les Streptogramines produites par les différents microorganismes ont des structures identiques ou similaires (voir figures 1-3), 2) les gènes de biosynthèse des Streptogramines sont organisés en clusters, et 3) les systèmes enzymatiques responsables de cette biosynthèse n'ont pas une spécificité absolue pour leurs substrats.

Par ailleurs, le clonage de gènes impliqués dans la biosynthèse des Streptogramines peut aussi se faire en utilisant des oligonucléotides dégénérés, préparés à partir des séquences des gènes sna ou snb cités plus haut, ou des fragments de ces gènes, ou des fragments contigus à ces gènes. Il est ainsi possible d'aller piocher les gènes de biosynthèse des composants A et B des différentes souches productrices de Streptogramines. Ces souches peuvent faire partie du genre Streptomyces, mais aussi d'autres genres (Cf tableau 1). En outre, si l'ADN génomique des souches de départ utilisées a une composition en G+C différente de celle observée chez les Streptomyces les sondes utilisées peuvent être synthétisées avec un biais de codon spécifique du genre ou de l'espèce à partir duquel on veut isoler l'ADN.

Un autre objet de la présente invention concerne les polypeptides résultant de l'expression des séquences nucléotidiques définies ci-dessus. Plus particulièrement, la présente invention concerne les polypeptides comprenant tout ou partie des polypeptides SnaA (SEQ ID n° 2), SnaB (SEQ ID n° 3), SnaC (SEQ ID n° 7), SnaD (SEQ ID n° 8), PapA (SEQ ID n° 9), PapM (SEQ ID n° 10), SamS (SEQ ID n° 4), SnbA (SEQ ID n° 5), SnbC (SEQ ID n° 11 et 12), SnbD (SEQ ID n° 13 et 14), SnbE (SEQ ID n° 15 et 16) et SnbR (SEQ ID n° 6) ou de dérivés de ceux-ci. Au sens de la présente invention, le terme dérivé désigne toute molécule obtenue par modification de nature génétique et/ou chimique de la séquence peptidique. Par modification de nature génétique et/ou chimique, on peut entendre toute mutation, substitution, délétion, addition et/ou modification d'un ou plusieurs résidus. De tels dérivés peuvent être générés dans des buts différents, tels que notamment celui d'augmenter l'affinité du peptide pour son(ses) substrat (s), celui d'améliorer ses niveaux de production, celui d'augmenter sa résistance à des protéases, celui d'augmenter et/ou de modifier son activité, ou celui de lui conférer de nouvelles propriétés biologiques. Parmi les dérivés résultant d'une addition, on peut citer par exemple les polypeptides chimères comportant une partie hétérologue supplémentaire liée à une extrêmité. Le terme dérivé comprend également les polypeptides homologues aux polypeptides décrits dans la présente invention, issus d'autres sources cellulaires et notamment de souches productrices de Streptogramines.

L'invention a également pour objet toute cellule recombinante contenant une séquence nucléotidique ou un vecteur tel que défini ci-avant. Les cellules recombinantes selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre Saccharomyces, Kluyveromyces, Pichia, Schwanniomyces, ou Hansenula. S'agissant de cellules animales, on peut citer les cellules COS, CHO, C127, les oeufs de Xénope, etc. Parmi les champignons, on peut citer plus particulièrement Micromonospora, Aspergillus ssp. ou Trichoderma ssp. Comme cellules procaryotes, on préfère utiliser les bactéries suivantes Actinomycètes, et notamment Streptomyces, E.coli (exemple 11), Bacillus. Préférentiellement, les cellules recombinantes de l'invention sont choisies parmi les cellules productrices de Streptogramines (Cf tableau 1). Les cellules recombinantes de l'invention peuvent être obtenues par toute méthode permettant d'introduire une séquence nucléotidique étrangère dans une cellule. Il peut s'agir notamment de transformation, électroporation, conjugaison, fusion de protoplastes, ou toute autre technique connue de l'homme de l'art

L'invention a encore pour objet un procédé de production d'un polypeptide impliqué dans la biosynthèse des Streptogramines selon lequel on cultive une cellule recombinante telle que définie ci-avant et on récupère le polypeptide produit.

L'invention a également pour objet l'utilisation d'une cellule recombinante telle que définie ci-avant exprimant un polypeptide au moins impliqué dans la biosynthèse des Streptogramines, dans une réaction de bioconversion. En particulier, ces cellules peuvent permettre de transformer une Streptogramine en une forme dérivée. Par exemple, la Pristinamycine IIB peut être, de cette manière, transformée en Pristinamycine IIA. Le même raisonnement peut être appliqué à tout intermédiaire de biosynthèse. Ces cellules peuvent également permettre de fabriquer des antibiotiques hybrides ayant des propriétés pharmacologiquement intéressantes (Hopwood et al. 1985a, Hopwood et al. 1985b, Hutchinson et al. 1989). Ces bioconversions peuvent se faire soit à l'aide de cellules entières, soit à l'aide d'extraits acellulaires desdites cellules.

Un autre objet de l'invention concerne l'utilisation d'une séquence nucléotidique telle que définie ci-avant pour amplifier la production de Streptogramine. L'invention concerne également un procédé de production de Streptogramines selon lequel on introduit et/ou on amplifie dans une cellule productrice de Streptogramines ou potentiellement productrice de Streptogramines une ou plusieurs séquences nucléotidiques selon l'invention, on cultive ladite cellule dans des conditions de production des Streptogramines, et on récupère les Streptogramines produites. -

La surexpression de certains gènes impliqués dans la biosynthèse peut permettre l'augmentation de la production des Streptogramines A et/ou B des souches productrices. Cette surproduction peut se faire dans plusieurs souches : soit des souches qui ne produisent que des molécules de la famille des Streptogramines A, soit des souches qui ne produisent que des molécules de la famille des Streptogramines B, soit des souches qui produisent les deux composants A et B. Ces surexpressions peuvent résulter de l'augmentation du taux de synthèse, donc de la productivité, des composants A et/ou B soit en erlenmeyer, soit en petits fermenteurs, soit en gros fermenteurs industriels. Par ailleurs, la surexpression spécifique d'un gène impliqué dans la biosynthèse d'un composant A ou B permet également de faire varier le % de composants A et B produits par la souche, et ainsi d'obtenir une meilleure synergie entre ces molécules. En outre, les gènes de biosynthèse isolés à partir d'un microorganisme producteur de Streptogramines peuvent être utilisés pour amplifier la production dans un autre microorganisme producteur.

Un autre objet de l'invention concerne un procédé de préparation de cellules bloquées dans une étape de la voie de biosynthèse des Streptogramines selon lequel on effectue, sur une cellule productrice de Streptogramines, une mutagénèse au niveau d'un gène au moins de la voie de biosynthèse.

Préférentiellement, la mutagénèse est effectuée in vitro ou in situ, par suppression, substitution, délétion et/ou addition d'une ou plusieurs bases dans le gène considéré, ou par disruption génique.

Un autre aspect de la présente invention réside en effet dans la construction de mutants bloqués dans certaines étapes de biosynthèse des Streptogramines. L'intérêt réside d'une part dans l'étude de la fonctionnalité des protéines mutées et d'autre part dans la réalisation de souches produisant des intermédiaires de biosynthèse. Ces intermédiaires peuvent être modifiés, éventuellement après séparation, soit par ajout de composants particuliers dans les milieux de production, soit par introduction dans les souches ainsi mutées d'autres gènes susceptibles de modifier l'intermédiaire en s'en servant de substrat. Ces intermédiaires peuvent ainsi être modifiés par voie chimique, biochimique, enzymatique et/ou microbiologique. Dans ce cadre, le mutant SP92::pVRC505 de la souche S. pristinaespiralis SP92 a été construit : S. pristinaespiralis SP92::pVRC505 a été isolée par intégration homologue dans le gène snaA d'un plasmide suicide pVRC505, construit à partir du vecteur pDH5 et d'un fragment interne au gène snaA. Les mutants suivants ont également été construits : SP92 samS::ΩamR; SP92::pVRC508: SP92::pVRC404 et SP92::pVRC1000 (exemple 9).

L'invention concerne donc également un procédé de préparation d'un intermédiaire de biosynthèse des Streptogramines selon lequel :
- on prépare une cellule bloquée dans une étape de la voie de biosynthèse des Streptogramines telle que décrite ci-avant,
- on cultive ladite cellule, et
- on récupère l'intermétliaire accumulé.

L'invention concerne également un procédé de préparation d'une molécule dérivée des Streptogramines selon lequel :
- on prépare une cellule bloquée dans une étape de la voie de biosynthèse des Streptogramines telle que décrite ci-avant,
- on cultive ladite cellule, et,
- on modifie l'intermédiare accumulé par cette cellule, éventuellement après séparation du milieu de culture.

La présente invention est illustrée à l'aide des exemples qui suivent, qui doivent être considérés comme illustratifs et non limitatifs.

### LISTE DES FIGURES.

Figure 1 : Exemple de structure des composants A des Streptogramines.

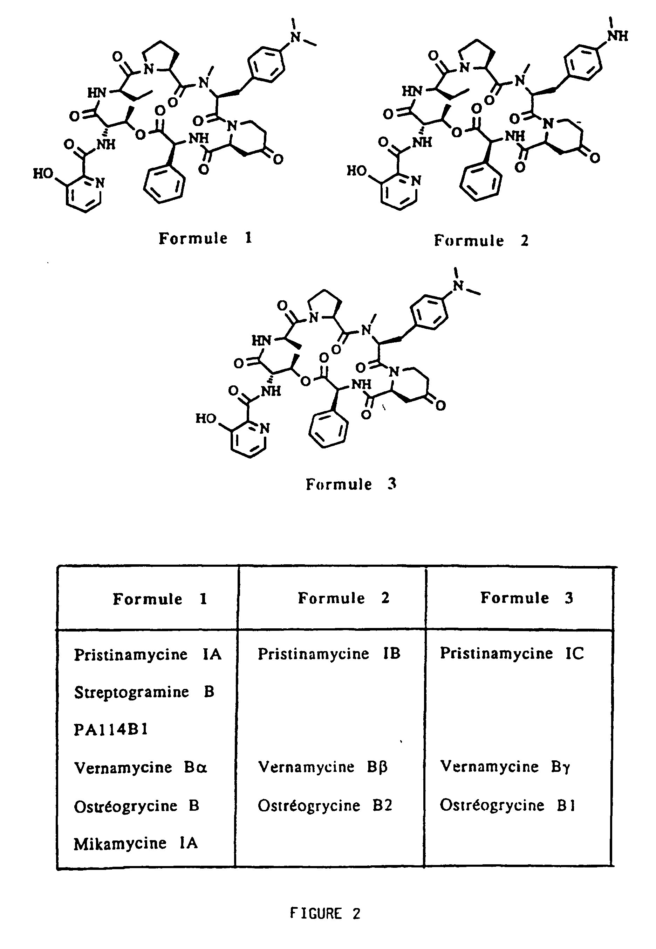

Figure 2 : Exemple de structure des composants B des Streptogramines.

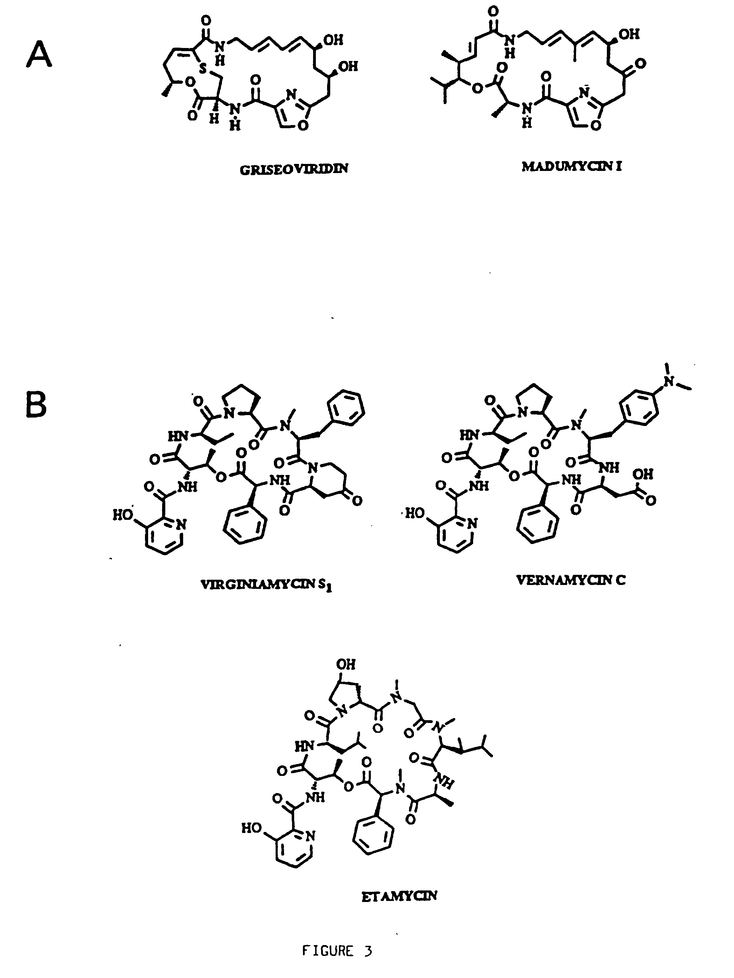

Figure 3 : Autres exemples de structures de Streptogramines.
Figure 4 : Représentation du cosmide pIBV1.
Figure 5 : Représentation du cosmide pIBV2.
Figure 6 : Représentation du cosmide pIBV3.
Figure 7 : Représentation du cosmide pIBV4.

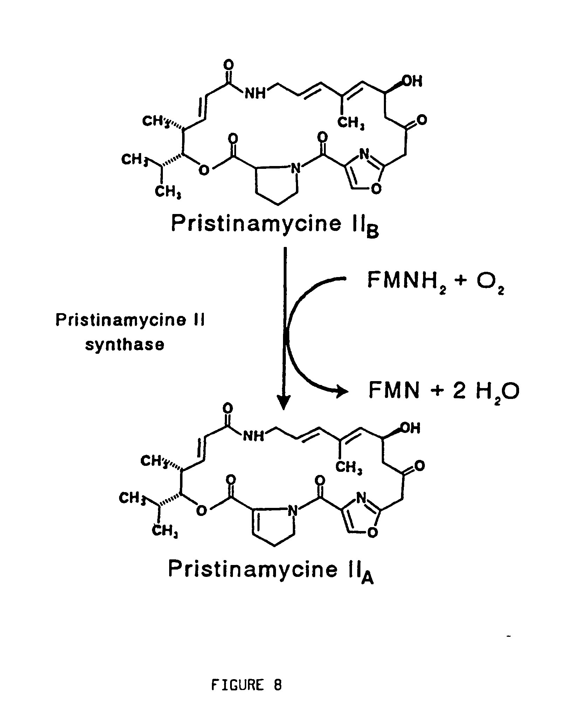

Figure 8 : Réaction catalysée par la Pristinamycine IIA synthase.

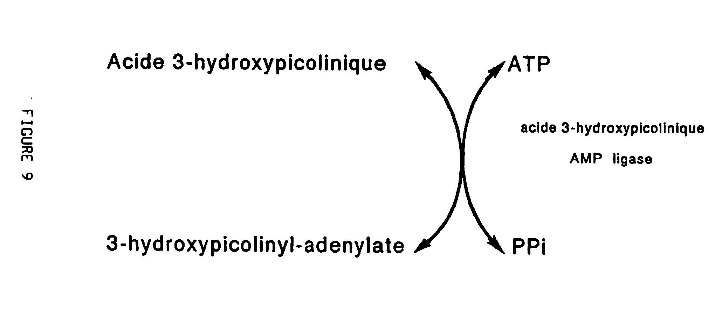

Figure 9 : Réaction catalysée par l'acide 3-hydroxypicolinique-AMP ligase.

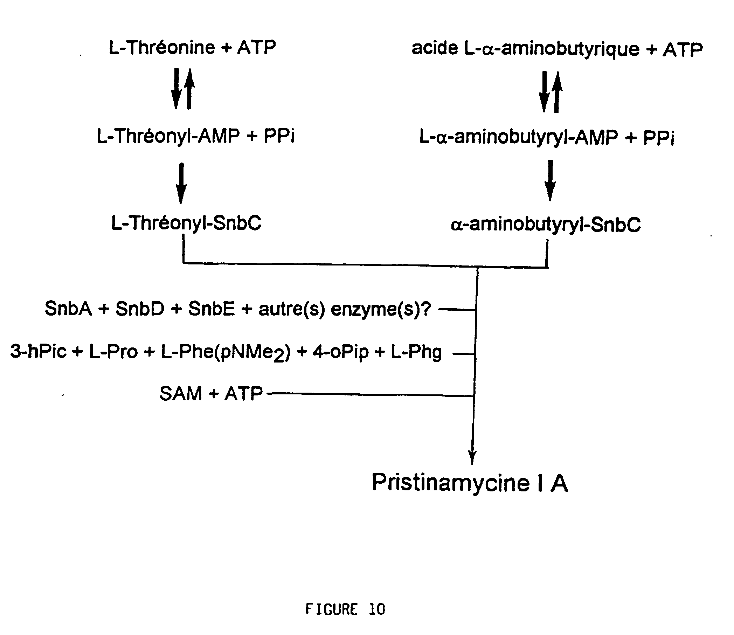

Figure 10 : Réaction catalysée par SnbC.

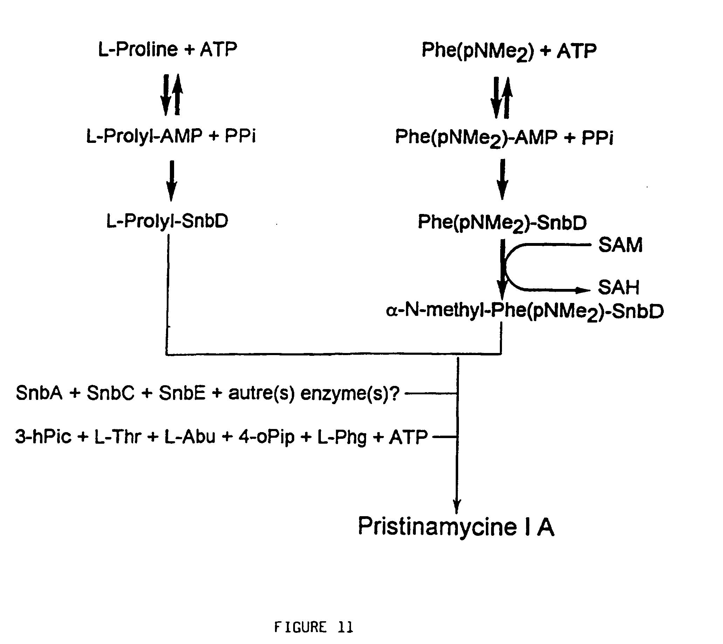

Figure 11 : Réaction catalysée par SnbD.

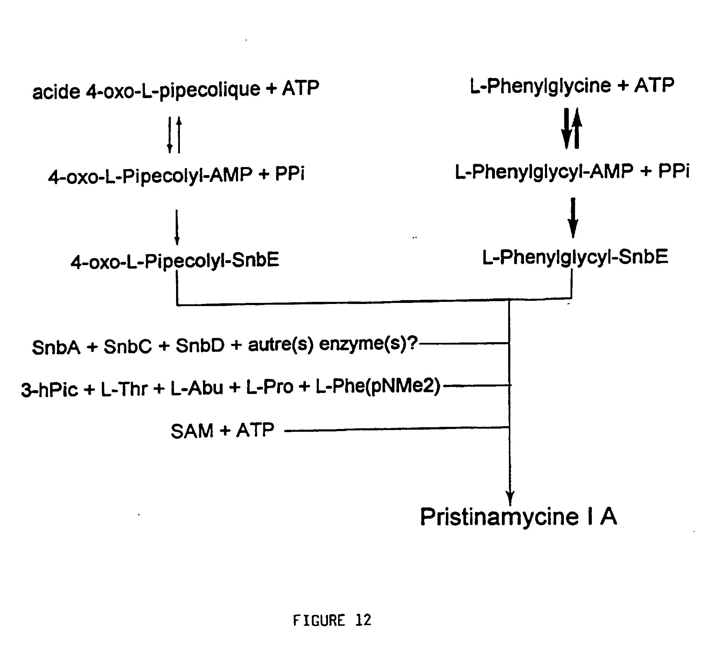

Figure 12 : Réaction catalysée par SnbE.

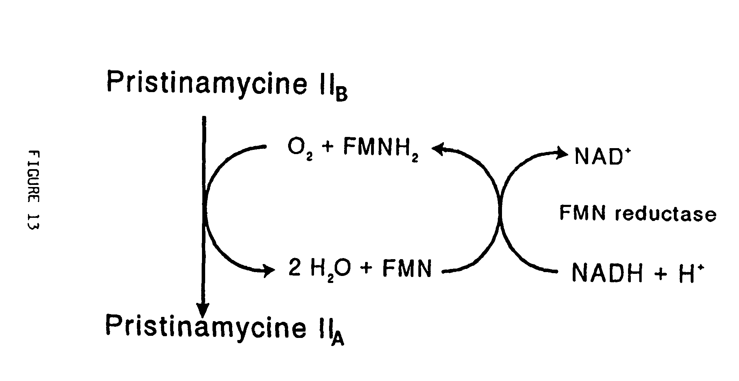

Figure 13 : Réaction catalysée par SnaC.

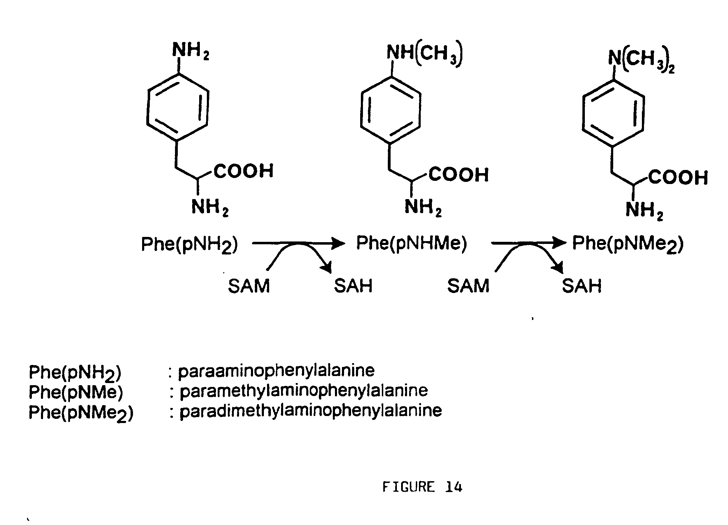

Figure 14 : Réaction catalysée par PapM.

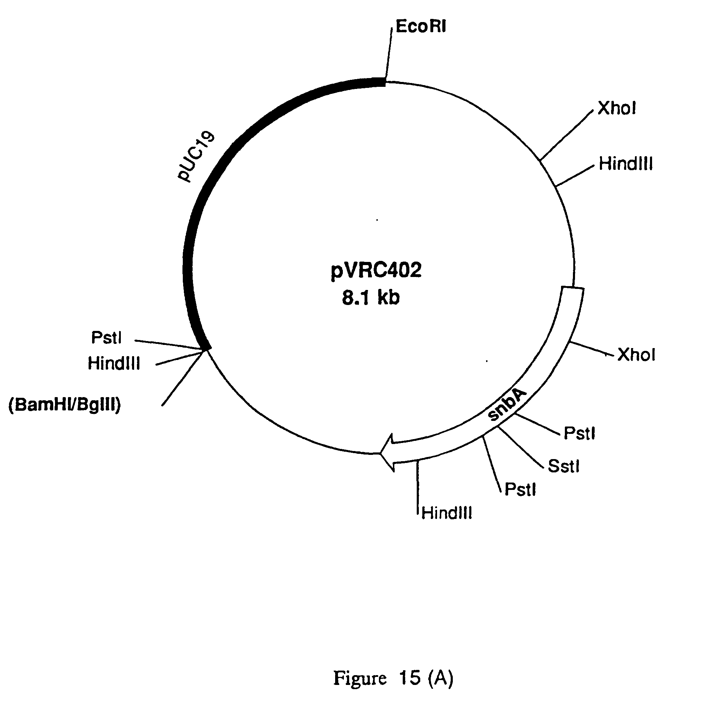

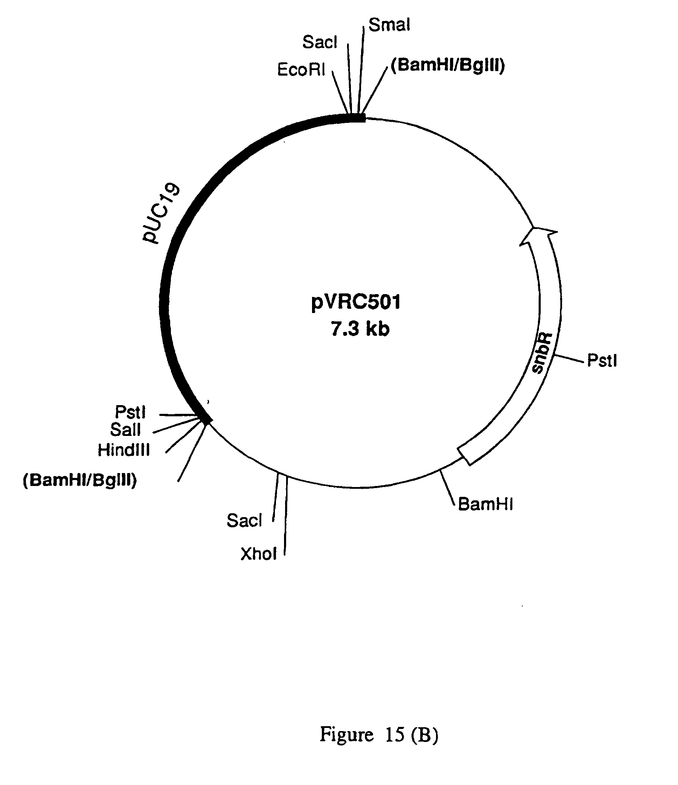

Figure 15 : Représentation des plasmides pVRC402 (A) et pVRC501 (B).

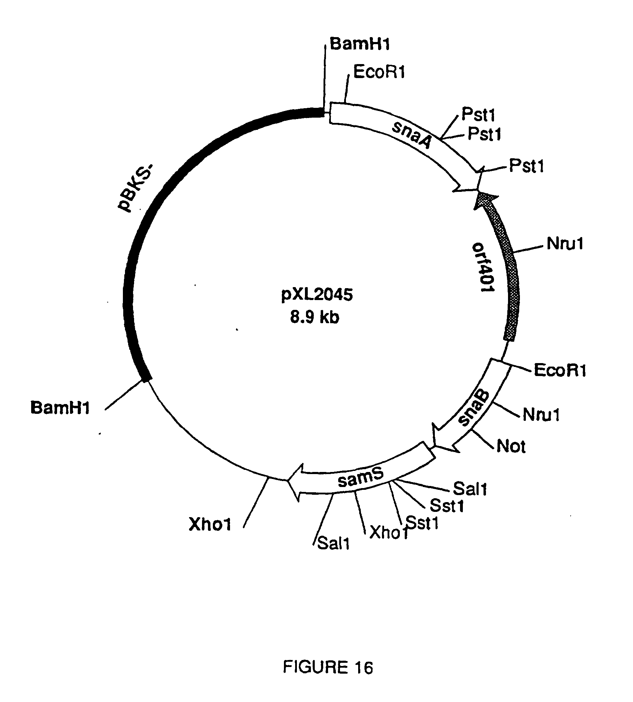

Figure 16 : Représentation du plasmide pXL2045.

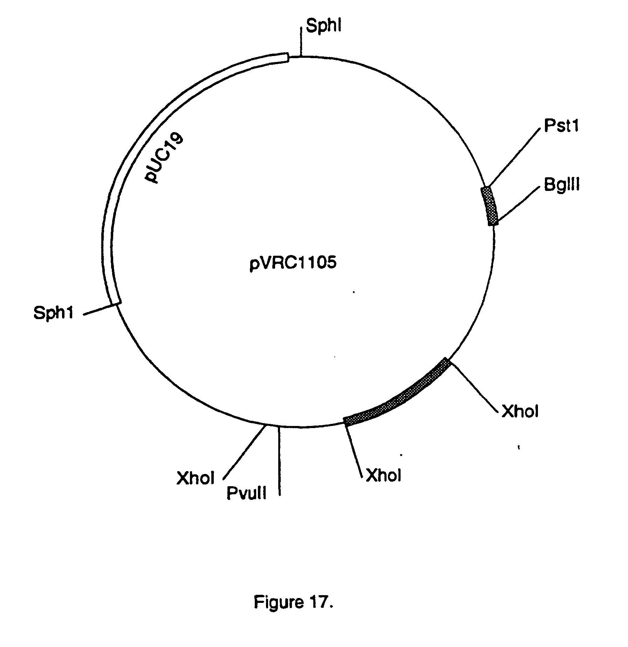

Figure 17 : Représentation du plasmide pVRC1105.

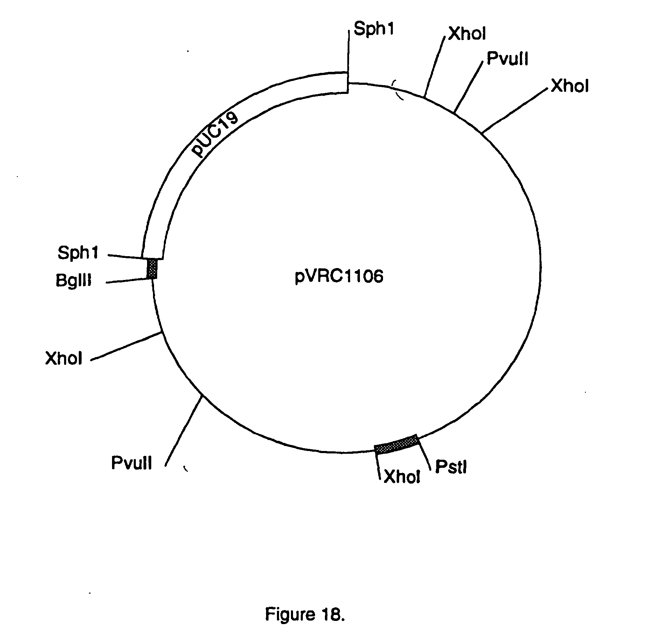

Figure 18 : Représentation du plasmide pVRC1106.

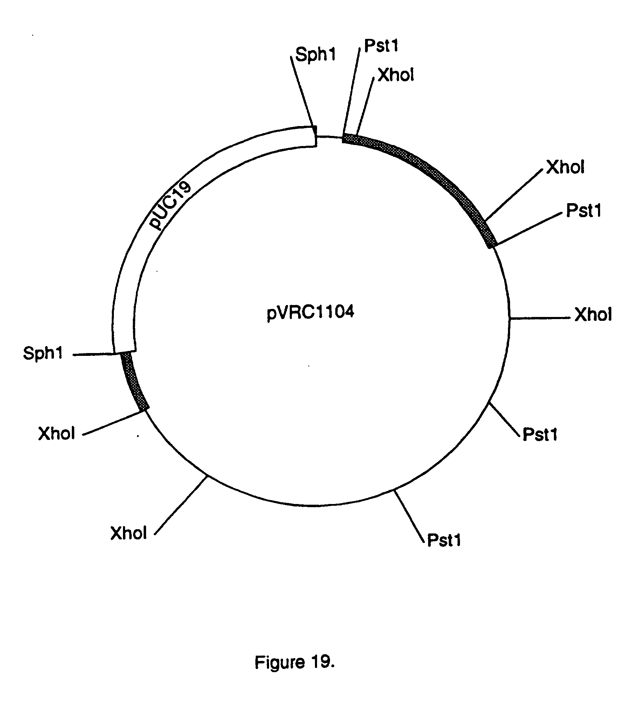

Figure 19 : Représentation du plasmide PVRC1104.

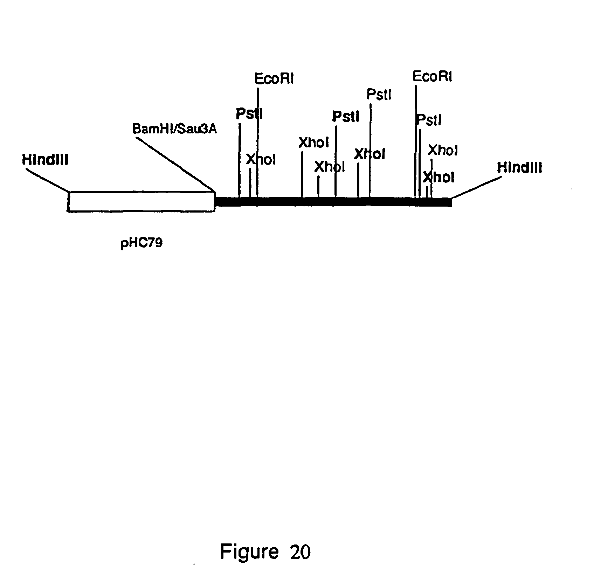

Figure 20 : Représentation du plasmide pVRC900.

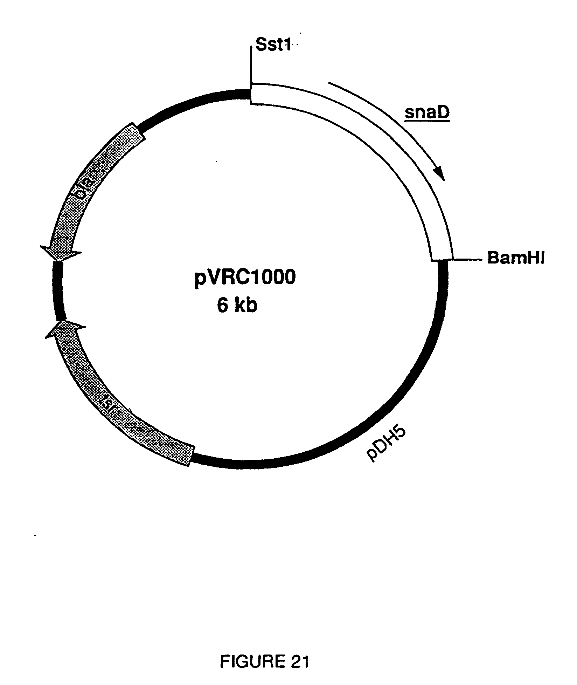

Figure 21 : Représentation du plasmide pVRC1000.

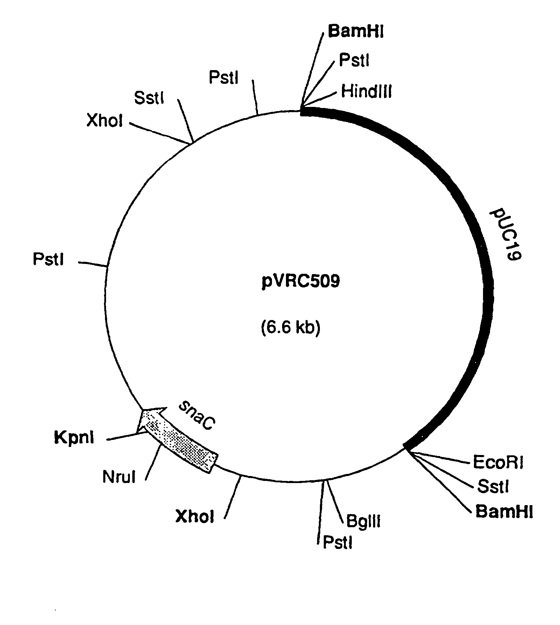

Figure 22 : Représentation du plasmide pVRC509.

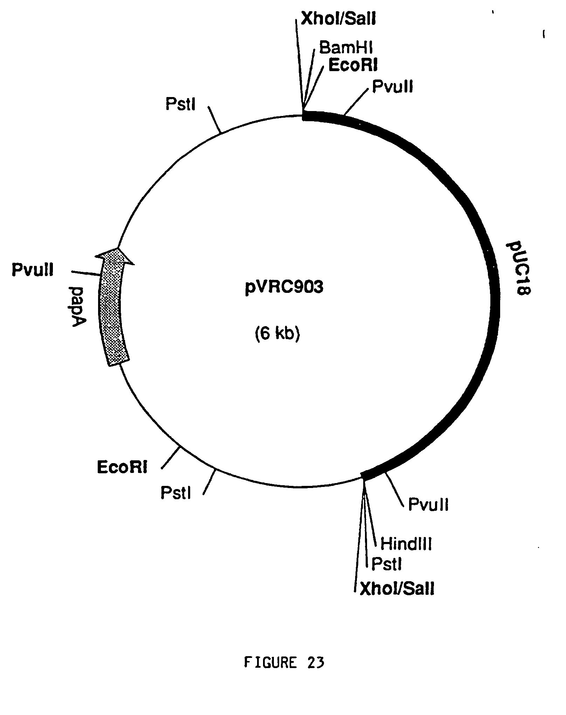

Figure 23 : Représentation du plasmide pVRC903.

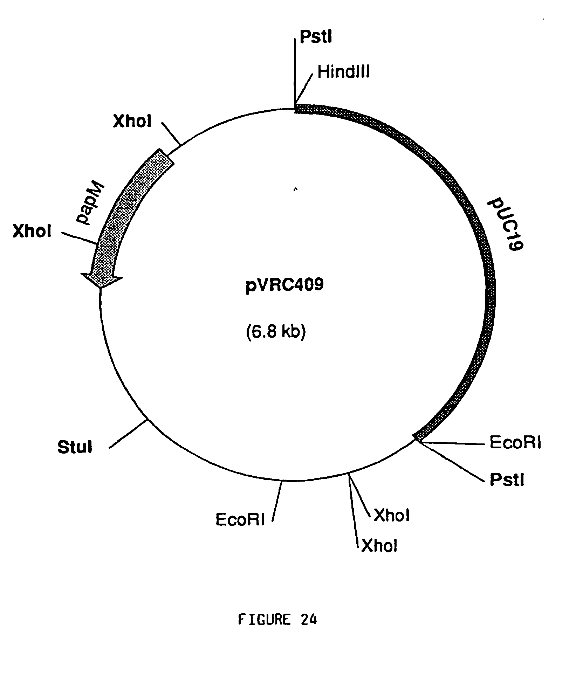

Figure 24 : Représentation du plasmide pVRC409.

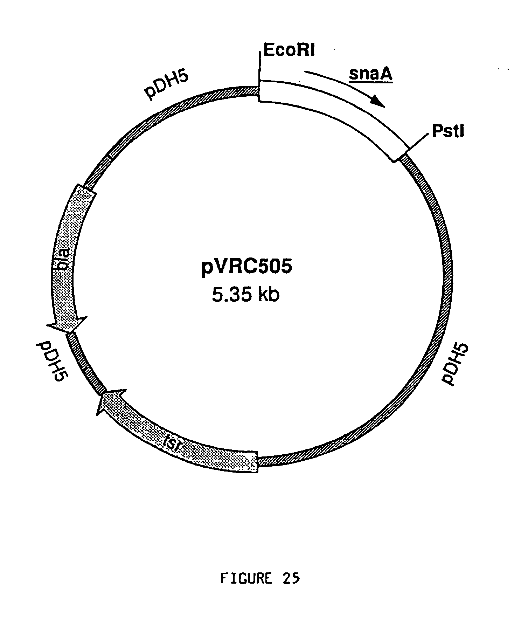

Figure 25 : Représentation du plasmide pVRC505.

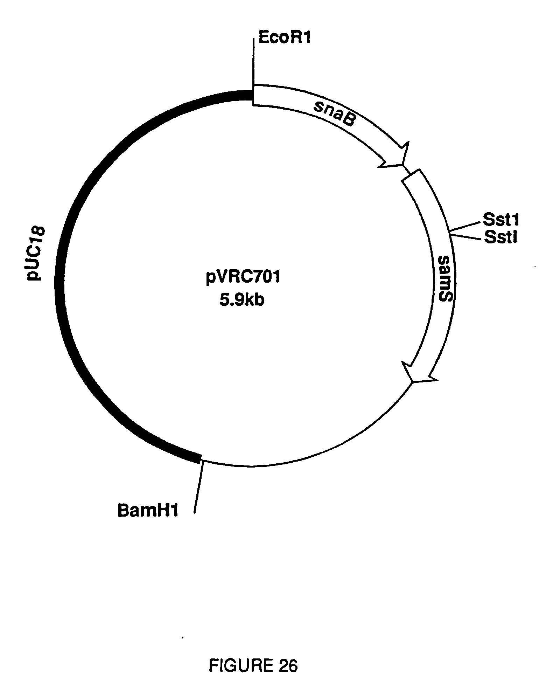

Figure 26 : Représentation du plasmide pVRC701.

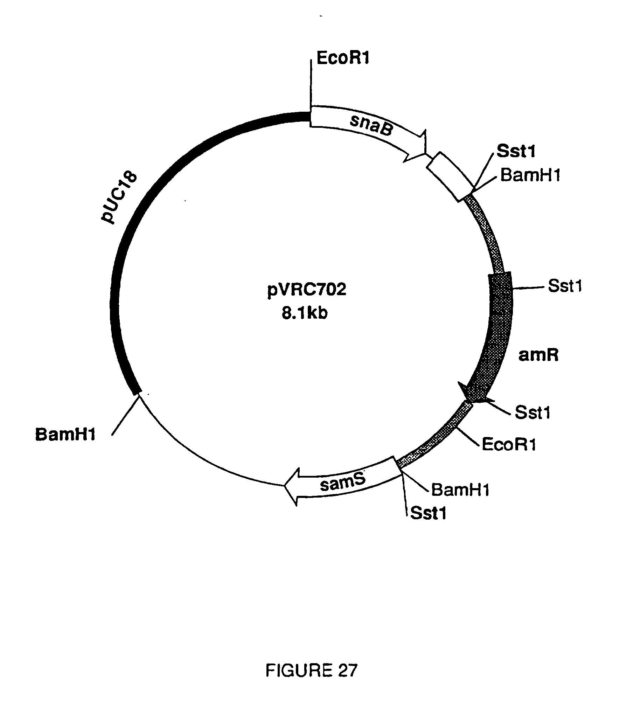

Figure 27 : Représentation du plasmide pVRC702.

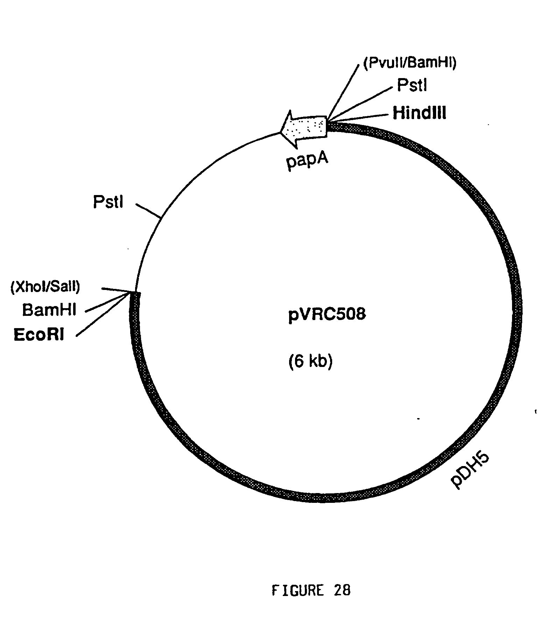

Figure 28 : Représentation du plasmide pVRC508.

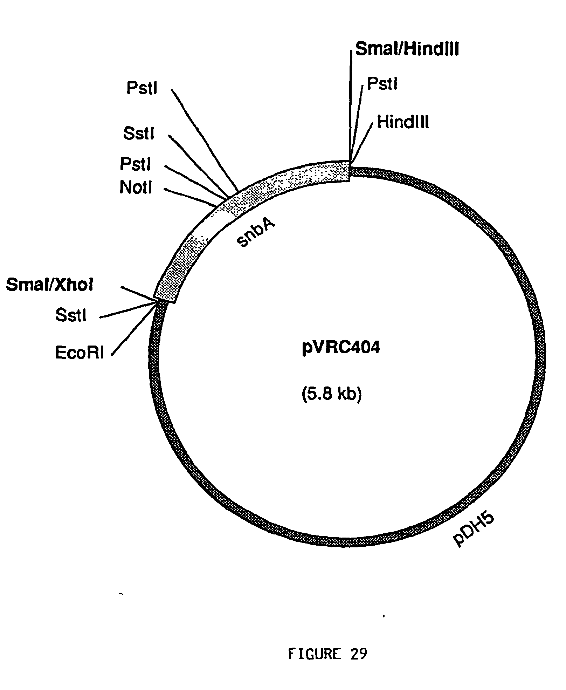

Figure 29 : Représentation du plasmide pVRC404.

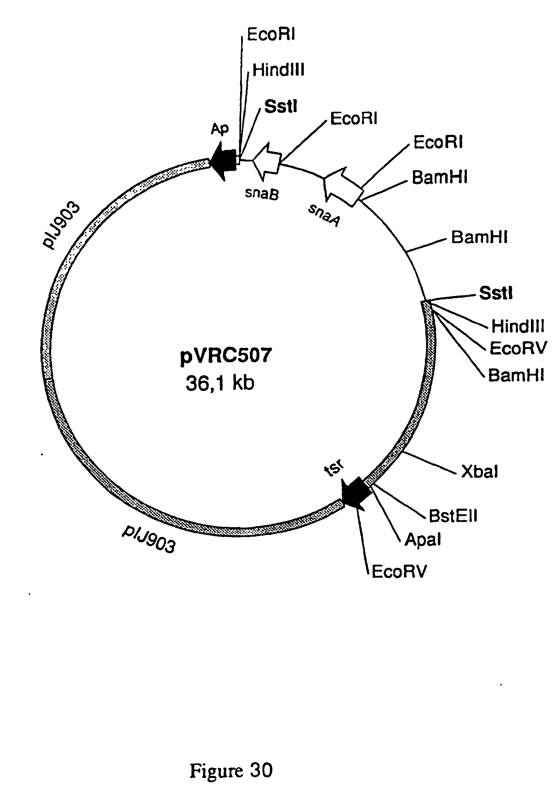

Figure 30 : Représentation du plasmide pVRC507.

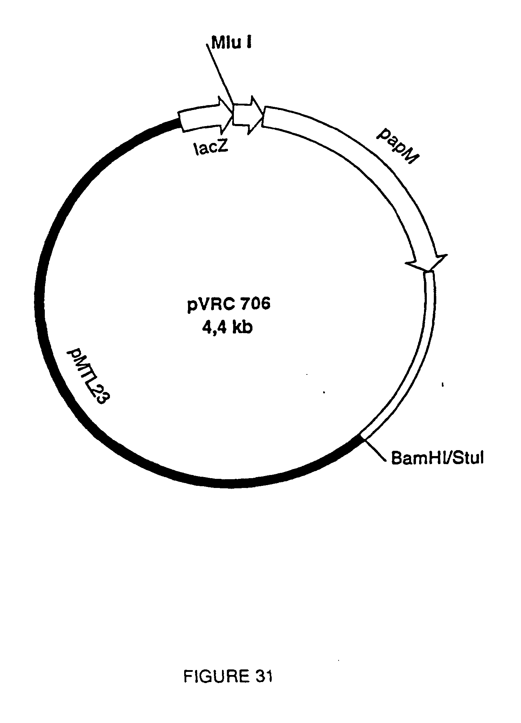

Figure 31 : Représentation du plasmide pVRC706.

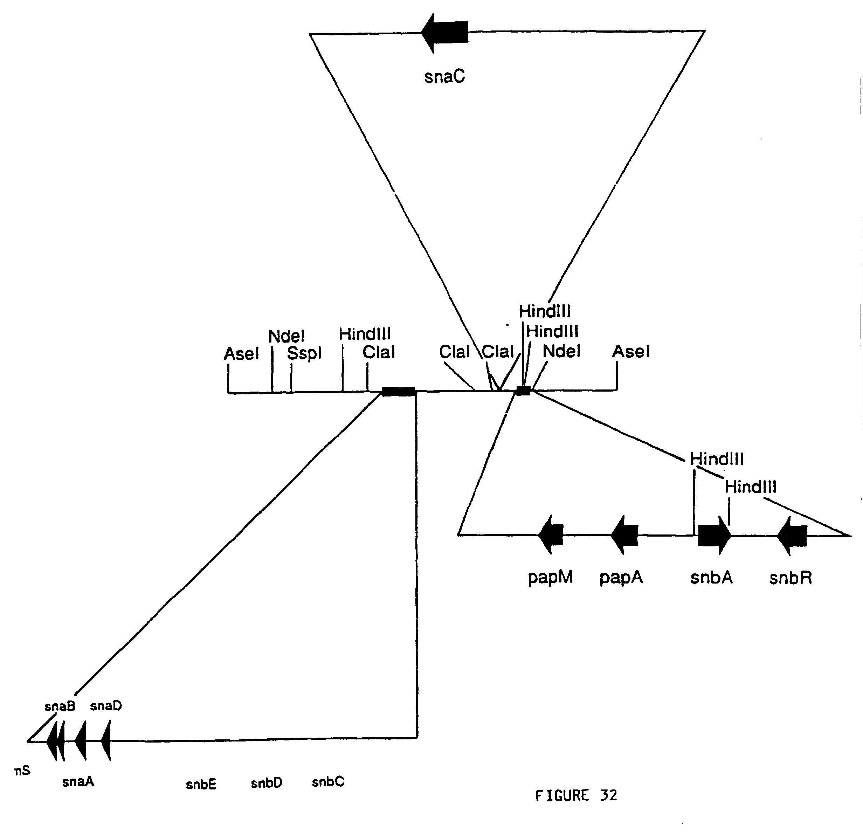

Figure 32 : Cartographie générale.

### MATERIELS.

Colonne Bio-Sil SEC 125 et 250 (Bio-Rad)
Colonne MonoQ HR 5/5,10/10 et 16/10 (Pharmacia)
Colonne PD-10 (Pharmacia)
Colonne Superose 6 HR 10/30 (Pharmacia)
Colonne Superdex 200 Hi-Load 16/60 et 75 HR 10/30 (Pharmacia)
Colonne Superose 12 prep grade (Pharmacia)
Colonne Vydac C4 et C18 (The Separations Group)
Colonne Nucleosil 5-C18 (Macherey-Nagel)
Colonne Phenyl Superose HR 10/10 (Pharmacia)
Colonne TSK G2000 SW (Tosoh, Japon)
Phenyl Sepharose (Pharmacia)
FMN agarose (Sigma)
Q-Sepharose Fast Flow (Pharmacia)
Sephadex G25 Fine (Pharmacia)
Centricon 10 ou 30 (Amicon)
Centriprep 10 ou 30 (Amicon)
Centrilutor (Amicon)

### EXEMPLE 1 : Isolement de l'ADN total de la souche Streptomyces pristinaespiralis SP92.

Cet exemple illustre comment l'ADN de S. pristinaespiralis SP92 peut être purifié.

La souche S. pristinaespiralis SP92 dérive de la souche S. pristinaespiralis DS5647 (ATCC25486).

50 ml de milieu YEME (34 % de sucrose, 5 mM MgCl₂, glycine 0.25 % (D. Hopwood et al. 1985)) sont inoculés avec 10⁸ spores de S.pristinaespiralis SP92 et la culture est incubée 40 heures à 30°C, sous agitation de 280 tours/mn.

Le mycélium est récolté et lavé avec 15 ml de saccharose 10.3 %. Environ 1 g du culot de mycélium est repris par 5 ml de TE complémenté par 34 % de sucrose, auxquels sont ajoutés, 1 ml de lysozyme à 50 mg/ml dans une solution de Tris-HCl 10 mM, pH 8,0 et 1 ml d'EDTA 0.25 M pH 8,0. Après incubation à 30°C pendant une durée de 30 à 60 min, le mélange est éclairci par ajout de 0.8 ml de sarkosyl 10 %. Puis sont ajoutés, 2 ml d'EDTA 0.25 M pH 8,0, 10 ml de TE, 18 g de CsCl et 1.2 ml de BET à 10 mg/ml. La préparation est ultracentrifugée pendant une nuit à 55 000 tours/min, à 20°C.

L'ADN chromosomique présent dans le gradient de CsCl sous forme d'une bande, est récupéré à l'aide d'une pipette Pasteur. Le BET est éliminé par plusieurs lavages avec une solution d'isopropanol saturée par du tampon TE, 5 M NaCl. L'ADN est précipité par ajout de 3 volumes de TE et 4 volumes d'isopropanol. Après lavage à l'éthanol 70 %, l'ADN est repris dans un volume approprié de TE. La quantité d'ADN total obtenu varie entre 250 et 500 µg par g de mycélium.

### EXEMPLE 2 : Isolement d'ADN plasmidique d'E. coli :

Cet exemple illustre comment l'ADN plasmidique d'E. coli est préparé à partir des souches d'E. coli recombinantes.

### 2.1. Préparation d'ADN plasmidique d'E.coli en grosses quantités.

Cet exemple illustre comment les maxipréparations d'ADN plasmidique sont réalisées chez E. coli.

Cette préparation est effectuée à partir d'une culture de 500 ml en milieu LB contenant 150 µg/ml d'ampicilline. Le protocole d'extraction est dérivé des méthodes décrites par Birnboim et Doly (1979) et Ish-Horowicz et Burke (1981) et est décrit dans Maniatis et al. (1989).

Après cette extraction, l'ADN plasmidique est purifié par gradient de CsCl, comme décrit dans Maniatis et al. (1989). L'ADN plasmidique est ensuite précipité par ajout de 3 volumes de TE et 4 volumes d'isopropanol. Après centrifugation, le culot est repris dans 0.5 à 1 ml de TE.

### 2.2. Préparation d'ADN plasmidique d'E. coli. en petites quantités.

Cet exemple illustre comment les minipréparations d'ADN plasmidique sont réalisées chez E.coli.

Cette préparation est réalisée à partir de 1.5 ml de culture en milieu LB contenant 150 µg/ml d'ampicilline. La procédure est celle décrite par Birnboim et Doly (1979).

### EXEMPLE 3 : Construction de la banque d'ADN génomique de S. pristinaespiralis SP92 chez E. coli et préparation des membranes d'hybridation.

Cet exemple illustre comment une banque d'ADN génomique de S.pristinaespiralis SP92 est réalisée chez E.coli.

### 3.1. Préparation des fragments d'ADN génomique.

Cet exemple illustre comment des fragments d'ADN génomique de haut poids moléculaire peuvent être préparés.

L'ADN total de la souche SP92, préparé comme décrit dans l'exemple 1, est digéré partiellement par Sau3A (New England Biolabs, Beverly, MA. 01915-5510 USA) dans le tampon préconisé par le fournisseur : 100 mM NaCl, 10 mM Tris-HCl (pH7.5), 10 mM MgCl₂, 100 µg/ml BSA. La quantité d'enzyme utilisée pour obtenir des fragments d'ADN de haut poids moléculaire, a été déterminée empiriquement. Environ 0.025 unités d'enzyme sont utilisées pour digérer 1 µg d'ADN total pendant 20 min à 37°C. La réaction est ensuite arrêtée par une incubation de 15 min à 65°C et l'enzyme éliminée par l'addition d'un volume égal de phénol-chloroforme. Après centrifugation, le surnageant contenant l'ADN total partiellement digéré est précipité par ajout d'acétate de sodium 0.3 M final et 2.5 volumes d'éthanol.

Environ 100 µg d'ADN total sont ainsi digérés, puis les fragments d'ADN de taille comprise entre 30 et 50 kb sont isolés par gradient de sucrose 10-40 %. Leur taille est vérifiée par électrophorèse sur gel d'agarose à 0,4 %.

### 3.2. Préparation du cosmide pHC79.

Cet exemple illustre comment le cosmide pHC79 est préparé à partir d'E. coli.

Le cosmide pHC79 (Hohn, B. and Collins, 1980) comprend une partie de pBR322 (Bolivar, F. et al., 1977), la région cro-cII de λ et la région contenant la séquence cos de Charon 4A (Blattner, F.R. et al., 1977).

L'extraction du cosmide a été réalisée comme décrit dans l'exemple 2.1., à partir d'une souche d'E.coli TG1 (K12, Δ(lac-pro) supE thi hsd DS F traD36 proA⁺B⁺ lacI^{q} LacZ ΔM15, Gibson, 1984).

500 ng de cosmide pHC79 ont été digérés par BamHI (New England Biolabs, Beverly, MA. 01915-5510 USA) dans 20 µl de tampon 150 mM NaCl, 6 mM Tris-HCl pH 7,9, 6 mM MgCl₂, 6 mM 2-mercaptoéthanol, 100 µg/ml BSA.

### 3.3. Ligation des fragments d'ADN et du cosmide.

Cet exemple illustre comment les fragments du génome de S. pristinaespiralis SP92, issus d'une digestion Sau3A, peuvent être ligués avec le vecteur pHC79 linéarisé par BamHI.

Environ 150 ng de cosmide linéarisés comme décrit plus haut ont été précipités à l'éthanol avec 350 ng de fragments d'ADN total de S.pristinseapiralis SP92 préparés comme décrit à l'exemple 3.2. Le culot a été repris dans 10 µl de tampon de ligation : 50 mM Tris-HCl pH 7,8, 10 mM MgCl₂, 20 mM DTT, 1 mM ATP, 50 µg/ml de BSA et 0.5 µl de T4 DNA ligase à 400 000 unités par ml (New England Biolabs, Beverly, MA. 01915-5510 USA) ont été ajoutés. L'incubation a été réalisée durant une nuit à 15°C.

### 3.4. Réalisation de l'encapsidation in vitro.

Cet exemple illustre comment les cosmides construits en 3.3 sont encapsidés in vitro.

L'encapsidation des cosmides hybrides après ligation a été réalisée à partir du kit Gigapack II Gold, développé par Stratagene (Stratagene Cloning Systems, La Jolla, CA 92037, USA).

2 x 4 µl de mélange de ligation, soit 2 x 70 ng de cosmides hybrides ont été encapsidés in vitro selon la procédure décrite par le fournisseur.

### 3.5. Transfection des souches d'E.coli DH1 et HB101.

Cet exemple illustre comment les cosmides sont introduits chez E.coli.

Deux transfections en parallèle ont été réalisées avec les souches d' E.coli DH1 (F⁻ gyrA96 recA1 relA1 endA1 thi-1 hsdR17 supE44L⁻. Low 1968) et HB101 (F⁻ supE44 hsdS20(rB⁻mB⁻) recA13 ara-14 proA2 lacY1 galK2 rpsL20 xyl-5 mtl-1, Boyer et Roulland-Dussoix 1969).

Les cellules ont été préparées selon le protocole suivant : une préculture de 100 ml est réalisée en milieu LB complémenté par 0.2 % Maltose et 10 mM MgSO₄, pendant 4 à 5 heures jusqu'à ce que la DO₆₀₀ atteigne la valeur de 0.8. La culture est alors centrifugée et le culot repris dans 40 ml de MgSO₄ 10 mM et dilué jusqu'à DO₆₀₀=0,5, dans la même solution. 200 µl de la suspension cellulaire ainsi préparée sont mélangés à 100 µl de mélange d'encapsidation. Après 20 mn de contact à 37°C, 1 ml de LB est ajouté et l'ensemble est incubé 1 heure à 37°C. Les transfectants sont ensuite sélectionnés sur milieu LB solide contenant 150 µg/ml d'ampicilline. Le nombre de transfectants obtenus est d'environ 10⁴ par µg de cosmide recombinant.

### 3.6. Stockage des banques d'ADN génomique de S. pristinaespiralis SP92.

Cet exemple illustre comment les banques d'ADN génomique de S.pristinaespiralis SP92 sont conservées.

Après vérification de la taille moyenne des fragments insérés dans le cosmide pHC79, environ 1500 colonies issues de chacune des transfections réalisées avec les souches HB101 et DH1 sont repiquées dans des plaques de micro-titration à 96 puits contenant 200 µl de milieu Hogness (milieu LB complémenté avec 8.8 % glycérol, 3 mM sodium acétate, 55 mM K₂HPO₄, 26 mM KH₂PO₄, 1 mM MgSO₄, 15 mM (NH₄)₂SO₄, ampicilline 150 µg/ml). Ces plaques sont incubées une nuit à 37°C puis stockées à -80°C.

### 3.7. Préparation des membranes d'hybridation à partir des banques génomiques de S. pristinaespiralis SP92.

Cet exemple illustre comment l'ADN des colonies constituant les banques génomiques de S. pristinaespiralis SP92 est transféré sur une membrane d'hybridation.

Ces membranes d'hybridation ont été réalisées en double pour chacune des 2 banques, selon le protocole suivant :

Les 15 plaques de micro-titration de chaque banque sont répliquées à l'aide d'une brosse à clous sur milieu LB agar contenant 150 µg/ml d'ampicilline. Après une nuit de croissance à 37°C, le transfert des colonies est effectué sur membrane Biohylon Z⁺ (Bioprope System) selon le protocole suivant : la membrane découpée à la taille adéquate est laissée au contact des colonies pendant 1 min. Puis une dénaturation est effectuée par imbibation de la membrane avec une solution de NaOH 0.5 M, NaCl 1.5 M pendant 5 min, suivie d'une neutralisation par imbibation de la membrane dans une solution d'acétate de sodium 3 M pendant 5 min. L'ADN est fixé sur la membrane par exposition sous une lampe UV pendant 5 min.

### EXEMPLE 4

### 4.1. Préparation de l'ADN chromosomique de la souche S. pristinaespiralis SP92 et de souches dérivées de SP92 sous forme d'inserts pour l'électrophorèse à champ pulsé.

Cet exemple illustre comment l'ADN de la souche S. pristinaespiralis SP92 et des souches dérivées de SP92 est préparé sous forme d'inserts pour l'électrophorèse à champ pulsé.

Cette préparation est effectuée à partir d'une culture de mycélium obtenue de la façon suivante: 30 ml de milieu YEME contenant 0.25 % de glycine sont inoculés avec 10⁸ spores de la souche étudiée, la culture est incubée pendant 48 heures à 30°C et agitée à 280 rpm dans des erlens de 250 ml. Le mycélium est ensuite récolté par centrifugation de 10 min à 3800 rpm et lavé deux fois avec du saccharose 10 %. Le culot de mycélium est ensuite remis en suspension dans 5ml de solution I (EDTA 250 mM pH 8,0, saccharose 20.6 %). A 200 µl de mycélium ainsi obtenu, on ajoute 400 µl d'une solution de lysozyme à 50 mg/ml dans la solution I ainsi que 800 µl d'agarose LMP à 1 % dans 25 mM EDTA pH 8 et 10.3 % saccharose, maintenu à 42°C. Le mélange maintenu à 42°C est ensuite coulé dans les puits de peignes spéciaux que l'on ferme avec du ruban adhésif et que l'on garde 30 min à 4°C. Le mélange se solidifie et les 30 à 40 inserts ainsi obtenus et contenus dans les puits sont démoulés avec précaution.

Les inserts sont d'abord rincés pendant 30 min à 4°C dans une solution contenant 25 mM EDTA et 10.3 % saccharose. On les fait ensuite tremper dans une solution d'EDTA 500 mM, 1 % de Lauryl sarcosyl et 1 mg/ml de protéinase K pendant deux fois 24 heures à 50°C en agitant de temps en temps. Puis les inserts sont lavés 3 fois une heure dans du TE contenant 1mM PMSF en changeant la solution après chaque lavage. Les inserts ainsi obtenus sont conservés à 4°C pendant 4 mois maximum dans de l'EDTA 0.5 M pH 8,0.

### 4.2. Digestion d'inserts de l'ADN de la souche S. pristinaespiralis SP92 et de souches dérivées de SP92 et analyse par électrophorèse à champ pulsé.

Cet exemple illustre comment l'ADN chromosomique de la souche S. pristinaespiralis SP92 et de souches dérivées de SP92 préparé sous forme d'inserts comme il est décrit dans l'exemple 4.1., est coupé par différentes enzymes de restriction pour l'électrophorèse à champ pulsé.

### 4.2.1. Digestion de l'ADN chromosomique en inserts:

Les inserts sont d'abord lavés six fois dans du TE puis incubés deux fois pendant une heure dans le tampon de l'enzyme de restriction choisie. Chaque insert est ensuite déposé dans le couvercle d'un tube eppendorf contenant 160 µl de tampon de l'enzyme de restriction et 40 unités d'enzyme. L'ensemble est recouvert d'un parafilm et l'eppendorf est fermé pour maintenir le parafilm qui permet d'éviter toute évaporation du tampon. Les tubes sont incubés à la température voulue dans une étuve pendant une nuit.

### 4.2.2. Analyse de l'ADN digéré par électrophorèse à champ pulsé:

La technique d'électrophorèse à champ pulsé choisie pour cette étude est celle du système CHEF (Clamped Homogenous Electric Field) mis au point par Chu et al (1986) qui permet d'obtenir deux champs alternatifs homogènes orientés de 120° l'un par rapport à l'autre et des trajectoires rectilignes pour les molécules d'ADN. L'appareilllage utilisé est le "Pulsafor System" commercialisé par Pharmacia-LKB.

On a fait varier les paramètres de migration électrophorétique tels que le temps de pulse (i.e. la durée d'application du champ électrique) et la durée de migration de manière à obtenir une séparation optimale de fragments d'ADN dont la taille s'échelonne entre 10 et 2500 kb. Les trois conditions de migration utilisées sont les suivantes: pour séparer des grands fragments d'une taille de 200 à 1700 kb, la migration choisie est de 40 heures avec un temps de pulse de 90 secondes; pour séparer des fragments d'une taille de 50 à 400 kb, la migration choisie est de 20 heures avec un temps de pulse de 10 secondes puis 20 heures avec un temps de pulse de 30 secondes; enfin, pour séparer des plus petits fragments d'une taille de 10 kb à 200 kb, la migration choisie est de 24 heures avec un temps de pulse de 10 secondes. Pour ces trois conditions de migration, le voltage est fixé à 150 Volts constants, la température est maintenue à 13°C et les gels électrophorétiques contiennent 1.3 % d'agarose.

Les inserts contenant l'ADN chromosomique de la souche S. pristinaespiralis SP92 et des souches dérivées de SP92 sont digérés par les enzymes de restriction comme il est décrit ci-dessus, et sont déposés dans les puits du gel électrophorétique à l'aide de deux lames de scalpel. Les marqueurs de poids moléculaire utilisés sont le "Yeast chromosome PFG marker" et le "Lambda ladder PFG marker" commercialisés par la société New England Biolabs. La migration s'effectue dans une des conditions décrites précédemment et le gel est ensuite coloré dans un bain de BET (bromure d'éthidium) à 4 µg/ml pendant 20 min puis décoloré dans de l'eau pendant 20 min. Après photographie du gel, les fragments d'ADN sont transferés sur une membrane de nylon puis hybridés avec des sondes marquées au [α-³²P]dCTP comme il est décrit dans l'exemple 9.1.

### EXEMPLE 5 : Isolement de cosmides portant les gènes codant pour des protéines purifiées impliquées dans la biosynthèse des Streptogramines.

Cet exemple décrit comment, à partir d'une protéine purifiée intervenant dans la biosynthèse des Pristinamycines, dont la séquence NH₂-terminale, ou une séquence interne ont été établies, il est possible d'isoler à partir des banques génomiques précédemment réalisées, un cosmide portant le gène de structure de cette même protéine ou bien d'identifier parmi les gènes déjà séquences portés par les cosmides le gène de structure correspondant.

### 5.1. Isolement des cosmides pIBV1 et pIBV3 portant l'un ou les deux gènes de structure des deux sous-unités de la Pristinamycine IIA synthase.

### 5.1.1. Identification et purification d'une des protéines impliquées dans l'étape finale de la synthèse des Pristinamycines II : la Pristinamycine IIA synthase.

Comme il a été indiqué dans l'introduction, la dernière étape de synthèse de la Pristinamycine IIA correspond à une oxydation de la liaison 2-3 de la D-proline en déhydroproline. La protéine responsable de cette activité a été purifiée jusqu'à homogénéité comme l'illustre cet exemple.

### 5.1.1.A. Dosage de l'activité Pristinamycine IIA synthase.

Cet exemple illustre le dosage d'une activité de la voie de biosynthèse de la Pristinamycine IIA qui n'a encore jamais été décrite et qui possède la propriété remarquable de n'être exprimée que pendant la période de production des Pristinamycines. Il s'agit de la Pristinamycine IIA synthase qui catalyse la conversion de la Pristinamycine IIB en Pristinamycine IIA par oxydation du résidu D-proline de la Pristinamycine IIB en résidu 2-3 déhydroproline (figure 8) en présence d'oxygène moléculaire et de FMNH₂. Les fractions enzymatiques à doser (0,002 à 0,005 unités) sont incubées 1 heure à 27°C dans un volume total de 500 µl de tampon bis-tris-propane 50 mM pH 6,8 contenant NADH (500 µM), FMN (5 µM), Pristinamycine IIB (20 µM) et 0,02 unités de FMN réductase (Boehringer Mannheim).

La Pristinamycine IIA formée est dosée par CLHP après l'arrêt de l'incubation par ajout de 500 µl d'acide chlorhydrique 0,1 N et 500 µl d'acétonitrile, et une centrifugation de l'échantillon pendant 5 min à 5000 g. 150 µl du surnageant de centrifugation sont injectés sur une colonne Nucléosil 5-C8 de 15 cm éluée par un mélange de 34 % d'acétonitrile et 66 % tampon phosphate 0,1 M pH 2,9. Les Pristinamycines IIA et IIB sont détectées grâce à leur absorbance UV à 206 nm.

L'unité d'activité enzymatique est définie comme la quantité d'enzyme nécessaire pour synthétiser 1 µmole de Pristinamycine IIA par heure dans les conditions décrites.

### 5.1.1.B. Purification de la Pristinamycine IIA synthase de S. pristinaespiralis SP92.

Cette expérience illustre comment une enzyme de S. pristinaespiralis SP92 intervenant dans la voie de biosynthèse de la Pristinamycine IIA peut être purifiée.

En utilisant le dosage décrit précédemment à l'exemple 5.1.1.A la purification de la Pristinamycine IIA synthase est réalisée comme décrit ci-dessous en prenant soin de congeler et conserver les fractions actives à -30°C entre chaque étape si nécessaire.

150 g d'un culot de centrifugation, lavé par un tampon phosphate 0,1 M pH 7,2 à 10 % v/v de glycérol, d'une culture de S. pristinaespiralis SP92 récoltée en début de phase de production des Pristinamycines sont repris par 450 ml de tampon bis-tris-propane 50 mM pH 6,8 contenant 5 mM de DTT et 0,2 mg/ml de lysozyme. La suspension ainsi obtenue est incubée pendant 45 minutes à 27°C puis centrifugée à 50 000 g durant 1 heure. L'extrait brut ainsi recueilli est fractionné par précipitation au sulfate d'ammonium. La fraction protéique précipitant entre 40 et 55 % de saturation est dessalée sur une colonne de Sephadex G25 Fine puis injectée (100 mg par injection) dans le tampon pH 6,8 bis-tris-propane 50 mM, DTT 1 mM sur une colonne MonoQ HR 10/10. Les protéines sont éluées avec un gradient linéaire de KCl (0 à 0,5M). Les fractions contenant l'activité enzymatique (mises en évidence grâce au test décrit à l'exemple 5.1.1.A) sont regroupées et concentrées à 20 ml sur Centriprep 10. Après dilution par un volume de tampon pH 6,8 bis-tris-propane 50 mM, DIT 1 mM contenant 2 M de sulfate d'ammonium, les protéines sont chromatographiées (22,5 mg par injection) sur une colonne Phenyl Superose HR 10/10 avec un gradient décroissant de sulfate d'ammonium (1,0 M à 0 M). Les meilleures fractions contenant l'activité recherchée sont rassemblées, reconcentrées à 1 ml sur Centriprep 10, puis appliquées (200 µl par injection) sur une colonne Bio-Sil SEC 250. Le pic d'activité est détecté dans cette technique à un poids moléculaire centré à 77 000. La fraction contenant l'activité est injectée sur une colonne MonoQ HR 5/5 dans le tampon pH 6,8 bis-tris-propane 50 mM, DTT 1 mM éluée avec un gradient linéaire de KCl (0 à 0,5 M).

Après cette étape, l'enzyme est pure et en éléctrophorèse PAGE-SDS, deux sous unités de poids moléculaire estimé à 35 000 et 50 000 sont mises en évidence. Elles sont séparées sur colonne Vydac C4 de 25 cm éluée avec un gradient linéaire de 30 à 50 % d'acétonitrile dans l'eau à 0,07 % d'acide trifluoroacétique.

**Tableau:**

| Purification de la Pristinamycine IIA synthase | | | | | |
|---|---|---|---|---|---|
| Etape de Purification | vol. (ml) | Protéines (mg) | Act. Spé. µmole/h/mg | Rendement (%) | Facteur de purification |
| Extrait brut | 490 | 1690 | 0,14 | 100 | 1 |
| 40-55% S. A. | 60 | 1050 | 0,19 | 85 | 1,4 |
| MonoQ 10/10 | 95 | 45 | 3,0 | 58 | 21 |
| Phényl-Superose | 8 | 2,8 | 12 | 14 | 86 |
| BioSil SEC | 5 | 1,3 | 18 | 14 | 130 |
| MonoQ 5/5 | 10 | 0,7 | 23 | 10 | 160 |

Le facteur de purification est calculé d'après l'augmentation de l'activité spécifique des fractions au cours de la purification.

### 5.1.2. Réalisation d'oligonucléotides à partir des séquences protéiques :

Cet exemple décrit comment à partir des séquences NH₂-terminales des deux sous-unités de la Pristinamycine IIA synthase purifiée comme décrit dans l'exemple 5.1.1.B., il est possible de synthétiser des oligonucléotides. Les deux sous-unités de la Pristinamycine IIA synthase sont appelées SnaA et SnaB et correspondent aux polypeptides de poids moléculaire de 50000 et 35000 respectivement tel que cela est décrit à l'exemple 5.1.1.B.

Les séquences NH₂-terminales des proteines SnaA et SnaB, correspondant aux sous unités de la Pristinamycine IIA synthase, ont été réalisées par microséquençage. Ceci est fait par la technique de dégradation d'Edman, en utilisant un séquenceur automatisé (Applied Biosystems modèle 407A) couplé à un appareil CLHP pour l'identification des dérivés phénylthiohydantoïnes. Une trentaine de résidus ont été déterminés pour chacune d'entre elles.

### Protéine SnaA : (Cf résidus 2 à 29 sur SEQ ID n° 2)

### Protéine SnaB : (Cf résidus 2 à 31 sur SEQ ID n° 3)

Par ailleurs des séquences internes à ces deux polypeptides ont été déterminées après digestions trypsiques de SnaA et SnaB et purification des fragments obtenus sur colonne CLHP Vydac C18. Les séquences internes suivantes ont été trouvées:

### Protéine SnaA : (Cf résidus 365 à 384 sur SEQ ID n° 2)

### Protéine SnaB : (Cf résidus 122 à 136 sur SEQ ID n° 3)

A partir des régions soulignées dans chacune des séquences des fragments internes aux protéines SnaA et SnaB et, en fonction de la dégénérescence du code génétique spécifique des Streptomyces (cf. Exemple 8), les mélanges d'oligonucléotides suivants ont été synthétisés par un synthétiseur automatisé Biosearch 8600. Ils ont ensuite été purifiés par la technique déjà décrite (Sawadogo M. et Von Dyke M. W., 1991). Les gènes snaA et snaB désignent les gènes de structure des protéines SnaA et SnaB respectivement.

### Mélange correspondant à la partie soulignée de la séquence interne de SnaA :

### Mélange correspondant à la partie soulignée de la séquence interne de SnaB :

### 5.1.3. Marquage des mélanges d'oligonucléotides synthétiques et hybridation avec les banques d'ADN génomique de la souche SP92.

Cet exemple décrit comment des oligonucléotides spécifiques d'un gène de biosynthèse des Pristinamycines peuvent être marqués radioactivement puis hybridés avec des membranes sur lesquelles l'ADN des banques génomiques de S. pristinaespiralis SP92 a été transféré.

Le marquage des oligonucléotides est réalisé par transfert en position 5' terminale, du groupement [γ-³²P]phosphate de l'ATP par la T4 polynucléotide kinase. Ce marquage est réalisé comme indiqué dans Maniatis et al. (1989). Après le marquage, les oligonucléotides sont utilisés sans purification.

Environ 2 X 500 ng de chaque mélange d'oligonucléotides ont été ainsi marqués au ³²P et ont été utilisés pour hybrider chacune des deux banques.

L'hybridation des membranes de chaque banque est réalisée selon un protocole dérivé de ceux développés par Meinkoth, J. and Wahl, G. (1984) et Hames, B.D. and Higgins, SJ. (1985) : les 15 membranes sont préhybridées pendant 3 heures à 50°C dans 40 ml d'une solution contenant : Denhardt (X5) [Denhardt (X100) : 2 % (p/v) Ficoll, 2 % (p/v) polyvinyl-pyrrolidone, 2 % (p/v) BSA)], SSC (X5) [SSC (X20) : NaCl 3 M, citrate de sodium 0.3 M), NaPO₄ pH 6,5 50 mM, SDS 0.1 %, ADN de sperme de saumon 250 µg/ml].

L'hybridation est ensuite réalisée pendant une nuit à 50°C, dans 20 ml de la même solution auxquels sont ajoutés les 500 ng d' oligonucléotides marqués.

Les filtres sont ensuite lavés dans une solution de SSC (X 6) et SDS 0.5 %, 2 fois 30 min à température ambiante puis de façon empirique à des températures graduellement plus élévées (50 à 65°C). La température de ces derniers lavages est progressivement augmentée après des expositions autoradiographiques successives afin de déterminer la spécificité des clones hybridants avec les mélanges d'oligonucléotides.

5.1.4. Isolement des cosmides pIBV1 et pIBV3 et détermination des régions contenant les gènes snaA et snaB.

Cet exemple illustre comment il est possible d'isoler des cosmides construits comme il est décrit dans l'exemple 3, contenant des gènes de biosynthèse des Pristinamycines.

Les cosmides pIBV1 et pIBV3 ont été isolés de deux clones provenant respectivement de la banque réalisée dans la souche HB101 et de la banque réalisée dans la souche DH1, ayant hybridé avec les deux mélanges d'oligonucléotides simultanément pour pIBV1 et avec le mélange d'oligonucléotides issus de la séquence interne de la protéine SnaA pour pIBV3.

Ces cosmides ont été purifiés comme décrit dans l'exemple 2. Les cosmides pIBV1 et pIBV3 contiennent respectivement un insert d'ADN génomique de S. pristinaespiralis SP92 dont les tailles ont été estimées respectivement à 30 kb et 34 kb. Une cartographie (figure 4 et 6) a été établie à partir de digestions avec différentes enzymes de restriction, selon les protocoles du fournisseur (New England Biolabs, Beverly, MA. 01915-5510 USA).

Des hybridations en Southern de l'ADN de pIBV1 et pIBV3 digéré par différentes enzymes, avec les mélanges d'oligonucléotides, ont permis d'identifier la région de ce cosmide contenant les gènes snaA et/ou snaB.

Les Southerns ont été réalisés comme décrit dans Maniatis et al. (1989). Après séparation des fragments de restriction par électrophorèse sur gel d'agarose 0.8 %, l'ADN est transféré sur membrane Biohylon Z⁺ (Bioprope System). L'hybridation de l'ADN ainsi transféré sur les membranes avec les mélanges d'oligonucléotides a été réalisée comme décrit dans l'exemple 5.1.3.

Ces Southerns ont permis de montrer que le cosmide pIBV1 possédait un fragment BamHI de 6 kb contenant les séquences homologues aux sondes synthétisées dans l'exemple 5.1.2 (issues des protéines SnaA et SnaB) ainsi qu'un fragment EcoRI de 2,5 kb interne au fragment BamHI contenant les séquences homologues aux sondes issues exclusivement de la protéine SnaA. De plus les signaux d'hybridation obtenus avec le cosmide pIBV3 ont montré qu'il ne possédait que le fragment EcoRI de 2,5 kb contenant les séquences homologues aux sondes issues exclusivement de la protéine SnaA.

### 5.2. Isolement du cosmide pIBV2 contenant le gène de structure de l'acide 3-hydroxypicolinique-AMP ligase (snbA).

Cet exemple illustre comment il est possible d'obtenir un cosmide, tel que construit à l'exemple 3, contenant au moins un gène de biosynthèse des Pristinamycines L

### 5.2.1. Identification et purification de la protéine impliquée dans l'activation de l'acide 3-hydroxypicolinique.

Cet exemple illustre comment la protéine responsable de l'activation de l'acide 3-hydroxypicolinique peut-être purifiée jusqu'à homogénéité, à partir de S. pristinaespiralis SP92.

### 5.2.1.A. Dosage de l'activité acide 3-hydroxypicolinique-AMP ligase.

Cet exemple illustre le dosage d'une activité de la voie de biosynthèse de la Pristinamycine IA qui n'a encore jamais été décrite et qui possède la propriété remarquable de n'être exprimée que pendant la période de production des Pristinamycines. Il s'agit de l'acide 3-hydroxypicolinique-AMP ligase qui catalyse la formation de l'adenylate de l'acide 3-hydroxypicolinique (figure 9) à partir de cet acide libre et de l'ATP en présence de MgCl₂.

Les fractions enzymatiques à doser (0,002 à 0.020 unités) sont incubées pendant 15 min à 27°C dans un volume total de 250 µl de tampon pH 6,8 bis-tris-propane 50 mM, DTT 1 mM, 10 % v/v de glycérol, en présence d'acide 3-hydroxypicolinique (1mM), ATP (2mM), MgCl₂ (5mM) et tetrasodium pyrophosphate marqué avec l'isotope 32 radioactif de l'atome de phosphore (200 µM).

La réaction est arrêtée par ajout de 1 ml d'une suspension de charbon activé à 10 g/l dans un mélange de 75 % de tetrasodium pyrophosphate 0,1 M et 25 % d'acide perchlorique à 14 %. Après agitation le charbon est recueilli et lavé par deux fois 1 ml du mélange pyrophosphate-acide perchlorique. Les molécules organiques radioactives sont alors éluées par trois fois 1 ml d'un mélange de 50 % de méthanol et 50 % d'ammoniaque N, dans une fiole de comptage contenant 12 ml d'eau. La radioactivité est mesurée par effet Cerenkov avec un compteur à scintillation (Minaxi TriCarb 4000 PACKARD).

L'unité d'activité enzymatique est définie comme la quantité d'enzyme nécessaire pour incorporer 1 µmole de pyrophosphate dans l'ATP en 1 heure dans les conditions décrites ci-dessus.

### 5.2.1.B. Purification de l'acide 3-hydroxypicolinique-AMP ligase de S. pristinaespiralis SP92.

Cette expérience illustre comment une enzyme de S. pristinaespiralis SP92 intervenant dans la voie de biosynthèse de la Pristinamycine IA peut être purifiée.

En utilisant le dosage décrit précédemment à l'exemple 5.2.1.A, la purification de l'acide 3-hydroacypicolinique-AMP ligase est réalisée comme décrit ci-dessous en prenant soin de congeler à -70°C les fractions actives et de les conserver à -30°C entre chaque étape si nécessaire.

234 g d'un culot de centrifugation, lavé par un tampon phosphate 0,1 M pH 7,2 à 10 % v/v de glycérol, d'une culture de S. pristinaespiralis SP92 récoltée en début de phase de production des Pristinamycines sont repris par 234 ml de tampon pH 8,0 Tris-HCl 100 mM contenant 4 mM DTE, 1 mM benzamidine, 1 mM PMSF, 15 % v/v glycérol et 0,6 mg/ml de lysozyme. La suspension ainsi obtenue est incubée pendant 30 minutes à 27°C puis centrifugée à 50 000 g durant 1 heure. L'extrait brut ainsi recueilli est injecté dans le tampon pH 8,0 Tris-HCl 100 mM, DTE 4 mM, 1 mM benzamidine, 1 mM PMSF, 15 % v/v glycérol sur une colonne (80 ml) de Q-Sepharose Fast Flow. Les protéines sont éluées avec un gradient linéaire de KCl (0 à 0,4 M). Les fractions contenant l'activité enzymatique (mises en évidence grâce au test décrit à l'exemple 5.2.1.A) sont regroupées et diluées par un volume de tampon pH 8,0 Tris-HCl 100 mM, 1 mM benzamidine, 1 mM PMSF, 15 % v/v glycérol contenant 2 M de sulfate d'ammonium. Les protéines sont alors chromatographiées sur une colonne (50 ml) de Phényl-Sepharose avec un gradient décroissant de sulfate d'ammonium (1,0 M à 0 M) dans le tampon pH 8,0 Tris-HCl 100 mM, 1 mM benzamidine, 1 mM PMSF, 15 % v/v glycérol. Après ajout de 4 mM DTE, les fractions actives sont rassemblées, reconcentrées à 5 ml sur Centriprep 10, puis appliquées sur une colonne (100 ml) de Superose 12 prep grade. Les fractions contenant l'activité recherchée sont regroupées et injectées dans le tampon pH 8,0 Tris-HCl 100 mM, DTE 4 mM, 1 mM benzamidine, 1 mM PMSF, 15 % v/v glycérol (environ 6 mg par injection) sur une colonne de MonoQ HR 5/5 éluée avec un gradient linéaire de KCl (0 à 0,4 M). Les fractions actives sont regroupées, concentrées à 1 ml sur Centricon 10, diluées par 3 volumes de tampon pH 6,8 bis-tris-propane 50 mM, DTE 4 mM, 1 mM benzamidine, 1 mM PMSF, 15 % v/v glycérol, puis injectées (2 mg par injection) dans ce dernier tampon sur une colonne de MonoQ HR 5/5 éluée avec un gradient linéaire de KCl (0 à 0,3 M). Les meilleures fractions contenant la ligase recherchée sont regroupées, puis appliquées dans un tampon pH 6,8 phosphate de sodium 20 mM, sulfate de sodium 50 mM sur une colonne Bio-Sil SEC 250. Le pic d'activité est détecté dans cette technique à un poids moléculaire centré à 60 000.

La protéine possédant l'activité d'activation de l'acide 3-hydroxypicolinique est désignée par la suite SnbA.

Après cette étape, l'enzyme est pure et en éléctrophorèse PAGE-SDS son poids moléculaire est estimé à environ 67 000.

**Tableau:**

| Purification de l'acide 3-hydroxypicolinique-AMP ligase | | | | | |
|---|---|---|---|---|---|
| Etape de Purification | vol. (ml) | Protéines (mg) | Act. Spé. µmole/h/mg^{a} | Rendement (%) | Facteur de purification |
| Extrait brut | 246 | 2050 | (0,06) | | |
| Q-Sepharose | 40 | 188 | 0,47 | 100 | 1 |
| Phényl-Sepharose | 70 | 35 | 2,21 | 88 | 4,7 |
| Superose 12 | 16 | 17 | 2,03 | 39 | 4,3 |
| ManoQ pH 8,0 | 4,5 | 9,0 | 2,09 | 21 | 4,5 |
| MonoQ pH 6,8 | 1.0 | 2,0 | 2,9 | 6,6 | 6,2 |
| Bio-Sil 250 | 2,5 | 0,23 | 12,4 | 3,2 | 26 |

| | | | | | |
|---|---|---|---|---|---|
| ^{a} L'activité dans l'extrait brut ne peut pas être mesurée précisément à cause d'échanges entre le pyrophosphate et l'ATP non spécifiques de l'acide 3-hydroxypioolinique. | | | | | |

Le facteur de purification est calculé d'après l'augmentation de l'activité spécifique des fractions au cours de la purification.

### 5.2.2. Réalisation d'oligonucléotides à partir de la séquence protéique:

Cet exemple décrit comment à partir des séquences NH₂-terminale et interne de la protéine 3-hydroxypicolinique-AMP ligase, il est possible de synthétiser des oligonucléotides.

La séquence NH₂-terminale de la protéine SnbA a été réalisée par microséquençage comme décrit dans l'exemple 5.1.2. Une vingtaine de résidus ont été ainsi identifiés.

Une séquence interne à la protéine SnbA, d'environ 20 acides aminés, a également été identifiée, après hydrolyse trypsique et purification des fragments obtenus sur colonne CLHP Vydac C18.

### Séquence NH₂-terminale de la protéine 3-hydroxypicolinique-AMP ligase : (Cf résidus 1 à 21 sur SEQ ID n° 5)

### Séquence interne de la protéine 3-hydroxypicolinique-AMP ligase: (Cf résidus 448 à 467 sur SEQ ID n° 5)

A partir des régions soulignées dans chacune des séquences et en fonction de la dégénérescence du code génétique spécifique des Streptomyces (cf. Exemple 8), les mélanges d'oligonucléotides suivants ont été synthétisés :

### Mélange correspondant à la partie soulignée de la séquence NH₂-terminale de la protéine 3-hydroxypicolinique-AMP ligase:

### Mélange correspondant à la partie soulignée de la séquence interne de la protéine 3-hydroxypicolinique-AMP ligase :

### 5.2.3. Marquage des mélanges d'oligonucléotides synthétiques et hybridation des banques d'ADN génomique de S. pristinaespiralis SP92.

Cet exemple décrit comment des oligonucléotides spécifiques d'un gène de biosynthèse des Pristinamycines peuvent être marqués radioactivement puis hybridés avec des membranes sur lesquelles l'ADN des banques génomiques de S. pristinaespiralis a été transféré.

Le marquage des oligonucléotides est réalisé par transfert en position 5' terminale, du groupement [γ-³²P]phosphate de l'ATP, par la T4 polynucléotide kinase, comme indiqué dans l'exemple 5.1.3.

Environ 2 X 500 ng de chaque mélange d'oligonucléotides ont été ainsi marqués au ³²P et ont été utilisés pour hybrider chacune des deux banques.

L'hybridation des membranes de chaque banque a été réalisée comme indiqué dans l'exemple 5.1.3.

### 5.2.4. Isolement du cosmide pIBV2 et détermination de la région contenant le gène de structure de l'acide 3-hydroxypicolinique-AMP ligase.

Cet exemple illustre comment il est possible d'obtenir un cosmide, tel que construit à l'exemple 3, contenant au moins le gène de structure de l'acide 3-hydroxypicolinique-AMP ligase.

Le cosmide pIBV2 a été isolé d'un clone de la banque réalisée dans la souche d'E. coli DH1, ayant hybridé avec les deux mélanges d'oligonucléotides simultanément.

Ce cosmide a été purifié comme décrit dans l'exemple 2. Il contient un insert d'ADN génomique de S. pristinaespiralis SP92 dont la taille à été estimée à 47 kb. Une cartographie (figure 5) a été établie à partir de digestions avec différentes enzymes de restriction, comme indiqué dans l'exemple 5.1.4.

Des hybridations en Southern de l'ADN de pIBV2 digéré par différentes enzymes, avec les mélanges d'oligonucléotides, ont permis d'identifier la région contenant le gène de structure de l'acide 3-hydroxypicolinique-AMP ligase. Les Southerns et les hybridations ont été réalisés comme décrit dans l'exemple 5.1.4.

Les résultats d'hybridation ont permis de montrer que le cosmide pIBV2 possédait un fragment EcoRI-BglII de 5,5 kb, contenant la séquence homologue aux sondes synthétisées dans l'exemple 5.2.2.

### 5.3. Mise en évidence de la présence d'une partie du gène de structure de la Pristinamycine I synthase II (SnbC) sur le cosmide pIBV3.

Cet exemple illustre comment il est possible d'identifier la présence de gènes de biosynthèse des Pristinamycines I sur un cosmide déjà isolé (Exemple 5.1).

### 5.3.1. Identification de la Pristinamycine 1 synthase II impliquée dans l'incorporation des résidus thréonine et acide aminobutyrique dans la chaîne peptidique de la Pristinamycine IA.

Cet exemple illustre comment la protéine responsable de l'incorporation des résidus thréonine et acide aminobutyrique dans la chaîne peptidique de la Pristinamycine IA peut être purifiée jusqu'à homogénéité, à partir de S. pristinaespiralis SP92.

### 5.3.1.A. Dosage des activités partielles de la Pristinamycine I synthase IL

Cet exemple illustre le dosage d'activités de la voie de biosynthèse de la Pristinamycine IA qui n'ont encore jamais été décrites et qui possèdent la propriété remarquable de n'être exprimées que pendant la période de production des Pristinamycines. Il s'agit des activités partielles de la peptide synthase responsable de l'incorporation des résidus thréonine et acide aminobutyrique dans la chaîne peptidique de la Pristinamycine IA (figure 10) en présence d'ATP et de MgCl₂.

Les activités thréonine-AMP ligase et acide aminobutyrique-AMP ligase sont mesurées dans un test enzymatique d'échange ATP-pyrophosphate analogue à celui décrit en 5.2.1.A pour l'acide 3-hydroxypicolinique-AMP ligase.

Les réactions d'aminoacylation de l'enzyme avec la thréonine ou l'alanine (un analogue de l'acide aminobutyrique que l'on retrouve dans la Pristinamycine IC) permettent de différencier la peptide synthase d'autres enzymes qui peuvent effectuer un échange ATP-pyrophosphate et notamment les aminoacyl-tRNA synthases. C'est donc le test d'aminoacylation de l'enzyme avec la thréonine marquée au tritium décrit ci-dessous qui a été utilisé dans cet exemple.

Les fractions enzymatiques à doser (0,2 à 2 unités) sont incubées pendant 15 min à 27°C dans un volume total de 250 µl de tampon pH 6,8 bis-tris-propane 50 mM, DTT 1 mM, 10 % v/v de glycérol, en présence de 1 µCi de [3-³H] L-threonine (15 Ci/mmol), d'ATP (2 mM) et de MgCl₂ (5 mM).

La réaction est arrêtée par ajout de 150 µl d'une solution d'acide trichloroacétique à 25 %. Les protéines précipitées sont recueillies sur un microfiltre et lavées par 3 fois 400 µl d'acide trichloroacétique à 7 % avant d'être éluées par 2 fois 400 µl de soude N dans une fiole de comptage contenant 1 ml HCl N et 12 ml de cocktail scintillant (Readygel Beckmann). La quantité de radioactivité contenue dans cette fiole est mesurée avec un compteur à scintillation (Minaxi TriCarb 4000 PACKARD). Elle représente la quantité de thréonine fixée de façon covalente à la peptide synthase recherchée.

L'unité d'activité enzymatique est définie comme la quantité d'enzyme nécessaire pour fixer de manière covalente 1 picomole de thréonine en 15 min dans les conditions décrites ci-dessus.

### 5.3.1.B. Purification de la Pristinamycine I synthase II.

Cette expérience illustre comment une enzyme de S. pristinaespiralis SP92 intervenant dans la voie de biosynthèse de la Pristinamycine IA peut être purifiée.

En utilisant le dosage décrit précédemment à l'exemple 5.3.1.A. la purification de la peptide synthase responsable de l'incorporation des résidus thréonine et acide aminobutyrique dans la chaîne peptidique de la Pristinamycine IA est réalisée comme décrit ci-dessous en prenant soin de travailler à 4°C et de conserver à -70°C les fractions actives.

150 g d'un culot de centrifugation, lavé par un tampon phosphate 0,1 M pH 7,2 à 10 % v/v de glycérol, d'une culture de S. pristinaespiralis SP92 récoltée en début de phase de production des Pristinamycines sont repris par 450 ml de tampon pH 8,0 Tris-HCl 100 mM contenant 4 mM DTE, 1 mM benzamidine, 1 mM PMSF, 1 mM EDTA, 1 mM EGTA, 15 % v/v glycérol. La suspension ainsi obtenue est broyée à l'aide d'une French Press réglée à la pression de 5000 psi puis centrifugée à 50 000 g durant 1 heure. L'extrait brut ainsi recueilli est injecté dans le tampon pH 8 tris-HCl 100 mM, DTE 4 mM, 2 mM benzamidine, 2 mg/l leupeptine, 1mg/l E-64, 15 % v/v glycérol sur une colonne (200 ml) de Q-Sepharose Fast Flow. Les protéines sont éluées avec un gradient linéaire de KCl (0 à 0,6 M). A la sortie de la colonne chaque fraction est additionnée d'un dixième de son volume d'une solution de PMSF 1 mM, EDTA 5 mM. EGTA 5 mM. Les fractions contenant l'activité enzymatique (mises en évidence grâce au test décrit à l'exemple 5.3.1.A) sont regroupées et reconcentrées par ultrafiltration sur Centriprep 30 jusqu'à un volume final de 28 ml. Ce concentrat est injecté par aliquote de 4 ml sur une colonne de perméation Superdex 200 Hi-Load 16/60 équilibrée dans le tampon pH 6,8 bis-tris-propane 50 mM, 1 mM benzamidine, 4 mM DTE, 0,2 mM Pefabloc, 1 mM EDTA, 0,1 M KCl, 20 % v/v glycérol. Après dosage, les fractions actives sont rassemblées et reconcentrées à 15 ml sur Centriprep 30, puis dessalées sur PD-10 dans le tampon pH 8,0 Tris-HCl 100 mM, DTE 4 mM, 2 mM benzamidine, 2 mg/l leupeptine, 1mg/l E-64, 20 % v/v glycérol et appliquées en deux fois sur une colonne MonoQ HR 10/10 équilibrée et éluée avec un gradient linéaire de 0,4 M de KCl dans ce même tampon. Les fractions contenant l'activitée recherchée sont regroupées, reconcentrées sur Centriprep 30 puis Centricon 30 jusqu'à un volume final de 1 ml et injectées en cinq fois sur une colonne de Superose 6 HR 10/30 dans le tampon pH 6,8 bis-tris-propane 50 mM, 1 mM benzamidine, 4 mM DTE, 0,2 mM Pefabloc, 1 mM EDTA, 0,1 M KCl, 20 % v/v glycérol. Le pic d'activité est détecté dans cette technique à un poids moléculaire centré à 450 000.

Après cette étape, l'enzyme est pure et en électrophorèse PAGE-SDS son poids moléculaire est estimé à environ 240 000. Cette bande contient également toute la radioactivité de la protéine marquée par aminoacylation avec la thréonine tritiée.

A ce stade l'activité maximale de l'enzyme en utilisant une concentration de 100 µCi/ml de thréonine (15 Ci/mmole) s'élève à 3670 unités/mg; l'enzyme est également capable de former des adénylates avec l'acide-L-aminobutyrique ou la L-alanine ; une réaction d'aminoacylation de l'enzyme avec l'alanine tritiée est détectée et l'activité maximale en présence de 200 µCi/ml de [2,3-³H] L-alanine (15 Ci/mmole) est de 2290 pmoles/mg en 15 min.

**Tableau:**

| Purification de la Pristinamycine I synthase II. | | | | | |
|---|---|---|---|---|---|
| Etape de Purification | vol. (ml) | Protéines (mg) | Act. Spé^{a} (unités /mg) | Rendement (%) | Facteur de purification |
| Extrait brut | 445 | 4700 | (1) | - | - |
| Q-Sepharose | 308 | 834 | 7 | 100 | 1 |
| Superdex 200 | 120 | 105 | 22 | 40 | 3,1 |
| MonoQ HR | 15 | 11,5 | 96 | 19 | 14 |
| Superose 6 | 7,5 | 2,8 | 122 | 6 | 17 |

| | | | | | |
|---|---|---|---|---|---|
| a: L'activité dans l'extrait brut ne peut pas être mesurée précisemment. | | | | | |

Le facteur de purification est calculé d'après l'augmentation de l'activité spécifique des fractions au cours de la purification.

### 5.3.2. Réalisation d'oligonucléotides à partir de la séquence protéique.

Cet exemple décrit comment à partir de séquences internes de la Pristinamycine I synthase II il est possible de synthétiser des oligonucléotides.

Les séquences internes de la peptide synthase responsable de l'incorporation des résidus thréonine et acide aminobutyrique dans la chaîne peptidique de la Pristinamycine IA ont été réalisées par microséquençage comme décrit dans l'exemple 5.1.2. après hydrolyse trypsique et purification des fragments obtenus sur colonne Vydac C18.

### Séquences internes à la protéine Pristinamycine I synthase II. (Cf résidus 49 à 61 sur SEQ ID n° 12)

A partir des régions soulignées dans ces séquences et en fonction de la dégénérescence du code génétique spécifique des Streptomyces (cf. Exemple 8), les mélanges d'oligonucléotides suivants ont été synthétisés :

### Mélange correspondant à la partie soulignée de la séquence 1 interne à la protéine Pristinamycine I synthase II:

### Mélange correspondant à la partie soulignée de la séquence 2 interne à la protéine Pristinamycine I synthase II:

### 5.3.3. Marquage des mélanges d'oligonucléotides synthétiques et hybridation en Southern de l'ADN du cosmide pIBV3.

Cet exemple décrit comment des oligonucléotides spécifiques d'un gène de biosynthèse des Pristinamycines I peuvent être marqués radioactivement puis hybridés avec une membrane sur laquelle l'ADN du cosmide pIBV3 a été transféré.

Le marquage des oligonucléotides est réalisé par transfert en position 5' terminale, du groupement [γ-³²P]phosphate de l'ATP, par la T4 polynucléotide kinase, comme indiqué dans 5.1.3.

Environ 500 ng du mélange d'oligonucléotides a été ainsi marqué au ³²P et a été utilisé pour hybrider en Southern de l'ADN de pIBV3 digéré par différentes enzymes. Ces hybridations ont permis de montrer qu'une partie du gène de structure de la Pristinamycine I synthase II était porté par le cosmide pIBV3 et d'identifier la région contenant ce gène. Les Southerns et les hybridations ont été réalisés comme décrit dans l'exemple 5.1.4.

Les résultats d'hybridation ont permis de montrer que le cosmide pIBV3 possédait un fragment SphI de 6,2 kb, contenant la séquence homologue aux sondes synthétisées dans l'exemple 5.3.2.

### 5.4. Mise en evidence de la présence d'une partie du gène de structure de la Pristinamycine I Synthase III (SnbD) sur le cosmide pIBV3.

Cet exemple illustre comment il est possible d'identifier la présence de gènes de biosynthèse des Pristinamycines I sur un cosmide déjà isolé (Exemple 5.1).

### 5.4.1. Identification de la pristinamycine I synthase III impliquée dans l'incorporation des résidus proline et p-dimethylaminophenylalanine dans la chaîne peptidique de la Pristinamycine IA.

Cet exemple illustre comment la protéine responsable de l'incorporation des résidus proline et p-dimethylaminophenylalanine dans la chaîne peptidique de la Pristinamycine IA peut être purifiée jusqu'à homogénéité, à partir de S. pristinaespiralis SP92.

### 5.4.1.A. Dosage des activités partielles de la pristinamycine I synthase III.

Cet exemple illustre le dosage d'activités de la voie de biosynthèse de la Pristinamycine IA qui n'ont encore jamais été décrites et qui possèdent la propriété remarquable de n'être exprimées que pendant la période de production des Pristinamycines. Il s'agit des activités partielles de la peptide synthase responsable de l'incorporation des résidus proline et paradimethylaminophenylalanine dans la chaîne peptidique de la Pristinamycine IA (figure 11) en présence de SAM, d'ATP et de MgCl₂.

Les activités proline-AMP ligase et p-dimethylaminophenylalanine-AMP ligase sont mesurées dans un test enzymatique d'échange ATP-pyrophosphate analogue à celui décrit en 5.2.1.A. pour l'acide 3-hydroxypicolinique-AMP ligase.

Les réactions d'aminoacylation de l'enzyme avec la proline et la p-dimethylaminophenylalanine permettent de différencier la peptide synthase d'autres enzymes qui peuvent effectuer un échange ATP-pyrophosphate et notamment les aminoacyl-tRNA synthases. Il en est de même pour la N-methylation de la fonction α-aminée de la p-dimethylaminophenylalanine acylée sur l'enzyme. C'est donc ce dernier test caractéristique de N-methylation qui a été utilisé dans cet exemple.

Les fractions enzymatiques à doser (0,2 à 2 unités) sont incubées pendant 15 min à 27°C dans un volume total de 250 µl de tampon pH 6,8 bis-tris-propane 50 mM, DTT 1 mM, 10 % v/v de glycérol, en présence de 1 µCi de [méthyl-³H] SAM (15 Ci/mmol), de paradimethylamino-L-phenylalanine (1 mM), d'ATP (2 mM) et de MgCl₂ (5 mM).

La réaction est arrêtée par ajout de 150 µl d'une solution d'acide trichloroacétique à 25 %. Les protéines précipitées sont recueillies sur un microfiltre et lavées par 3 fois 400 µl d'acide trichloroacétique à 7 % avant d'être éluées par 2 fois 400 µl de soude N dans une fiole de comptage contenant 1 ml HCl N et 12 ml de cocktail scintillant (Readygel Beckmann). La quantité de radioactivité contenue dans cette fiole est mesurée avec un compteur à scintillation (Minaxi TriCarb 4000 PACKARD). Elle représente la quantité de paradimethylaminophenylalanine N-méthylée fixée de façon covalente à la peptide synthase recherchée.

L'unité d'activité enzymatique est définie comme la quantité d'enzyme nécessaire pour fixer de manière covalente 1 picomole de p-dimethylaminophénylalanine N-méthylée en 15 min dans les conditions décrites ci-dessus.

### 5.4.1.B. Purification de la pristinamycine I synthase III.

Cette expérience illustre comment une enzyme de S. pristinaespiralis SP92 intervenant dans la voie de biosynthèse de la Pristinamycine IA peut être purifiée.

En utilisant le dosage décrit précédemment à l'exemple 5.4.1.A la purification de la peptide synthase responsable de l'incorporation des résidus proline et paradiméthylaminophénylalanine dans la chaîne peptidique de la Pristinamycine IA est réalisée comme décrit ci-dessous en prenant soin de travailler à 4°C et de conserver à -70°C les fractions actives.

250 g d'un culot de centrifugation, lavé par un tampon 0.1 M phosphate pH 7,2, 1 mM PMSF, 5 mM EDTA, 5 mM EGTA, 0,5 M KCl, 10 % v/v de glycérol, d'une culture de S. pristinaespiralis SP92 récoltée en début de phase de production des Pristinamycines sont repris par 750 ml de tampon pH 8,0 Tris-HCl 100 mM contenant 4 mM DTE, 5 mM benzamidine, 0,2 mM Pefabloc, 1 mM EDTA, 1 mM EGTA, 2 mg/l leupeptine, 2 mg/l STI, 2 mg/l aprotinine, 1 mg/l E-64, 20 % v/v glycérol. La suspension ainsi obtenue est broyée à l'aide d'une French Press réglée à la pression de 5000 psi puis centrifugée à 50 000 g durant 1 h. L'extrait brut ainsi recueilli est fractionné par précipitation au sulfate d'ammonium. La fraction protéique tombant entre 0 et 35 % de saturation en sulfate d'ammonium est redissoute dans le tampon de cassage et dessalée sur une colonne de Sephadex G 25 Fine équilibrée et éluée dans ce même tampon. Les protéines ainsi préparées sont injectées dans le tampon pH 8,0 Tris-HCl 100 mM, DTE 4 mM, 2 mM benzamidine, 2 mg/l leupeptine, 1mg/l E-64. 20 % v/v glycérol sur une colonne (200 ml) de Q-Sepharose Fast Flow, puis sont éluées avec un gradient linéaire de KCl (0 à 0,6 M). A la sortie de la colonne chaque fraction est additionnée d'un dixième de son volume d'une solution de Pefabloc 2 mM, EDTA 5 mM, EGTA 5 mM, benzamidine 5 mM. Les fractions contenant l'activité enzymatique (mises en évidence grâce au test décrit à l'exemple 5.4.1.A) sont regroupées et précipitées avec du sulfate d'ammonium à 80 % de saturation. Les protéines tombées sont redissoutes dans un tampon pH 6,8 bis-tris-propane 50 mM, 1 mM benzamidine, 1 mM DTE, 0,2 mM Pefabloc, 1 mM EDTA, 1 mM EGTA, 2 mg/l leupeptine, 0,15 M NaCl, 20 % v/v glycérol et injectées en 5 fois par aliquote de 4 ml sur une colonne de perméation Superdex 200 Hi-Load 16/60 équilibrée et éluée dans ce même tampon. Après dosage, les fractions actives sont rassemblées et reconcentrées à 3 ml sur Centriprep 30, puis rediluées jusqu'à 20 ml avec un tampon pH 8.0 Tris-HCl 100 mM, DTE 4 mM, 1 mM benzamidine, 1 mM PMSF, 20 % v/v glycérol et appliquées en deux fois sur une colonne MonoQ HR 10/10 équilibrée et éluée avec un gradient linéaire de 0,4 M de KCL dans ce même tampon. Les meilleures fractions contenant l'activité recherchée sont regroupées et servent de matériel pour la caractérisation des activités de l'enzyme et pour son microséquençage.

Après cette étape, l'enzyme est pure et en éléctrophorèse PAGE-SDS son poids moléculaire est estimé à environ 250 000. Cette bande contient également toute la radioactivité de la protéine marquée par aminoacylation avec la SAM tritiée et la paradiméthylaminophénylalanine. En perméation sur Superose 6 HR 10/30 le poids émoléculaire natif de l'enzyme est estimé à 700 000.

A ce stade l'enzyme est également capable de former des adénylates avec la proline; une réaction d'aminoacylation de l'enzyme avec la proline tritiée est détectée et l'activité maximale en présence de 200 µCi/ml de [5-³H] L-proline (34 Ci/mmole) est de 2490 pmoles/mg en 15 min.

**Tableau:**

| Purification de la pristinamycine I synthase III. | | | | | |
|---|---|---|---|---|---|
| Etape de Purification | vol. (ml) | Protéines (mg) | Act. Spé^{a} (unités /mg) | Rendement (%) | Facteur de purification |
| Extrait brut | 800 | 8100 | (4) | - | - |
| 35 % S.A. | 200 | 4000 | (6) | - | - |
| Q-Sepharose | 132 | 498 | 46 | 100 | 1 |
| Superdex 200 | 45 | 39,5 | 417 | 71 | 9 |
| MonoQ HR | 9 | 5,3 | 1070 | 25 | 23 |

| | | | | | |
|---|---|---|---|---|---|
| a: L'activité dans l'extrait brut et après la précipitation au sulfate d'ammonium ne peut pas être mesurée précisemment. | | | | | |

Le facteur de purification est calculé d'après l'augmentation de l'activité spécifique des fractions au cours de la purification.

### 5.4.2. Réalisation d'oligonucléotides à partir de la séquence protéique.

Cet exemple décrit comment à partir de séquences internes de la pristinamycine I synthase III il est possible de synthétiser des oligonucléotides.

Une séquence interne de la peptide synthase responsable de l'incorporation des résidus proline et paradimethylaminophenylalanine dans la chaîne peptidique de la Pristinamycine IA a été réalisée par microséquençage comme décrit dans l'exemple 5.1.2. après traitement au bromure de cyanogène et purification des fragments obtenus sur colonne CLHP Vydac C18.

### Séquence interne à la protéine Pristinamycine I synthase III : 1 (Cf résidus 2 à 20 sur SEQ ID n° 13)

A partir de la région soulignée dans cette séquence et en fonction de la dégénérescence du code génétique spécifique des Streptomyces (cf. Exemple 8), le mélange d'oligonucléotides suivant a été synthétisé:

### Mélange correspondant à la partie soulignée de la séquence interne à la protéine Pristinamycine I synthase III:

### 5.4.3. Marquage des mélanges d'oligonucléotides synthétiques et hybridation en Southern de l'ADN du cosmide pIBV3:

Cet exemple décrit comment des oligonucléotides spécifiques d'un gène de biosynthèse des Pristinamycines I peuvent être marqués radioactivement puis hybridés avec une membrane sur laquelle l'ADN du cosmide pIBV3 a été transféré.

Le marquage des oligonucléotides est réalisé par transfert en position 5' terminale, du groupement [γ-³²P]phosphate de l'ATP, par la T4 polynucléotide kinase, comme indiqué dans 5.1.3.

Environ 500 ng du mélange d'oligonucléotides a été ainsi marqué au ³²P et a été utilisé pour hybrider en Southern de l'ADN de pIBV3 digéré par différentes enzymes. Ces hybridations ont permis de montrer qu'une partie du gène de structure de la Pristinamycine I synthase III était porté par le cosmide pIBV3 et d'identifier la région contenant ce gène. Les Southerns et les hybridations ont été réalisés comme décrit dans l'exemple 5.1.4.

Les résultats d'hybridation ont permis de montrer que le cosmide pIBV3 possédait un fragment SphI de 8,4 kb, contenant la séquence homologue à la sonde synthétisée dans l'exemple 5.4.2.

### 5.5. Mise en évidence de la présence d'une partie du gène de structure de la Pristinamycine I synthase IV (SnbE) sur le cosmide pIBV3.

Cet exemple illustre comment il est possible d'identifier la présence de gènes de biosynthèse des Pristinamycines I sur un cosmide déjà isolé (Exemple 5.1).

### 5.5.1. Identification de la peptide synthase (appelée Pristinamycine I synthase IV) responsable de l'incorporation du résidu phénylglycine dans la chaîne peptidique de la Pristinamycine IA.

### 5.5.1.A. Dosage d'activités enzymatiques portées par la peptide synthase (Pristinamycine I synthase IV) responsable de l'incorporation du résidu phénylglycine dans la chaîne peptidique de la Pristinamycine IA.

Cet exemple illustre le dosage d'une activité enzymatique de la voie de biosynthèse de la Pristinamycine IA qui n'a pas été décrite à ce jour, et qui possède la propriété remarquable de n'être exprimée que durant la période de production des Pristinamycines dans le microorganisme sauvage. Il s'agit de l'activité de la peptide synthase (Pristinamycine I synthase IV) responsable de l'incorporation du résidu L phénylglycine dans la chaîne peptidique (figure 12) en présence d'ATP et de MgCl₂. L'activité phénylglycine-AMP ligase de la Pristinamycine I synthase IV est mesurée dans un test enzymatique d'échange ATP-pyrophosphate similaire à celui décrit en 5.2.1.A. pour l'activité acide 3-hydroxypicolinique-AMP ligase, en présence de de L-phénylglycine (1 mM) et de KCl (50 mM) dans-le tampon d'incubation.

### 5.5.1.B. Purification de la peptide synthase responsable de l'incorporation du résidu phénylglycine (Pristinamycine I synthase IV) dans la chaîne peptidique de la Pristinamycine IA.

Cet exemple illustre comment une enzyme de S. pristinaespiralis SP92 intervenant dans la voie de biosynthèse de la Pristinamycine IA peut être purifiée. En utilisant le dosage décrit précédemment à l'exemple 5.5.1.A. La purification de la Pristinamycine I synthase IV est réalisée comme décrit ci-dessous. Toutes les opérations sont effectuées à 4°C. Les fractions contenant l'activité sont immédiatement congelées et conservées à -70°C.

70 g de cellules humides récoltées comme décrit dans l'exemple 5.2.1.B. sont resuspendus dans 250 ml de tampon de lyse cellulaire (Tris-HCl 100 mM pH 8,0 contenant 25 % de glycérol, 4 mM DTE, 1 mM EGTA, 1 mM EDTA, 1 mM PMSF, 1 mg/l E64, 2 mg/l STI, 2 mg/l α2 macroglobuline, 1 mg/l leupeptine, 2 mg/l aprotinine, 5 mM benzamidine, 0.6 mg/ml lysozyme. La solution ainsi obtenue est maintenue 1 heure à 4°C sous agitation puis centrifugée à 50 000 g durant 1 heure. Le surnageant est ensuite injecté dans le tampon de lyse cellulaire sur une colonne de sephadex-G-25, et la fraction exclue (environ 250 mg de protéines injectés lors de chaque chromatographie) est injectée sur une colonne de Mono Q HR 16/10 (Pharmacia) équilibrée avec le tampon Tris-HCl 100 mM pH 8,0, 4 mM DTE, 1 mg/l E-64, 2 mg/l STI, 20 % glycérol. Les protéines sont éluées avec un gradient linéaire de 0 à 0,6 M de KCl et, à la sortie de la colonne, chaque fraction est additionnée d'un 10ème de son volume d'une solution de Pefabloc 2mM, 5 mM EGTA, 5 mM EDTA. Les fractions contenant l'activité sont regroupées puis mélangées avec 1 volume de Tris-HCl 100 mM pH 8,0, 15 % glycérol, 1 mM PMSF, 1 mM benzamidine, 4 mM DTT, 3,4 M sulfate d'ammonium pour 3 volumes de fraction. La solution est injectée sur une colonne de Phényl-Superose HR 10/10 (le cinquième de la solution est injecté à chaque chromatographie), et les protéines sont éluées avec un gradient linéaire décroissant de 0.9 à 0 M de sulfate d'ammonium. Les fractions contenant l'activité sont regroupées. La solution est concentrée à 3500 µl dans un Centriprep 30 et injectée en 2 fois sur une colonne Superdex 200 Hi-Load 16/60 équilibrée et éluée avec du tampon bis-tris propane 50 mM pH 6,8 contenant 20 % de glycérol, 0,15 M NaCl, 4 mM DTT, 1 mM PMSF, 1 mM benzamidine, 1 mM EDTA. La fraction active est diluée par 9 volumes de tampon bis-tris propane 50 mM pH 6,8 contenant 25 % de glycérol, 4 mM DTT, 1 mM PMSF, 1 mM benzamidine, puis injectée sur une colonne de Mono Q HR 5/5 équilibrée dans le même tampon. L'activité recherchée est éluée avec un gradient linéaire de 0 à 0,4 M de KCl, et concentrée à 630 µl dans un Centricon-30. La protéine recherchée est ensuite purifiée par électrophorèse en gel de polyacrylamide à 6 % après dénaturation de l'échantillon par chauffage 10 min à 80°C avec un mélange SDS-mercaptoéthanol. Après électrophorèse et coloration du gel au bleu de Coomassie, la bande de gel contenant la protéine est découpée et la protéine est électroéluée du gel dans un Centrilutor.

Remarque: la bande correspondant à la Pristinamycine I synthase IV est identifiée par comparaison avec un standard de Pristinamycine I synthase IV tritiée (par liaison covalente à la phénylglycine tritiée; voir description dans l'exemple 5.5.2.).

Après cette étape, l'enzyme est pure en électrophorèse (PAGE-SDS). Son poids moléculaire est estimé à environ 170 000.

### 5.5.2. Marquage de la Pristinamycine I synthase IV par thioestérification de la phénylglycine radioactive sur l'enzyme.

Après activation sous forme d'adénylate par l'activité phénylglycine-AMP ligase, la phénylglycine est transférée sur un groupement thiol du site actif de l'enzyme avant d'être incorporée dans la chaine peptidique en cours d'élongation (processus général de la biosynthèse des antibiotiques peptidique connu sous le nom de "thiotemplate mechanism"). De façon générale, le marquage radioactif de la protéine effectuant l'activation d'un acide aminé peut donc être effectué en préparant le dérivé thioester avec une forme radioactive de l'acide aminé.

A titre d'exemple, le marquage radioactif de la Pristinamycine I synthase IV est effectué en incubant durant 1 heure à 27°C, 50 µg de la protéine (fraction active en sortie de la colonne de chromatographie Mono Q HR 5/5 ; voir ci-dessus dans l'exemple 5.5.1.B.) avec 100 µCi de (R,S)-2-phényl[2-³H]glycine (18 Ci/mmol; Amersham) dans 70 µl de tampon bis-tris propane 50 mM pH 6,8 contenant 20 % de glycérol, 25 mM MgCl₂, 5 mM ATP, 0,15 M NaCl, 4 mM DTT, 1 mM PMSF, 1 mM benzamidine, 1 mM EDTA. Après dénaturation (SDS seul sans mercaptoéthanol), les protéines sont séparées par électrophorèse (PAGE-SDS, gel à 6 %) et révélées au bleu de Coomassie. L'analyse du profil de radioactivité par comptage des bandes de protéines ainsi que par autoradiographie (Hyperfilm MP; fluorographie après imprégnation du gel avec Amplify Amersham) révèle une seule bande radioactive au poids moléculaire de 170 000.

**Tableau:**

| purification de la Pristinamycine I synthase IV. | | | | |
|---|---|---|---|---|
| Etape de Purification | Protéines (mg) | Act. Spé. (cpm/mg)^{a} | Protéines (mg) | Facteur de purification |
| Extrait brut | 2200 | 3,6 | - | - |
| Mono Q 16/10 | 136 | 58 | 100 | 16 |
| Phényl-Superose | 32,6 | 175 | 72 | 49 |
| Superdex-200 | 3,1 | 870 | 34 | 240 |
| Mono Q 5/5 | 2,0 | 1000 | 25 | 280 |
| Electroélution PAGE-SDS | 0,1 | - | - | - |

| | | | | |
|---|---|---|---|---|
| ^{a} L'activité spécifique ne peut pas être mesurée précisément dans l'extrait brut en raison du niveau élevé d'échange ATP-pyrophosphate non dépendant de la phénylglycine. La valeur de l'activité spécifique a été calculée à partir du nombre d'unités retrouvées en sortie de première étape chromatographique rapporté à la quantité de protéines dans l'extrait brut. | | | | |

### 5.5.3. Autres activités portées par la Pristinamycine I synthase IV.

La purification de la peptide synthase responsable de l'incorporation de la phénylglycine décrite dans l'exemple 5.5.2. a conduit à une protéine pure de poids moléculaire 170 000. Cette protéine n'active pas les autres acides aminés testés, en particulier l'acide pipécolique ou l'acide 4-oxopipécolique. Une seconde préparation de cette protéine, effectuée dans les conditions décrites en 5.5.1.B., en supprimant toutefois l'étape de Phényl-Superose, à partir d'une autre culture de S. pristinaespiralis SP92 dont l'extrait brut a été préparé à la French Press comme décrit en 5.4.1B, a conduit à une protéine qui, en sortie de l'étape de Mono Q HR 5/5, était de pureté équivalente à celle obtenue à la même étape dans l'exemple décrit en 5.5.1.B., mais présentait un poids moléculaire de 250 000 environ en PAGE-SDS. Cette nouvelle préparation était compétente pour l'activation et la thioestérification de la phénylglycine, mais possédait en outre une activité d'échange ATP-pyrophosphate avec l'acide L-pipécolique (1 mM) dans le test d'échange analogue à celui décrit en 5.2.1.A. pour l'acide 3-hydroxypicolinique. D'autre part, il a pu être montré que la protéine de 170 000 ne possède pas d'activité d'échange ATP-pyrophosphate avec l'acide L-pipécolique, même dans les préparations encore très impures de la protéine. Il est à noter que S. pristinaespiralis SP92 produit naturellement en faible quantité un analogue de la Pristinamycine IA ayant un résidu acide pipécolique en lieu et place de l'acide 4-oxopipécolique. Ceci démontre donc que la peptide synthase responsable de l'incorporation de la phénylglycine (la Pristinamycine I synthase IV) catalyse aussi l'incorporation du résidu précédent (vraissemblablement l'acide pipécolique). La différence de poids moléculaire obtenue pour la Pristinamycine I synthase IV dans les deux préparations (170 000 et 250 000) est attribuée à un phénomène de clivage protéolytique partiel dans le premier cas, conduisant à la perte de l'activité d'activation de l'acide L-pipécolique.

### 5.5.4. Synthèse d'oligonucléotides à partir de la séquence protéique.

Cet exemple décrit comment à partir d'une séquence interne de la Pristinamycine I synthase IV, il est possible d'aller rechercher le gène correspondant à l'aide d'oligonucléotides convenablement choisis.

Une séquence interne de la Pristinamycine I synthase IV de 15 acides aminés a été identifiée après clivage au bromure de cyanogène de la protéine purifiée et purification des fragments obtenus sur colonne CLHP Vydac C18.

### Séquence interne à la protéine Pristinamycine 1 synthase IV : (Cf résidus 82 à 98 sur SEQ ID n° 16)

A partir de la région soulignée dans cette séquence et en fonction de la dégénérescence du code génétique spécifique des Streptomyces (cf. Exemple 8), le mélange d'oligonucléotides suivant a été synthétisé :

### Mélange correspondant à la partie soulignée de la séquence interne de la protéine Pristinamycine I synthase IV:

### 5.5.5. Marquage des mélanges d'oligonucléotides synthétiques et hybridation en Southern de l'ADN du cosmide pIBV3:

Cet exemple décrit comment des oligonucléotides spécifiques d'un gène de biosynthèse de la Pristinamycines I peuvent être marqués radioactivement puis hybridés avec une membrane sur laquelle l'ADN du cosmide pIBV3 a été transféré.

Le marquage des oligonucléotides est réalisé par transfert en position 5' terminale, du groupement [γ-³²P]phosphate de l'ATP, par la T4 polynucléotide kinase, comme indiqué dans 5.1.3.

Environ 500 ng du mélange d'oligonucléotides a été ainsi marqué au ³²P et a été utilisé pour hybrider en Southern de l'ADN de pIBV3 digéré par différentes enzymes. Ces hybridations ont permis de montrer que le gène de structure de la Pristinamycine I Synthase IV était porté par le cosmide pIBV3 et d'identifier la région contenant ce gène. Les Southerns et les hybridations ont été réalisés comme décrit dans l'exemple 5.1.4.

Les résultats d'hybridation ont permis de montrer que le cosmide pIBV3 possédait un fragment SphI de 6,6 kb, contenant la séquence homologue aux sondes synthétisées dans l'exemple 5.5.4.

### 5.6. Isolement du cosmide pIBV4 contenant le gène de structure de la FMN réductase (snaC).

Cet exemple illustre comment il est possible d'obtenir un cosmide, tel que construit à l'exemple 3., contenant au moins un gène de biosynthèse des PII.

### 5.6.1. Identification de la FMN réductase associée à la Pristinamycine IIA synthase.

Cet exemple illustre comment la protéine responsable de la réduction du FMN par le NADH pour former le FMNH₂ nécessaire à la réaction catalysée par la Pristinamycine IIA synthase peut être purifiée jusqu'à homogénéité à partir de S.pristinaespiralis SP92.

### 5.6.1.A. Dosage de l'activité FMN réductase.

Cet exemple illustre le dosage d'une activité de la voie de biosynthèse de la Pristinamycine IIA qui n'a encore jamais été décrite et qui possède la propriété remarquable de n'être exprimée que pendant la période de production des Pristinamycines. Il s'agit de la FMN réductase, appelée encore NADH:FMN oxydoréductase, qui catalyse la réduction du FMN en FMNH₂ (figure 13) en présence de NADH. Des FMN réductases catalysant la même réaction, spécifiques ou non pour le NADH ou le NADPH, associées à d'autres voies de biosynthèse ont été décrites par ailleurs (Duane et al., 1975, Jablonski et al., 1977, Watanabe et al., 1982).

Deux dosages sont utilisés pour détecter cette activité :

Le premier repose sur un couplage avec la Pristinamycine IIA synthase décrite dans l'exemple 5.1.1. et est utilisé pour les premières étapes de la purification. Les fractions enzymatiques à doser (0,002 à 0,005 unités) sont incubées 1 heure à 27°C dans un volume total de 500 µl de tampon bis-tris-propane 50mM pH 6,8 contenant NADH (500 µM), FMN (2 µM), Pristinamycine IIB (20 µM) et 0,01 unités de Pristinamycine IIA synthase décrite dans l'exemple 5.1.1. La Pristinamycine IIA formée est dosée par CLHP comme décrit à l'exemple 5.1.1.A.

L'unité d'activité enzymatique est définie comme la quantité d'enzyme nécessaire pour synthétiser 1 µmole de Pristinamycine IIA par minute dans les conditions décrites ci-dessus.

Le second est un dosage spectrophotométrique et ne peut être employé qu'avec des fractions purifiées au moins partiellement. Les fractions enzymatiques à doser (0,006 à 0,030 unités) sont incubées 13 min à 27°C dans un volume total de 3 ml de tampon bis-tris-propane 50 mM pH 6,8 contenant NADH (500 µM) et FMN (2 µM). Après 7 min d'incubation, 6 lectures de la densité optique à 340 nm espacées de 1 min sont effectuées contre une cuve de référence sans enzyme. L'activité en µmol/min est calculée en divisant la pente de diminution par min de la densité optique par un facteur 6,2 (densité optique de 1 mole de NADH à 340 nm).

L'unité d'activité enzymatique est définie comme la quantité d'enzyme nécessaire pour consommer 1 µmole de NADH par minute dans les conditions décrites ci-dessus.

### 5.6.1.B. Purification de la FMN réductase de S. pristinaespiralis SP92.

Cette expérience illustre comment une enzyme de S. pristinaespiralis SP92 intervenant dans la voie de biosynthèse de la Pristinamycine IIA peut être purifiée.

En utilisant les dosages décrits précédemment à l'exemple 5.6.1.A. la purification de la FMN réductase est réalisée comme décrit ci-dessous en prenant soin de congeler et conserver les fractions actives à -30°C entre chaque étape si nécessaire.

500 g d'un culot de centrifugation, lavé par un tampon phosphate 0,1 M pH 7,2 à 10 % v/v de glycérol, d'une culture de S. pristinaepiralis SP92 récoltée en début de phase de production des Pristinamycines sont repris par 1500 ml de tampon bis-tris-propane 50 mM pH 6,8 contenant 5 mM de DTT, 10 % v/v de glycérol et 0,2 mg/ml de lysozyme. La suspension ainsi obtenue est incubée pendant 45 min à 27°C puis centrifugée à 50 000 g durant 1 heure. L'extrait brut ainsi recueilli est fractionné par précipitation au sulfate d'ammonium. La fraction protéique précipitant entre 40 et 75 % de saturation est dessalée sur une colonne de Sephadex G25 Fine puis injectée dans le tampon pH 6,8 bis-tris-propane 50 mM, DTT 5 mM, 10 % v/v de glycérol sur une colonne (300 ml) de Q-Sepharose Fast Flow. Les protéines actives ne sont pas retenues sur la colonne et elles sont déssalées sur une colonne de Sephadex G25 Fine puis réinjectées dans le tampon pH 8,2 Tris-HCl 50 mM, DTT 5 mM, 10 % v/v de glycérol sur une colonne (35 ml) de Q-Sepharose Fast Flow et éluées avec un gradient linéaire de KCl (0 à 0,5M). Les fractions contenant l'activité enzymatique (mises en évidence grâce au premier test décrit à l'exemple 5.6.1.A) sont regroupées, dessalées sur une colonne de Sephadex G25 Fine puis injectées dans le tampon pH 8,2 Tris-HCl 50 mM, DTT 5 mM, 10 % v/v de glycérol sur une colonne MonoQ HR 10/10. Les protéines retenues sont éluées directement par le même tampon auquel on a ajouté 0,2 M de KCl. Elles sont recueillies dans un volume de 1 ml réinjecté immédiatement sur une colonne de Superdex 75 HR 10/30 éluée avec le tampon pH 6,8 bis-tris-propane 50 mM, DTT 1 mM, 10 % v/v de glycérol. Les fractions contenant l'activité recherchée (mises en évidence à partir de cette étape grâce au test spectrophotométrique comme décrit à l'exemple 5.6.1.A) sont rassemblées et le volume total du pool amené à 7 ml ; ces 7 ml sont injectés sur une colonne remplie de 8 ml de FMN agarose ; la colonne est lavée par le tampon pH 6,8 bis-tris-propane 50 mM, DTT 1 mM, 10 % v/v de glycérol, puis éluée avec le même tampon contenant 10 µM de FMN. Les fractions actives sont rassemblées, dessalées sur des colonnes PD-10 et injectées dans le tampon pH 8,2 tris-HCl 50 mM, DTT 5 mM, 10 % v/v de glycérol sur une colonne MonoQ HR 5/5 et éluées avec un gradient linéaire de KCl (0 à 0,25 M).

Aprés cette étape, l'enzyme est pure. En éléctrophorèse PAGE-SDS une seule bande assez large apparaît, centrée à un poids moléculaire estimé à 28 000, tandis qu'en chromatographie de perméation de gel Bio-Sil SEC 125 cette protéine forme un pic symétrique centré sur un poids moléculaire d'environ 30 000.

Pour le séquençage, la protéine est déssalée sur colonne Vydac C4 de 25 cm éluée avec un gradient linéaire de 30 à 70 % d'acétonitrile dans l'eau à 0,07 % d'acide trifluoroacétique.

**Tableau:**

| Purification de la FMN réductase | | | | | |
|---|---|---|---|---|---|
| Etapes de Purification | vol. (ml) | Protéines (mg) | Act. Spé^{a,b} (unités /mg) | Rendement (%) | Facteur de purification |
| Extrait brut | 1620 | 5100 | 0,004^{a} | 100 - | 1 |
| 40-75 % S.A. | 155 | 2930 | 0,005^{a} | 68 | 1,2 |
| Q-Seph. pH 6,8 | 357 | 180 | 0,058^{a} | 49 | 14 |
| Q-Seph. pH 8,2 | 153 | 15 | 0,36^{a} | 25 | 85 |
| MonoQ HR 10/10 | 1,0 | 8 | 0,50^{a} 4,4^{b} | 19 | 120 |
| Superdex 75 | 1,5 | 3,1 | 7,4^{b} | 12 | 200 |
| FMN-agarose | 7,5 | 0.28 | 96^{b} | 14 | 2600 |
| MonoQ HR 5/5 | 3,0 | 0,29 | 68^{b} | 11 | 1900 |
| Bio-Sil 125 | 7,5 | 0,18 | 106^{b} | 10 | 2900 |

| | | | | | |
|---|---|---|---|---|---|
| a: dosage couplé à la Pristinamycine IIA synthase | | | | | |
| b: dosage spectrophotométrique | | | | | |

Le facteur de purification est calculé d'après l'augmentation de l'activité spécifique des fractions au cours de la purification.

### 5.6.2. Réalisation d'oligonucléotides à partir de la séquence protéique.

Cet exemple décrit comment à partir des séquences NH₂-terminales et internes de la protéine FMN réductase, il est possible de synthétiser des oliginucléotides.

La séquence NH₂-terminale de la FMN réductase a été réalisée par microséquençage comme décrit dans l'exemple 5.1.2. Une trentaine de résidus ont été ainsi identifiés.

(Des séquences NH₂-terminales démarrant au 4ème et au 11ème résidus ont été également trouvées dans l'échantillon séquencé.)

Deux séquences internes à la FMN réductase, de 13 et 21 acides aminés, ont été également identifiées, après hydrolyse trypsique et purification des fragments obtenus sur colonne Vydac C18.

### Séquence NH₂-terminale de la protéine FMN réductase : (Cf résidus 2 à 25 sur SEQ ID n° 7)

### Séquences internes de la protéine FMN réductase : (Cf résidus 102 à 122 sur SEQ ID n° 7)

(Cf résidus 149 à 161 sur SEQ ID n° 7)

A partir des régions soulignées dans chacune des séquences et en fonction de la dégénérescence du code génétique spécifique des Streptomyces (cf. Exemple 8), les mélanges d'oligonucléotides suivants ont été synthétisés :

### Mélange correspondant à la séquence NH₂-terminale de la protéine FMN réductase :

### Mélanges correspondant aux séquences internes de la protéine FMN réductase :

### 5.6.3. Marquage des mélanges d'oligonucléotides synthétiques et hybridation des banques d'ADN génomique de S. pristinaespiralis SP92.

Cet exemple décrit comment des oligonucléotides spécifiques d'un gène de biosynthèse des pristinamycines peuvent être marqués radioactivement puis hybridés avec des membranes sur lesquelles l'ADN des banques génomiques de S. pristinaespiralis a été transféré.

Le marquage des oligonucléotides est réalisé par transfert en position 5' terminale, du groupement [γ-³²P]phosphate de l'ATP marqué, par la T4 polynucléotide kinase, comme indiqué dans 5.1.3.

Environ 2 x 500 ng de chaque mélange d'oligonucléotides ont été ainsi marqués au ³²P et ont été utilisés pour hybrider chacune des deux banques.

L'hybridation des membranes de chaque banque a été réalisée comme indiqué dans l'exemple 5.1.3.

### 5.6.4. Isolement du cosmide pIBV4 et détermination de la région contenant le gène de structure de la FMN réductase (snaC).

Le cosmide pIBV4 a été isolé d'un clone de la banque réalisée dans la souche d'E.coli HB101, ayant hybridé avec les trois mélanges d'oligonucléotides simultanément.

Ce cosmide a été purifié comme décrit dans l'exemple 2. Il contient un insert d'ADN génomique de S. pristinaespiralis SP92 dont la taille à été estimée à 48 kb. Une cartographie (figure 7) a été établie à partir de digestions avec différentes enzymes de restriction, comme indiqué dans 5.1.4.

Des hybridations en Southern de l'ADN de pIBV4 digéré par différentes enzymes, avec les mélanges d'oligonucléotides, ont permis d'identifier la région contenant snaC, le gène de structure de la FMN réductase. Les Southerns et les hybridations ont été réalisés comme décrit dans l'exemple 5.1.4.

Les résultats d'hybridation ont permis de montrer que le cosmide pIBV4 possédait un fragment BamHI-BamHI de 4 kb, contenant les séquences homologues aux sondes synthétisées dans l'exemple 5.6.3.

### 5.7. Mise en évidence de la présence du gène de structure de la p-aminophénylalanine (phényl-N)-méthyltransférase sur le cosmide pIBV2.

Cet exemple illustre comment il est possible, à partir d'une protéine purifiée, d'identifier le gène de structure correspondant parmi les gènes déjà analysés et séquencés comme décrit à l'exemple 6.7 et 7.8, et également exprimés chez E.coli comme décrit à l'exemple 11.

### 5.7.1. Identification et purification de la protéine impliquée dans la méthylation de la p-aminophénylalanine en p-diméthylaminophénylalanine.

Cet exemple illustre comment la protéine responsable de la méthylation de la p-aminophénylalanine en p-diméthylaminophénylalanine [la p-aminophénylalanine (phényl-N)-méthyltransférase], peut être purifiée jusqu'à homogénéité, à partir de la souche S. pristinaespiralis SP92 et comment on peut également l'obtenir pure à partir d'une souche recombinante de E. coli (exemple 11).

### 5.7.1.A. Dosage de l'activité de méthylation de la p-aminophénylalanine en p-méthylaminophénylalanine et de l'activité de méthylation de la p-méthylaminophénylalanine en p-diméthylaminophénylalanine.

Cet exemple illustre le dosage de deux activités terminales de la biosynthèse de la p-diméthylaminophénylalanine, composant de pristinamycine IA. Ces activités n'ont jamais encore été décrites et possèdent la propriété remarquable de n'être exprimées que pendant la période de production des pristinamycines. Il s'agit de la méthylation de la p-aminophénylalanine en p-méthylaminophénylalanine (méthylation 1), d'une part, et de la méthylation de la p-méthylaminophénylalanine en p-diméthylaminophénylalanine (méthylation 2), ces deux activités utilisant la SAM comme donneur de groupement méthyle (figure 14).

Les fractions enzymatiques à doser (1 à 20 unités) sont incubées pendant 30 min à 27°C dans un volume total de 200 µl de tampon pH 6,8 bis-tris-propane 50 mM contenant de la SAM (200 µM) dont le groupement méthyle est marqué radioactivement avec l'isotope 14 de l'atome de carbone (2 Ci/mol), en présence de p-amino-L-phénylalanine (1 mM) pour le dosage de la méthylation 1 ou de p-méthylamino-L-phénylalanine (2,5 mM) pour le dosage de la méthylation 2.

La réaction est arrêtée par ajout de 16 µl d'acide chlorhydrique à 37 % puis de 20 µl d'heptanesulfonate de sodium à 240 g/l. Après centrifugation, 150 µl de surnageant sont injectés dans le système de CLHP en mode gradient suivant :
- phase mobile : éluant A =1,2 g d'heptanesulfonate de sodium + 2,5 ml d'acide acétique glacial + eau (qsp 1000 ml)
éluant B =1,2 g d'heptanesulfonate de sodium + 2,5 ml d'acide acétique glacial + 300 ml d'acétonitrile + eau (qsp 1000 ml)

| | | |
|---|---|---|
| gradient : | t (min) | % B |
| | 0 | 30 |
| | 16 | 30 |
| | 17 | 100 |
| | 20 | 100 |
| | 21 | 30 |
| | 25 | 30 |

- phase stationnaire : colonne Nucléosil C18 5 µm 150 x 4,6 mm (Macherey-Nagel)

En sortie de colonne, les substrats et produits de la réaction enzymatique sont quantifiés par absorption à 254 nm. Cette détection est couplée à une détection radiochimique en ligne au moyen d'un détecteur Berthold LB506 équipé d'une cellule à scintillation solide de type GT400-U4. Ceci permet de suivre spécifiquement l'incorporation de groupements méthyles radioactifs dans les produits de la réaction.

L'unité d'activité enzymatique pour la méthylation 1 (pour la méthylation 2) est définie comme la quantité d'enzyme nécessaire pour incorporer 1 nmole de groupement méthyle dans la p-aminophénylalanine (dans la p-méthylaminophénylalanine).

### 5.7.1.B. Purification à partir de S. pristinaespiralis SP92 de la N-méthyltransférase SAM-dépendante catalysant la méthylation de la p-aminophénylalanine en p-diméthylaminophénylalanine [p-aminophénylalanine (phényl-N)-méthyltransférase].

Cette expérience illustre comment une enzyme de S. pristinaespiralis SP92 intervenant dans la voie de biosynthèse de la Pristinamycine IA peut être purifiée.

En utilisant le dosage décrit précédemment dans l'exemple 5.7.1.A, la purification de la N-méthyltransférase SAM-dépendante est réalisée comme décrit ci-dessous en prenant soin de congeler et conserver à -70°C les fractions actives entre chaque étape si nécessaire.

240 g d'un culot de centrifugation, lavé par un tampon pH 7,2 phosphate 100 mM, 1 mM PMSF, 5 mM EDTA, 5 mM EGTA, 0,5M KCl, 10 % v/v de glycérol, d'une culture de S. pristinaespiralis SP92 récoltée en début de phase de production des Pristinamycines sont repris dans 480 ml de tampon pH 8,0 Tris-HCl 0,1 M contenant 4 mM DTE, 5 mM benzamidine, 0,2 mM Pefabloc, 100 µg/l E-64, 2 mg/l leupeptine, 1 mM EDTA, 1 mM EGTA, 2 mg/l STI, 2 mg/l aprotinine, 20 % v/v glycérol et 2 mg/ml lysozyme, ce tampon étant maintenu à +4°C. La suspension ainsi obtenue est vigoureusement agitée à 4°C. Après 30 min d'agitation, sont ajoutés 0,2 mg/ml désoxyribonucléase 1 et 5 mM MgCl₂. Après 90 min d'agitation, l'extrait est centrifugé pendant 1 heure à 50 000 g. Le surnageant est réparti en 3 fractions d'environ 180 ml. Chacune est dessalée par perméation de gel sur une colonne de 500 ml de Sephadex G 25 Fine équilibrée au débit naturel dans un tampon pH 6,8 bis-tris 20 mM, contenant 4 mM DTE, 5 mM benzamidine, 0,2 mM Pefabloc, 100 µg/l E-64, 2 mg/l leupeptine, 1 mM EDTA, 1 mM EGTA, 2 mg/l STI, 2 mg/l aprotinine. 20 % v/v glycérol. L'éluat protéique est alors chromatographié (400 mg de protéine à chaque cycle) sur une colonne MonoQ HR 16/10 à un débit de 6 ml/min avec un gradient linéaire croissant de chlorure de sodium (0 à 0,3 M) dans un tampon pH 6,8 bis-tris 20 mM, contenant 4 mM DTE, 2 mM benzamidine, 100 µg/l E-64, 2 mg/l leupeptine, 20 % v/v glycérol. A la sortie de la colonne, les fractions sont complétées par 10 % v/v de tampon pH 6,8 bis-tris 20 mM, contenant 4 mM DTE, 30 mM benzamidine, 2mM Pefabloc, 100 µg/l E-64, 2 mg/l leupeptine, 5 mM EDTA, 5 mM EGTA, 10 mg/l STI, 10 mg/l aprotinine, 20 % v/v glycérol. Dans ces conditions, les deux activités de méthylation (1 et 2) sont détectées de façon identique dans les fractions d'exclusion et les premières fractions d'élution. Ces fractions sont regroupées, concentrées par ultrafiltration sur CentriPrep 10. Ce concentrat est amené à 0,85 M de sulfate d'ammonium puis chromatographié (20 à 80 mg à chaque cycle) sur une colonne Phényl-Superose HR 10/10 à un débit de 1 ml/min avec un gradient linéaire décroissant de sulfate d'ammonium (0,85 à 0 M) dans un tampon pH 6,8 bis-tris 50 mM, contenant 4 mM DTE, 2 mM benzamidine, 100 µg/l E-64, 2 mg/l leupeptine, 1 mM EDTA, 1 mM EGTA, 10 % v/v glycérol. A la sortie de la colonne, les fractions sont complétées par 10 % v/v de tampon pH 6,8 bis-tris 50 mM, contenant 4 mM DTE, 30 mM benzamidine, 2mM Pefabloc, 100 µg/l E-64, 2 mg/l leupeptine, 1 mM EDTA, 1 mM EGTA, 10 mg/l STI, 10 mg/l aprotinine, 10 % v/v glycérol. Dans ces conditions, les deux activités de méthylation (1 et 2) sont détectées de façon identique dans les fractions d'élution correspondant à environ 0,15 M sulfate d'ammonium. Ces fractions sont regroupées, concentrées par ultrafiltration sur Centricon 10, dessalées sur colonnes PD-10 équilibrées dans un tampon pH 8,2 (à 5°C) Tris 50 mM, contenant 4 mM DTE, 2 mM benzamidine, 100 µg/l E-64, 2 mg/l leupeptine, 20 % v/v glycérol, puis chromatographiées (environ 10 mg à chaque cycle) sur colonne MonoQ HR 5/5 équilibrée dans le même tampon à un débit de 1 ml/min. Dans ces conditions, les deux activités ne sont pas retenues sur la colonne. A la sortie de la colonne, les fractions d'exclusion contenant donc ces deux activités sont complétées par 10 % v/v de tampon pH 8,2 Tris 50 mM, contenant 4 mM DTE, 30 mM benzamidine, 2 mM Pefabloc, 100 µg/l E-64, 2 mg/l leupeptine, 1 mM EDTA, 1 mM EGTA, 20 % v/v glycérol. Ces fractions sont alors concentrées par ultrafiltration sur Centricon 10 puis chromatographiées sur colonne TSK G2000 SW 300 x 7,5 mm 10 µm équilibrée à un débit de 0,5 ml/min dans un tampon pH 7,0 Hepes 50 mM, contenant 4 mM DTE, 0.2 mM Pefabloc, 1 mM EDTA, 1 mM EGTA, 10 % v/v glycérol, 0.15 M chlorure de sodium. Les deux activités co-éluent dans cette technique à un temps de rétention correspondant à un poids moléculaire proche de 30 000. Après cette étape, une protéine majoritaire est visible en SDS-PAGE. Elle se situe aux environs de 32 000.

**Tableau :**

| | | | | | |
|---|---|---|---|---|---|
| Purification de l'enzyme de méthylation de la p-aminophénylalanine en p-diméthylaminophénylalanine. | | | | | |

| Etapes de purification | vol. (ml) | Protéines (mg) | Act. Spé (unités ^{a}/mg) | Rendement (%) | Facteur de purification |
|---|---|---|---|---|---|
| Extrait brut | 510 | 1800 | 29 | - | - |
| G25 Fine | 560 | 1560 | 34 | 102 | 1,17 |
| MonoQ HR 16/10 | 670 | 82 | 430 | 67 | 14,8 |
| Phényl-Superose | 10 | 3,48 | 6300 | 42 | 217 |
| MonoQ HR5/5 | 7 | 0,88 | 17200 | 29 | 593 |
| TSK G2000 | 0,8 | 0.113 | 40300 | 8,7 | 1390 |

| | | | | | |
|---|---|---|---|---|---|
| ^{a} Il s'agit des unités d'activité enzymatique pour la méthylation 1. A chaque étape la valeur des unités d'activité enzymatique pour la méthylation 2 était égale à 120 % de celle des unités pour la méthylation 1. | | | | | |

Le facteur de purification est calculé d'après l'augmentation de l'activité spécifique des fractions au cours de la purification.

### 5.7.1.C. Purification à partir de E. coli::pVRC706 de la protéine recombinante de S. pristinaespiralis SP92 présentant l'activité N-méthyltransférase SAM-dépendante catalysant la méthylation de la p-aminophénylalanine en p-diméthylaminophénylalanine.

Cette expérience illustre comment une enzyme de S. prisinaespiralis SP92 intervenant dans la voie de biosynthèse de la Pristinamycine IA et exprimée chez E. coli par clonage du gène papM peut être purifiée.

En utilisant le dosage décrit précédemment dans l'exemple 5.7.1.A., nous avons montré que les extraits bruts de la souche recombinante E. coli::pVRC706 présentent une forte activité pour la méthylation 1 et pour la méthylation 2, alors que dans la souche E. coli témoin (pMTL23) aucune de ces deux activités n'a été détectée. La purification de la p-aminophénylalanine (phényl-N)-méthyltransférase SAM-dépendante catalysant la méthylation de la p-aminophénylalanine en p-diméthylaminophénylalanine a été alors réalisée.

Dans les mêmes conditions que celles décrites dans l'exemple 5.7.1.B., excepté une étape de chromatographie qui a été supprimée (l'étape de purification sur MonoQ HR 5/5), nous avons purifié à homogénéité une protéine qui présente un poids moléculaire en chromatographie sur colonne TSK G2000 et en SDS-PAGE identique à celui présenté par la protéine purifiée dans l'exemple 5.7.1.B.

**Tableau :**

| | | | | | |
|---|---|---|---|---|---|
| Purification de l'enzyme de méthylation de la p-aminophénylalanine en p-diméthylaminophénylalanine à partir de la souche E. coli::pVRC706. | | | | | |

| Etape de Purification | vol. (ml) | Protéines (mg) | Act. Spé (unités ^{a}/mg) | Rendement (%) | Facteur de purification |
|---|---|---|---|---|---|
| Extrait brut | 15 | 190 | 235 | - | - |
| G2S Fine | 22 | 175 | 231 | 91 | 1 |
| MonoQ HR16/10 | 24 | 13,4 | 2100 | 63 | 8,9 |
| Phényl-Superose | 3,0 | 0,39 | 35500 | 31 | 145 |
| TSK G2000 | 0,8 | 0,092 | 45200 | 9,3 | 192 |

| | | | | | |
|---|---|---|---|---|---|
| ^{a} Il s'agit des unités d'activité enzymatique pour la méthylation 1. A chaque étape la valeur des unités d'activité enzymatique pour la méthylation 2 était égale à 120 % de celle des unités pour la méthylation 1. | | | | | |

Le facteur de purification est calculé d'après l'augmentation de l'activité spécifique des fractions au cours de la purification.

5.7.2. Identification du gène de structure de la p-aminophénylalanine (phényl-N)-méthyltransférase.

La séquence NH₂-terminale de la protéine de 32 000 purifiée à l'exemple 5.7.1.B. a été déterminée par microséquençage comme décrit dans l'exemple 5.1.2. Une dizaine de résidus ont été ainsi déterminés :

La séquence NH₂-terminale de la protéine de 32 000 purifiée à l'exemple 5.7.1.C a été déterminée par microséquençage comme décrit dans l'exemple 5.1.2. Une dizaine de résidus ont été ainsi déterminés :

Il s'agit dans les deux cas des mêmes résidus et cette séquence correspond exactement au début de la séquence protéique qui est déduite de la séquence du gène papM (Cf résidus 2 à 11 sur SEQ ID n° 10). La p-aminophénylalanine (phényl-N)-méthyltransférase purifiée est donc la protéine PapM.

### EXEMPLE 6 : Sous clonage des fragments d'ADN clonés dans des cosmides tels que préparés dans l'exemple 3 et contenant les gènes d'intérêt.

Cet exemple illustre comment à partir des cosmides construits comme décrit à l'exemple 3 et contenant des gènes de biosynthèse des Pristinamycines II ou des Pristinamycines I, il est possible de sous cloner des fragments d'ADN contenant ces gènes.

Ces sous clonages ont été effectués afin de pouvoir réaliser ultérieurement la séquence nucléique des gènes identifiés ainsi que les différentes constructions présentées dans les exemples suivants.

### 6.1. Isolement du fragment EcoRI-BglII de 5.5 kb contenant le gène de structure de l'acide 3-hydroxypicolinique-AMP ligase.

Cet exemple décrit comment à partir du cosmide pIBV2, contenant le gène de structure de l'acide 3-hydroxypicolinique-AMIP ligase, il est possible de sous cloner un fragment d'ADN de taille inférieure, contenant ce gène.

Environ 10 µg du cosmide pIBV2 ont été coupés successivement par les enzymes de restriction BglII et EcoRI (New England Biolabs) dans les conditions préconisées par le fournisseur. Les fragments de restrictions ainsi obtenus ont été séparés par électrophorèse sur gel d'agarose à 0.8 % et le fragment BglII-EcoRI de 5,5 kb a été isolé par électroélution comme décrit dans Maniatis et al. (1989).

Environ 100 ng de pUC19 (Yanisch-Perron et al., 1985) coupés par BamHI et EcoRI ont été ligués avec 200 ng du fragment BglII-EcoRI de 5,5 kb, dans les conditions décrites dans l'exemple 3.3.

Après transformation de la souche TG1 et sélection des transformants sur milieu LB solide contenant 150 µg/ml d'ampicilline et 20 µg/ml de X-gal, selon la technique décrite par Maniatis et al. (1989), un clone portant le fragment souhaité a été isolé. Le plasmide recombinant a été nommé pVRC402. Sa carte de restriction est présentée dans la figure 15(A). Il a été montré par hybridation, dans l'exemple 5.2., que le fragment EcoRI-BglII de 5,5 kb, contient le gène de structure de l'acide 3-hydroxypicolinique-AMP ligase de S. pristinaespiralis SP92. Le plasmide pVRC402 contient donc le gène de structure de l'acide 3-hydroxypicolinique-AMP ligase de S. pristinaespiralis SP92.

### 6.2. Isolement d'un fragment BgIII-BgIII de 4.6 kb à partir du cosmide pIBV2.

Cet exemple décrit comment à partir du cosmide pIBV2, il est possible de sous cloner un fragment d'ADN de taille inférieure, dans le but d'identifier dans les régions adjacentes au gène de structure de l'acide 3-hydroxypicolinique-AMP ligase, la présence d'autres gènes impliqués dans la biosynthèse des Pristinamycines L

Les différentes étapes de clonage ont été réalisées comme décrit plus haut.

Environ 10 µg du cosmide pIBV2 ont été coupés par BglII. Les fragments de restrictions ainsi obtenus ont été séparés par électrophorèse sur gel d'agarose à 0.8 % et le fragment BglII-BglII de 4.6 kb a été isolé par électroélution.

Environ 100 ng de pUC19 coupés par BamHI ont été ligués avec 200 ng du fragment BglII-BglII.

Un clone portant le fragment souhaité a été isolé après transformation de la souche TG1 comme décrit dans l'exemple 6.1. Le plasmide recombinant a été nommé pVRC501. Sa carte de restriction est présentée dans la figure 15(B).

### 6.3. Isolement du fragment BamHI-BamHI de 6 kb contenant les gènes de structure des deux sous-unités de la Pristinamycine IIA synthase.

Cet exemple décrit comment à partir du cosmide pIBV1, il est possible de sous cloner un fragment d'ADN de taille inférieure, contenant les gènes de structure des deux sous-unités de la Pristinamycine IIA-synthase.

Les différentes étapes de clonage ont été réalisées comme décrit plus haut.

Environ 10 µg du cosmide pIBV1 ont été coupés par BamHI. Les fragments de restrictions ainsi obtenus ont été séparés par électrophorèse sur gel d'agarose à 0.8 % et le fragment BamHI de 6 kb a été isolé par électroélution.

Environ 100 ng de pBKS⁻ (Stratagene Cloning Systems, La Jolla Californie) coupés par BamHI, ont été ligués avec 200 ng du fragment BamHI de 6 kb.

Un clone portant le fragment souhaité a été isolé après transformation de la souche TG1. Le plasmide recombinant a été nommé pXL2045. Sa carte de restriction est présentée dans la figure 16. Il a été montré par hybridation, dans l'exemple 5.1, que le fragment BamHI de 6 kb, contient les gènes snaA et snaB, codant pour les deux sous unités de la Pristinamycine IIA-synthase de S. pristinaespiralis SP92. Le plasmide pXL2045 contient donc les gènes snaA et snaB, codant pour les deux sous unités de la Pristinamycine IIA-synthase de S. pristinaespiralis SP92.

### 6.4. Isolement du fragment SphI de 6.2 kb contenant une partie du gène de structure de la Pristinamycine I synthase II.

Cet exemple décrit comment à partir du cosmide pIBV3, il est possible de sous cloner un fragment d'ADN de taille inférieure, contenant une partie du gène de structure de la Pristinamycine I synthase II.

Les différentes étapes de clonage ont été réalisées comme décrit plus haut.

Environ 10 µg du cosmide pIBV3 ont été coupés par SphI. Les fragments de restrictions ainsi obtenus ont été séparés sur gel d'agarose 0.8 % et le fragment SphI de 6,2 kb a été isolé par la technique du kit "Geneclean" commercialisé par la société Bio101-Ozyme.

Environ 100 ng de pUC19 coupés par SphI ont été ligués avec 200 ng du fragment SphI de 6,2 kb.

Un clone portant le fragment souhaité a été isolé après transformation de la souche TG1. Le plasmide recombinant a été nommé pVRC1105. Sa carte de restriction est présentée dans la figure 17.

### 6.5. Isolement du fragment SphI de 8.4 kb contenant une partie du gène de structure de la Pristinamycine I synthase III.

Cet exemple décrit comment à partir du cosmide pIBV3, il est possible de sous cloner un fragment d'ADN de taille inférieure, contenant une partie du gène de structure de la Pristinamycine I synthase III.

Les différentes étapes de clonage ont été réalisées comme décrit plus haut.

Environ 10 µg du cosmide pIBV3 ont été coupés par SphI. Les fragments de restrictions ainsi obtenus ont été séparés sur gel d'agarose 0.8 % et le fragment SphI de 8,4 kb a été isolé par la technique du kit "Geneclean" commercialisé par la société Bio101-Ozyme.

Environ 100 ng de pUC19 coupés par SphI ont été ligués avec 200 ng du fragment SphI de 8,4 kb.

Un clone portant le fragment souhaité a été isolé après transformation de la souche TG1. Le plasmide recombinant a été nommé pVRC1106. Sa carte de restriction est présentée dans la figure 18.

### 6.6. Isolement du fragment SphI de 6.6 kb contenant une partie du gène de structure de la Pristinamycine I synthase IV.

Cet exemple décrit comment à partir du cosmide pIBV3, il est possible de sous cloner un fragment d'ADN de taille inférieure, contenant une partie du gène de structure de la Pristinamycine I synthase IV.

Les différentes étapes de clonage ont été réalisées comme décrit plus haut.

Environ 10 µg du cosmide pIBV3 ont été coupés par SphI. Les fragments de restrictions ainsi obtenus ont été séparés sur gel d'agarose 0.8 % et le fragment SphI de 6,6 kb a été isolé par la technique du kit "Geneclean" commercialisé par la société Bio101-Ozyme.

Environ 100 ng de pUC19 coupés par SphI ont été ligués avec 200 ng du fragment SphI de 6,6 kb.

Un clone portant le fragment souhaité a été isolé après transformation de la souche TG1. Le plasmide recombinant a été nommé pVRC1104. Sa carte de restriction est présentée dans la figure 19.

### 6.7. Isolement du fragment HindIII-HindIII de 17 kb contenant le cosmide pHC79 et portant les gènes situés en amont de l'acide 3-hydroxypicolinique-AMP ligase (Pristinamycine I synthase I).

Cet exemple décrit comment à partir du cosmide pIBV2, contenant le gène de structure de l'acide 3-hydroxypicolinique-AMP ligase, il est possible de déléter une grande partie de ce cosmide pour ne garder que la partie située en amont de l'acide 3-hydroxypicolinique-AMP ligase.

Environ 200 ng du cosmide pIBV2 ont été coupés par l'enzyme de restriction HindIII. L'enzyme a été dénaturé 30 min à 85°C, comme préconisé par le fournisseur. Le cosmide pIBV2 ainsi digéré a été précipité à l'éthanol comme décrit dans Maniatis et al. (1989) et religuaturé sur lui-même dans un volume de 50 µl.

Après transformation de la souche TG1 et sélection des transformants sur LB solide + 150 µg/ml d'ampicilline selon la technique décrite par Maniatis et al. (1989), un clone contenant le cosmide pHC79 et la partie située en amont de l'acide 3-hydroxypicolinique-AMP ligase (l'ensemble correspondant à une taille d'environ 17 kb) a été isolé. Le plasmide recombinant a été nommé pVRC900. Sa carte de restriction est présentée dans la figure 20.

### 6.8. Isolement du fragment BamHI-SstI de 1.5 kb issu du cosmide pIBV3.

Cet exemple décrit comment à partir du cosmide pIBV3, contenant le gène snaA codant pour la grande sous-unité de la PIIA synthase, il est possible de sous-cloner un fragment d'ADN situé en amont pour l'étudier et le séquencer.

Environ 10 µg du cosmide pIBV3 ont été coupés successivement par les enzymes de restriction SstI et BamHI. Les fragments de restrictions ainsi obtenus ont été séparés sur gel d'agarose 0.8 % et le fragment BamHI-SstI de 1,5 kb a été isolé par la technique du kit "Geneclean" commercialisé par la société Bio101-Ozyme.

Environ 100 ng du plasmide pDH5 (Hilleman et al 1991) coupés par BamHI et SstI ont été ligués avec 200 ng du fragment BamHI-SstI, dans les conditions décrites dans l'exemple 3.3.

Après transformation de la souche TG1 et sélection des transformants sur LB solide + 150 µg/ml d'ampicilline + X-gal, selon la technique décrite par Maniatis et al. (1989), un clone portant le fragment souhaité a été isolé. Le plasmide recombinant a été nommé pVRC1000. Sa carte de restriction est présentée dans la figure 21.

### 6.9. Isolement du fragment BamHI-BamHI de 4 kb contenant le gène de structure de la FMN réductase.

Cet exemple décrit comment à partir du cosmide pIBV4, contenant le gène de structure de la FMN réductase (snaC), il est possible de sous-cloner un fragment d'ADN de taille inférieure, contenant ce gène.

Les différentes étapes de clonage ont été réalisées comme décrit plus haut.

Environ 10 (µg du cosmide pIBV4 ont été coupés par l'enzyme de restriction BamHI. Les fragments de restrictions ainsi obtenus ont été séparés sur gel d'agarose 0.8 % et le fragment BamHI-BamHI de 4 kb a été isolé par électroélution.

Environ 100 ng de pUC19 coupés par BamHI ont été ligués avec 200 ng du fragment BamHI-BamHI de 4 kb.

Après transformation de la souche E.coli DH5α (supE44 DlacU169 (f80lacZDM15) hsdR17 recA1 endA1 gyrA96 thi-1 relA1) (Hanahan, 1983) et sélection des transformants sur LB solide + 150 µg/ml d'ampicilline + X-gal, selon la technique décrite par Maniatis et al. (1989), un clone portant le fragment souhaité a été isolé. Le plasmide recombinant a été nommé pVRC509. Sa carte de restriction est présentée dans la figure 22..

### EXEMPLE 7 : Séquence des fragments d'ADN isolés contenant les gènes de biosynthèse des Pristinamycines de S. pristinaespiralis SP92.

Cet exemple illustre le séquençage de fragments d'ADN portant d'une part des gènes impliqués dans la biosynthèse des Pristinamycines de la famille des Pristinamycines I et d'autre part des gènes impliqués dans la biosynthèse des Pristinamycines de la famille des Pristinamycines II de la souche S. pristinaespiralis.

### 7.1. Séquençage d'un fragment BamHI-XhoI de 5 kb.

Cet exemple illustre comment la séquence nucléotidique d'un fragment contenant les gènes snaA et snaB de S. pristinaespiralis SP92 peut être obtenue.

Le fragment BamHI-XhoI fait partie du fragment BamHI-BamHI de 6 kb qui a été cloné dans le phasmide pBKS- pour donner le plasmide pXL2045 décrit dans l'exemple 6.3. Des sous-fragments de cet insert BamHI-XhoI de 5 kb ont ensuite été obtenus par digestion enzymatique puis sous-clonés dans les phages M13mp18 ou M13mp19 (Messing et al, 1981) dans les deux orientations. Les sites de sous-clonage utilisés sont les suivants : Ec*o*RI, PstI, PstI, NruI, EcoRI, NruI, NotI, SalI, SstI, XhoI, SalI et XhoI et sont représentés sur la figure 16.

Ces différents inserts ont été séquencés par la méthode de réaction de terminaison de chaîne en utilisant comme amorce de synthèse le primer universel (Maniatis et al, 1989) ou des oligonucléotides synthétisés (comme il est décrit dans l'exemple 5), et complémentaires d'une séquence de 20 nucléotides de l'insert à séquencer.

Le recouvrement entre ces différents inserts a permis d'établir la séquence nucléotidique totale sur les deux brins du fragment BamHI-XhoI qui comprend 5392 pb (SEQ ID n° 1).

### 7.2. Séquençage d'une région de 1870 bp du fragment-EcoRI-BglII de 5.5 kb.

Cet exemple illustre comment la séquence nucléotidique d'un fragment contenant le gène snbA de S. pristinaespiralis SP92 peut être obtenue.

La région de 1870 pb séquencée fait partie du fragment EcoRI-BglII de 5,5 kb qui a été cloné dans le plasmide pUC19 pour donner le plasmide pVRC402 décrit dans l'exemple 6 (figure 15(A). Des sous-fragments de l'insert EcoRI-BglII de 5,5 kb ont été obtenus par coupure enzymatique puis sous-clonés dans des phages M13mp18 ou M13mp19 dans les deux orientations. Les sites de sous-clonage sont HindIII, PstI et HindIII et sont représentés sur la figure 15(A).

Le recouvrement entre ces fragments a permis d'établir la séquence totale de la région Sau3A-Sau3A qui comprend 1870 pb (SEQ ID n° 5).

### 7.3. Séquence d'une région de 1830 pb dans le fragment BglII-BglII de 4.6 kb.

Cet exemple illustre comment la séquence nucléotidique d'un fragment adjacent à celui qui contient le gène snbA de S. pristinaespiralis SP92 peut être obtenue.

Cette séquence a été réalisée par sous-clonage des fragments BamHI-PstI de 1 kb et PstI-EcoRI de 2,1 kb (figure 15(B)) à partir du pVRC501 (Exemple 6) dans les vecteurs M13mp18 et M13mp19. Le site PstI a été traversé par sous-clonage d'un fragment Sau3A-Sau3A de 423 pb chevauchant ce site, puis séquençage. La séquence de 1830 pb ainsi obtenue est représentée sur la (SEQ ID n° 6).

### 7.4. Séquençage de deux régions de 227 pb et 247 pb du fragment SphI de 6.2 kb.

Cet exemple illustre comment la séquence nucléotidique de fragments contenant une partie du gène de structure de la Pristinamycine I synthase II (snbC) de S. pristinaespiralis peut être obtenue.

Les régions de 227 et 247 pb séquencées font parties du fragment SphI de 6.2 kb qui a été cloné dans le plasmide pUC19 pour donner le plasmide pVRC1105 décrit dans l'exemple 6.4. (figure 17). Des sous-fragments de l'insert SphI de 6.2 kb ont été obtenus par coupure enzymatique puis sous-clonés dans des phages M13mp18 ou M13mp19 dans les deux orientations. Les sites de sous-clonage sont XhoI, PstI et BglII et sont représentés sur la figure 17. Le fragment PstI-BglII de 227 pb a été entièrement séquencé, et 247 pb ont été séquencées à partir du fragment XhoI de 900 pb : ces séquences sont présentées sur les SEQ ID n° 11 et 12.

### 7.5. Séquençage de deux régions de 192 pb et 474 pb du fragment SphI de 8.4 kb.

Cet exemple illustre comment la séquence nucléotidique de fragments contenant des parties du gène de structure de la Pristinamycine I Synthase III (snbD) de S. pristinaepiralis peut être obtenue.

Les régions de 192 et 474 pb séquencées font parties du fragment SphI de 8,4 kb qui a été cloné dans le plasmide pUC19 pour donner le plasmide pVRC1106 décrit dans l'exemple 6.5. (figure 18). Des sous-fragments de l'insert SphI de 8,4 kb ont été obtenus par coupure enzymatique puis sous-clonés dans des phages M13mp18 ou M13mp19 dans les deux orientations. Les sites de sous-clonage sont XhoI, PstI, SphI et BglII et sont représentés sur la figure 18.

Les fragment BglII-SphI de 192 pb et PstI-XhoI de 474 pb ont été entièrement séquencés : ces séquences sont présentées sur les SEQ ID n° 13 et 14. -

### 7.6. Séquençape de deux régions de 485 pb et 291 pb du fragment SphI de 6,6 kb.

Cet exemple illustre comment la séquence nucléotidique de fragments contenant des parties du gène de structure de la Pristinamycine I synthase IV (snbE) de S. pristinaespiralis peut être obtenue.

Les régions de 291 et 485 pb séquencées font parties du fragment SphI de 6.6 kb qui a été cloné dans le plasmide pUC19 pour donner le plasmide pVRC1104 décrit dans l'exemple 6.6 (figure 19). Des sous-fragments de l'insert SphI de 6.6 kb ont été obtenus par coupure enzymatique puis sous-clonés dans des phages M13mp18 ou M13mp19 dans les deux orientations. Les sites de sous-clonage sont XhoI, PstI et SphI et sont représentés sur la figure 19. Le fragment XhoI-SphI de 485 pb a été entierement séquencé, et 291 pb ont été séquencées à partir du fragment PstI de 1500 pb : ces séquences sont présentées sur les SEQ ID n° 15 et 16.

### 7.7. Séquence d'une région de 645 pb dans un fragment XhoI-XhoI de 3.4 kb isolé de pVRC900.

Cet exemple illustre comment la séquence nucléotidique d'un fragment situé en amont de celui qui contient le gène snbA de S. pristinaespiralis peut être obtenue.

Pour réaliser cette séquence, un fragment XhoI-XhoI de 3,4 kb a été préalablement sous-cloné dans le vecteur pUC18, à partir du vecteur pVRC900 décrit en 6.7. Les différentes étapes de clonage ont été réalisées comme décrit en 6.1.: le plasmide pVRC900 a été digéré par l'enzyme de restriction XhoI et les fragments ainsi obtenus ont été séparés sur gel d'agarose 0.8 %. Le Fragment XhoI-XhoI de 3,4 kb a été purifié par électroélution et a été ligué avec du pUC18 coupé par l'enzyme de restriction SalI. Aprés transformation dans TG1, un clone portant le fragment XhoI-XhoI de 3,4 kb a été isolé. Le plasmide recombinant a été appelé pVRC903. Sa carte de restriction est présentée figure 23.

La séquence de 645 pb a été ensuite realisée par sous-clonage des fragments PvuII-EcoRI de 1,4 kb et PvuII-EcoRI de 0.9 kb (figure 23) à partir du pVRC903 décrit ci-dessus, dans les vecteurs M13mp18 et M13mp19. Pour réaliser ces clonages, les vecteurs M13mp18 et M13mp19 ont été digérés en premier lieu par l'enzyme de restriction BamHI; les extrémités cohésives ainsi libérées ont été remplies par le grand fragment de la DNA polymérase I (Klenow : New england Biolabs), selon la technique décrite par Maniatis et al. (1989), de manière à générer des extrémités franches compatibles avec les extrémités libérées par la digestion PvuII; les vecteurs ont été ensuite digérés par l'enzyme de restriction EcoRI. Le site PvuII a été traversé par sous-clonage d'un fragment PstI-PstI de 2,2 kb, isolé du pVRC903, chevauchant ce site. La séquence de 645 pb ainsi obtenue est représentée sur la SEQ ID n° 9.

### 7.8. Séquence d'une région de 1050 pb dans un fragment PstI-PstI de 4.1 kb isolé de pVRC900.

Cet exemple illustre comment la séquence nucléotidique d'un fragment situé en amont de celui qui contient le gène snbA de S. pristinaespiralis peut être obtenue.

Pour réaliser cette séquence, un fragment PstI-PstI de 4,1 kb a été préalablement sous-cloné dans le vecteur pUC19, à partir du vecteur pVRC900 décrit en 6-7. Les différentes étapes de clonage ont été réalisées comme décrit en 6-1. Le plasmide pVRC900 a été digéré par l'enzyme de restriction PstI et les fragments ainsi obtenus ont été séparés sur gel d'agarose 0.8 %. Le Fragment PstI-PstI de 4,1 kb a été purifié par électroélution et a été ligué avec du pUC19 coupé par l'enzyme de restriction PstI. Après transformation dans TG1, un clone portant le fragment PstI-PstI de 4,1 kb a été isolé. Le plasmide recombinant a été appelé pVRC409. Sa carte de restriction est présentée figure 24.

Cette séquence a été ensuite réalisée par sous-clonage des fragments XhoI-XhoI de 0.7 kb et XhoI-StuI de 1 kb (figure 24) à partir du pVRC409 décrit ci-dessus, dans les vecteurs M13mp18 et M13mp19. Le site XhoI interne à la séquence a été traversé par séquençage sur double brin à partir du plasmide pVRC409. La séquence de 1050 pb ainsi obtenue est représentée sur la SEQ ID n° 10.

### 7.9. Séquence d'une région de 640 pb dans le fragment BamHI-SstI de 1.5 kb.

Cet exemple illustre comment la séquence nucléotidique d'un fragment adjacent à celui qui contient les gènes snaA et snaB de S. pristinaespiralis codant pour les deux sous-unités de la PIIA synthase peut être obtenue.

Cette séquence a été realisée par sous-clonage du fragment BamHI-SstI de 1.5 kb (figure 21) à partir du pVRC1000 (Exemple 6.8) dans les vecteurs M13mp18 et M13mp19 (cf exemple 7.1). La séquence de 640 pb obtenue est représentée sur la SEQ ID n° 8.

### 7.10. Séquencage de la région XhoI-KpnI de 694 bp présente dans le fragment BamHI-BamHI de 4 kb.

Cet exemple illustre comment la séquence nucléotidique d'un fragment contenant le gène snaC de S. pristinaespiralis peut être obtenue.

La région de 694 pb séquencée fait partie du fragment BamHI-BamHI de 4 kb qui a été cloné dans le plasmide pUC19 pour donner le plasmide pVRC509 décrit dans l'exemple 6.9. Un fragment XhoI-KpnI de 694 pb, obtenu par double digestion du plasmide pVRC509 avec les enzymes de restriction XhoI et KpnI et hybridant avec les 3 sondes oligonucléotidiques décrites en 5.6., a été cloné dans les phages M13mp18 et M13mp19. Les sites de sous-clonage XhoI et KpnI sont représentés sur la figure 22.

La séquence du fragment de 694 pb ainsi obtenue est présentée SEQ ID n° 7.

### EXEMPLE 8: Analyse des séquences nucléotidiques par détermination des phases ouvertes.

Cet exemple illustre comment il est possible de déterminer les phases ouvertes de lecture présentes dans les séquences nucléotidiques définies à l'exemple 7, et d'identifier des gènes impliqués dans la biosynthèse des Pristinamycines I et des Pristinamycines II de S. pristinaespiralis SP92 ainsi que les polypeptides codés par ces gènes.

### 8.1. Fragment BamHI-XhoI de 5 kb (pXL2045).

Cet exemple illustre comment il est possible de déterminer les phases ouvertes de lecture présentes au sein du fragment BamHI-XhoI de 5 kb précédemment isolé et séquencé comme il est décrit dans les exemples 6 et 7.

Nous avons recherché la présence de phases ouvertes de lecture au sein du fragment BamHI-XhoI de 5 kb en utilisant le fait que l'ADN des Streptomyces présente un haut pourcentage en bases G et C ainsi qu'un fort biais dans l'usage des codons qui composent les phases codantes (Bibb et al. 1984). La méthode de Staden et McLachlan (1982) permet de calculer la probabilité des phases codantes en fonction de l'usage des codons de gènes de Streptomyces déjà séquencés et rassemblés dans un fichier contenant 19673 codons obtenu à partir du serveur informatique BISANCE (Dessen et al. 1990).

Cette méthode a permis de caractériser au sein du fragment BamHI-XhoI de 5 kb, quatre phases ouvertes de lecture fortement probables qui sont représentées dans le tableau suivant. Elles sont nommées phases 1 à 4 d'après leur position à partir du site BamHI. Pour chacune, on a indiqué leur longueur en bases, leur position au sein du fragment (le site BamHI étant situé à la position 1) ainsi que le poids moléculaire en kDa de la protéine correspondante. Les phases 1, 3 et 4 sont codées par le même brin et la phase 2 par le brin complémentaire (figure 16).

| Numéro de la phase et nom du gène | Position | nombre de nucléotides | nombre aminés | PM en kDa de PM en kDa de codée |
|---|---|---|---|---|
| 1 (snaA) | 48-1313 | 1266 | 422 | 46.5 |
| 2 | 2530-1328 | 1203 | 401 | - |
| 3 (snaB) | (inv) 2692-3522 | 831 | 277 | 29 |
| 4 (samS) | 3558-4763 | 1206 | 402 | 43 |

- Les phases 1 et 3 correspondent respectivement aux protéines SnaA (SEQ ID n° 2) et SnaB (SEQ ID n° 3) précédemment isolées comme il est décrit dans l'exemple 5 et dont le clonage des gènes est détaillé dans l'exemple 6. En effet les séquences NH₂-terminales des produits des ORFs 1 et 3 sont identiques aux séquences NH₂-terminales trouvées pour les protéines SnaA et SnaB respectivement, à l'exemple 5.1.2, sauf pour la méthionine amino-terminale qui a été excisée. Par ailleurs, les masses moléculaires calculées d'après les séquences sont comparables aux masses moléculaires apparentes des protéines SnaA et SnaB, estimées respectivement en PAGE-SDS comme il est décrit dans l'exemple 5.
- La comparaison du produit de la phase ouverte n° 4 avec les séquences protéiques contenues dans la banque NBRF fait apparaitre une homologie avec différentes S-adenosylmethionine (ou SAM) synthases, notamment de E.coli (Markham et al., 1984), de rat (Horikawa et al., 1989) et de S.cerevisiae (Thomas et al., 1988). Les pourcentages d'homologie calculés sur la totalité de la séquence à l'aide de l'algorithme de Kanehisa (1984) varient de 51,8 à 55.4 %.
   Ces comparaisons de séquences permettent donc de mettre en évidence que le produit de la phase ouverte n° 4 est une SAM synthase, impliquée dans la biosynthèse des Pristinamycines I ou II. Ce gène a été désigné samS (SEQ ID n° 4).
   La mise en évidence de l'implication du gène samS dans la biosynthèse des Pristinamycines est confirmée par la construction du mutant SP92 disrupté dans ce gène, comme décrit dans l'exemple 9.2.
- La comparaison de la séquence du produit de la phase ouverte n° 2 avec les séquences protéiques contenues dans la banque Genpro, fait apparaitre qu'une partie interne de cette protéine est homologue à 36 % avec une partie interne de la première phase ouverte de la séquence d'insertion (IS891) d'Anabaena (Bancroft et Wolk, 1989). Ce résultat suggère que la phase ouverte n° 2, désignée ORF 401, appartient à une séquence d'insertion, et donc qu'il existe une séquence d'insertion située entre les gènes snaA et snaB.

### 8. 2. Fragment Sau3A-Sau3A de 1870 pb (pVRC402) .

Cet exemple illustre comment il est possible de déterminer les phases ouvertes de lecture présentes au sein du fragment Sau3A-Sau3A de 1870 pb précédemment isolé et séquencé comme il est décrit dans les exemples 6 et 7.

La recherche de phases ouvertes pour le fragment Sau3A-Sau3A a été effectuée comme précédemment. Une seule phase ouverte de lecture complète a pu ainsi être mise en évidence. Ses caractéristiques sont les suivantes: cette phase s'étend de la position 109 à la position 1858 du fragment Sau3A-Sau3A, ce qui correspond à une phase de 1749 bases codant pour une protéine de 582 acides aminés ayant une masse moléculaire de 61400 Da. Cette protéine correspond à la protéine SnbA précédemment purifiée comme il est décrit dans l'exemple 5 et dont le clonage du gène est détaillé dans l'exemple 6. En effet la séquence NH₂-terminale du produit de l'ORF présente sur le fragment Sau3A.Sau3A est identique a la séquence NH₂-terminale trouvée pour la protéine SnbA, à l'exemple 5.2. La masse moléculaire de 61400 Da calculée d'après la séquence est comparable à la masse moléculaire apparente de la protéine SnbA, estimée à 67000 Da en PAGE-SDS et à 60000 Da par perméation sur gel comme il est décrit dans l'exemple 5.2.1.B.

Le gène snbA code donc pour l'enzyme qui catalyse la formation de l'acyladenylate 3-hydroxypicolinyl-AMP à partir d'une molécule d'acide 3-hydroxypicolinique et d'une molécule d'ATP: l'acide 3-hydroxypicolinique-AMP ligase (SEQ ID n° 5).

### 8.3. Fragment de 1830 pb (pVRC501).

Cet exemple illustre comment il est possible de déterminer les phases ouvertes de lecture présentes au sein du fragment de 1830 pb séquencé à partir du fragment BamHI-EcoRI de 3,1 kb précédemment isolé.

La recherche de phases ouvertes pour le fragment de 1830 pb a été effectuée comme précédemment. Une seule phase ouverte de lecture complète a pu ainsi être mise en évidence. Ses caractéristiques sont les suivantes: le démarrage probable de cette phase se situe à la position 103 et la fin à la position 1686 de la région de 1830 pb séquencée à partir du fragment BmHI-EcoRI, ce qui correspond à une protéine de 528 acides aminés ayant un poids moléculaire approximatif de 54000.

La comparaison de la séquence de cette protéine avec les séquences contenues dans la banque Genepro fait apparaitre qu'elle est homologue à des protéines ayant une fonction de transport pour différents métabolites, notamment pour la tétracycline chez différents microorganismes (Khan et Novick, 1983; Hoshino et al, 1985), l'actinorhodine (Fernandez-Moreno et al. 1991) et la méthylénomycine (Neal et Chater, 1987) chez S. coelicolor.

Ces données indiquent que le produit de la phase ouverte contenue dans le fragment BamHI-EcoRI de 3,1 kb est une protéine de transport permettant d'exporter les Pristinamycines I (et éventuellement les Pristinamycines II) à l'extérieur de la cellule. Cette protéine a été désignée SnbR et le gène correspondant snbR (SEQ ID n° 6).

L'analyse du profil d'hydrophobicité de la protéine SnbR par la méthode de Kyte et Doolittle (1982) corrobore sa localisation membranaire et donc sa fonction de transport.

### 8.4. Fragment de 1050 pb (pVRC409).

Cet exemple illustre comment il est possible de déterminer les phases ouvertes de lecture présentes au sein du fragment de 1050 pb précédemment séquence à partir du pVRC409, comme il est décrit dans l'exemple 7. 8.

La recherche de phases ouvertes pour le fragment de 1050 pb a été effectuée comme précédemment. Une seule phase ouverte de lecture complète a pu ainsi être mise en évidence. Ses caractéristiques sont les suivantes: cette phase s'étend de la position 84 à la position 962 de la portion séquencée, ce qui correspond à une phase de 878 bases codant pour une protéine de 292 acides aminés ayant une masse moléculaire de 32000 Da. Cette protéine a été appelée protéine PapM. Elle a été par ailleurs purifiée à partir de la souche S. pristinaespiralis SP92 comme il est décrit dans l'exemple 5. La masse moléculaire de 32000 Da calculée d'après la séquence est identique à la masse moléculaire apparente de 32000 Da estimée sur PAGE-SDS comme il est décrit dans l'exemple 5. Par ailleurs la séquence NH₂-terminale de cette protéine, réalisée comme décrit dans l'exemple 5 correspond bien a la séquence NH₂-terminale de la protéine PapM identifiée par l'analyse des phase ouvertes de la séquence de 1050 pb (SEQ ID n° 10).

### 8.5. Fragment de 227 pb et de 247pb (pVRC1105).

Cet exemple illustre comment il est possible de déterminer les phases ouvertes de lecture présentes au sein des fragments de 227pb et 247 pb séquencés à partir de pVRC1105, comme il est décrit dans les exemples 6 et 7.

La recherche de phase ouverte pour ces deux fragments a été effectuée comme précédemment. Une phase de lecture non complète a pu être mise en évidence dans les deux cas sur toute la longueur du fragment.

La séquence obtenue à partir de la phase ouverte identifiée sur le fragment de 247 pb isolé du fragment XhoI de 900 pb contient une des séquences internes de la protéine SnbC purifiée comme décrit dans l'exemple 5.

La comparaison du produit des phases ouvertes identifiées sur les fragments de 227 pb et 247 pb isolés de pVRC1105 avec les séquences de la banque Genpro fait apparaître qu'elles sont homologues à des peptides synthases. Celle déduite du fragment de 227 pb présente 24.5 % d'homologie avec l'α-aminoadipyl-cysteinylvaline synthase d'Acremonium chrysogenum (Gutierrez et al. 1991). Celle déduite du fragment de 247 pb présente 34.9 % d'homologie avec la gramicidine S synthase II de Bacillus brevis (Turgay et al. 1992) et 28 % avec l'α-aminoadipyl-cysteinylvaline synthase d'Acremonium chrysogenum (Gutierrez et al. 1991).

Ceci confirme que le cosmide pIBV3 isolé dans l'exemple 5.1 contient bien une partie du gène de structure de la Pristinamycine I synthase II décrite dans l'exemple 5.3, désignée SnbC (SEQ ID n° 11 et 12).

### 8.6. Fragment de 192 pb et de 474 pb (pVRC1106).

Cet exemple illustre comment il est possible de déterminer les phases ouvertes de lecture présentes au sein des fragments de 192 pb et 474 pb séquencés à partir de pVRC1106, comme il est décrit dans les exemples 6 et 7.

La recherche de phase ouverte pour ces deux fragments a été effectuée comme précédemment. Une phase de lecture non complète a pu aussi être mise en evidence sur le fragment de 192 pb isolé de pVRC1106. Ses caractéristiques sont les suivantes: cette phase démarre à la position 3 de la portion séquencée en direction du site BglII. Aucun codon stop n'a été identifié ce qui indique que cette phase ouverte n'est pas terminée.

La séquence obtenue à partir de la phase ouverte identifiée sur le fragment BglII-SphI de 192 pb contient la séquence interne de la protéine SnbD purifiée comme décrit dans l'exemple 5 qui s'avère en fait être la séquence NH₂-terminale de la protéine.

Une phase de lecture non complète a pu être mise en évidence sur toute la longueur du fragment XhoI- PstI de 474 pb.

La comparaison du produit de la phase ouverte identifiée sur le fragment de 474 pb isolé de pVRC1106 avec les séquences de la banque Genpro fait apparaitre que ce fragment de protéine présente de 30 à 40 % d'homologie avec des peptides synthases, par exemple 39.4 % avec la gramicidine S synthase II de Bacillus brevis (Turgay et al. 1992) et et 34 % avec l'α-aminoadipyl-cysteinyl-valine synthase d'Acremonium chrysogenum (Gutierrez et al. 1991).

Ceci confirme que le cosmide pIBV3 isolé dans l'exemple 5.1 contient bien une partie du gène de structure de la Pristinamycine I Synthase III décrite dans l'exemple 5.4, désignée SnbD (SEQ ID n° 13 et 14).

### 8.7. Fragment de 291 pb et de 485 pb (pVRC1104).

Cet exemple illustre comment il est possible de déterminer les phases ouvertes de lecture présentes au sein des fragments de 291 pb et 485 pb séquencés à partir de pVRC1104, comme il est décrit dans les exemples 6 et 7.

La recherche de phase ouverte pour ces deux fragments a été effectuée comme précédemment. Une phase de lecture non complète a pu être mise en évidence dans les deux cas sur toute la longueur du fragment.

La séquence obtenue à partir de la phase ouverte identifiée sur le fragment de 291 isolé à partir du fragment PstI de 1450 pb contient la séquence interne de la protéine SnbE purifiée comme décrit dans l'exemple 5.

La comparaison du produit de la phase ouverte identifiée sur le fragment XhoI-SphI de 485 pb isolé de pVRC1104 avec les séquences de la banque Genpro fait apparaître qu'elle est homologue à des peptides synthases, par exemple 34.7 % avec la gramicidine S synthase II de Bacillus brevis (Turgay et al. 1992) et et 36.2 % avec l'α-aminoadipyl-cysteinyl-valine synthase d'Acremonium chrysogenum (Gutierrez et al. 1991).

Ceci confirme que le cosmide pIBV3 isolé dans l'exemple 5.1 contient bien une partie du gène de structure de la Pristinamycine 1 synthase IV décrite dans l'exemple 5.5, désignée SnbE (SEQ ID 15 et 16).

### 8.8. Fragment de 645 pb (pVRC903).

Cet exemple illustre comment il est possible de déterminer les phases ouvertes de lecture présentes au sein du fragment de 645 pb précédemment séquencé à partir du plasmide pVRC903, comme il est décrit dans l'exemple 6.7.

La recherche de phases ouvertes pour le fragment de 645 pb a été effectuée comme précédemment. Une phase ouverte de lecture non complète a pu ainsi être mise en évidence. Ses caractéristiques sont les suivantes: cette phase présente deux possibilités de démarrage de traduction, un GTG à la position 61, un GTG à la position 70 de la portion séquencée (l'ATG situé en position 124 n'a pas été pris en compte en raison des homologies de séquences décrites plus loin). L'analyse des probabilités de présence des régions Shine-Dalgarno ne permet pas de distinguer lequel de ces codons correspond au démarrage. Aucun codon stop n'a été identifié, ce qui indique que cette phase ouverte n'est pas terminée. Le gène dont une partie a été identifiée a été appelé papA et la protéine correspondante a été appelée protéine PapA (SEQ ID n° 9).

La comparaison du produit de la phase ouverte identifiée dans le fragment XhoI-XhoI de 3,4 kb isolé de pVRC900, avec les séquences contenues dans la banque Genpro fait apparaitre qu'elle est homologue aux composants II des protéines de type p-aminobenzoate synthase et anthranilate synthase, impliquées respectivement dans la synthèse de l'acide p-aminobenzoïque (précurseur de l'acide folique) et dans la synthèse de l'acide anthranilique (précurseur du tryptophane) de différents microorganismes. Elle présente notamment une homologie de 48 % avec la protéine TrpG de Azospirillum (Zimmer et al 1991) et une homologie de 47 % avec la protéine PabA de Klebsiella pneumoniae (Kaplan et al 1985). Les protéines TrpG et PabA portent l'activité glutaminase impliquée dans la transamination de l'acide chorismique. Les homologies mises en évidence tendent à montrer que la protéine PapA serait elle aussi impliquée dans l'activité de transamination de l'acide chorismique. L'acide chorismique est proposé comme étant un précurseur de la p-diméthylamino phénylalanine, composant des Pristinamycines I, par analogie à la synthèse du chloramphénicol, antibiotique produit par des Streptomyces (Sharka et al 1970).

Le rôle de la protéine PapA est démontré ultérieurement (exemple 9.3.) par l'analyse de mutants de la souche SP92, dans le gène papA.

### 8.9. Fragment BamHI-SstI de 1.5 kb (pVRC1000).

Cet exemple illustre comment il est possible de déterminer les phases ouvertes de lecture présentes au sein du fragment BamHI-SstI de 1,5 kb précédemment isolé et séquence comme il est décrit dans les exemples 6.8 et 7.9.

La recherche de phases ouvertes pour la région séquencée de 640 pb présente dans le fragment BamHI-Sst1 de 1,5 kb a été effectuée comme il est décrit dans l'exemple 8.1. Une seule phase ouverte de lecture complète a pu ainsi être mise en évidence. Aucun démarrage et aucune fin de traduction n'ont pu être mis évidence, ce qui indique que la région de 640 pb séquencée est probablement interne à une phase de lecture beaucoup plus grande, désignée snaD (SEQ ID n° 8).

La comparaison de la séquence de la protéine codée par la région de 640 pb avec les séquences protéiques contenues dans les banques Genpro et NBRF fait apparaître que cette protéine est homologue à 20-25 % à une partie interne des peptide-synthases telles que la gramicidine synthase I de B. brevis (Hori et al, 1989), de la tyrocidine synthase I de B. brevis (Weckermann et al 1988), et l'ACV synthase d'Acremonium chrysogenum (Gutietrez et al, 1991).

Ces données indiquent que la protéine codée en partie par la région de 640 pb est probablement une peptide synthase impliquée dans la biosynthèse de la partie peptidique des Pristinamycines II: en effet toutes les peptides synthases impliquées dans la biosynthèse des Pristinamycines I ont déjà été identifiées dans d'autres régions du chromosome de S. pristinaespiralis comme il est décrit dans les exemples 5.2., 5.3., 5.4. et 5.5.

### 8.10. Fragment de 694 pb (pVRC509).

Cet exemple illustre comment il est possible de déterminer les phases ouvertes de lecture présentes au sein du fragment de 694 pb précédemment séquence à partir du pVRC509, comme il est décrit dans les exemples 6 et 7.

La recherche de phases ouvertes pour le fragment de 694 pb a été effectuée comme précédemment. Une phase ouverte de lecture non complète a pu ainsi être mise en évidence. Ses caractéristiques sont les suivantes : cette phase démarre à la position 210, de la portion séquencée. Aucun codon stop n'a été identifié, ce qui indique que cette phase ouverte n'est pas terminée. La masse moléculaire de la protéine correspondante ne peut donc pas être calculée et comparée à la masse moléculaire apparente de 28000 Da de la FMN réductase, estimée sur PAGE-SDS comme il est décrit dans l'exemple 5.6. Par contre la séquence NH₂-terminale de la protéine ainsi identifiée par l'analyse des phase ouvertes de la séquence de 694 pb, est identique a la séquence NH₂-terminale de la protéine SnaC purifiée comme décrit dans l'exemple 5. De même, on retrouve dans la protéine déduite de la portion séquencée, les deux séquences protéiques internes de la FMN réductase décrite en 5.6. Ceci confirme que le gène isolé à partir du cosmide pIBV4 correspond bien à la protéine FMN réductase décrite dans l'exemple 5.6, désignée SnaC (SEQ ID n°7).

L'étude des fragments d'ADN de la souche S. pristinaespiralis SP92, portés par les cosmides pIBV1 à pIBV4, a mis en évidence la présence de plusieurs gènes impliqués dans la biosynthèse des Pristinamycines II et des Pristinamycines I. Les gènes snaA, snaB et samS codent pour des enzymes intervenant dans la biosynthèse des Pristinamycines II, la Pristinamycine IIA synthase et une probable SAM synthase et sont groupés physiquement sur un large fragment d'ADN cloné dans le cosmide pIBV1. De même les gènes snbA, snbR, papA et papM -qui codent pour des protéines intervenant dans la biosynthèse des Pristinamycines I, l'acide 3-hydroxypicolinique-AMP ligase (SnbA), la protéine SnbR probablement responsable du transport des Pristinamycines I, la protéine PapA impliquée dans la biosynthèse, à partir de l'acide chorismique, de la p-aminophénylalanine, et la p-aminophénylalanine (phényl-N)-méthyltransférase (PapM)- sont groupés sur un large fragment d'ADN cloné dans le cosmide pIBV2. De même, les gènes snaA et snaD d'une part - qui codent pour des protéines intervenant dans la biosynthèse des Pristinamycines II, la protéine SnaD étant probablement une peptide synthase- et les gènes snbC, snbD et snbE, d'autre part -qui codent pour les 3 peptide synthases SnbC, SnbD et SnbE, impliquées dans la formation de la chaîne peptidique de la Pristinamycine I à partir de ses 6 acides aminés séparés- sont groupés sur un large fragment d'ADN cloné dans le cosmide pIBV3. Ces résultats confirment l'hypothèse du groupement des gènes de biosynthèse des Pristinamycines II et également des gènes de biosynthèse des Pristinamycines I et offrent la possibilité de cloner les autres gènes impliqués dans ces biosynthèses, par marche sur le chromosome, en amont et en aval des régions étudiées.

De plus, il est possible par hybridation de l'ADN total des différentes souches productrices de Streptogramines (cf tableau 1) avec les gènes snaA, snaB, snaC, snaD, samS, snbA, snbR, snbC, snbD, snbE, papA et papM ou avec les gènes identifiés par marche sur le chromosome, ou avec des fragments de ceux-ci, d'isoler les gènes correspondants aux mêmes fonctions dans les autres microorganismes producteurs de Streptogramines. Ceci permet par la même démarche que celle envisagée pour les Pristinamycines, d'isoler l'ensemble des gènes impliqués dans la biosynthèse des différentes Streptogramines.

### EXEMPLE 9 : Etude génétique de fragments d'ADN par disruption de gènes.

Cet exemple illustre comment il est possible de mettre en évidence l'implication de gènes dans la biosynthèse des Streptogramines en construisant des souches dérivées d'une souche productrice, mutées au niveau de ces gènes par disruption et en analysant le phénotype de tels mutants. Cet exemple montre de plus comment obtenir des souches ne produisant plus que l'un ou l'autre des composants A et B des Streptogramines, ou produisant des composants A ou B avec des ratios différents de ceux que l'on observe avec la souche SP92.

### 9.1. Construction d'un mutant de S. pristinaespiralis SP92 disrupté dans le gène snaA.

Cet exemple illustre comment il est possible par disruption du gène snaA, de construire une souche de S. pristinaepiralis SP92 qui ne produit plus de Pristinamycine IIA et qui produit par contre de la Pristinamycine IIB.

Ce mutant a été construit dans le but de confirmer la fonctionnalité de la protéine SnaA et de fournir un intermédiaire de production de la Pristinamycine II, pouvant par la suite être modifié.

Sa construction a été réalisée à l'aide d'un vecteur suicide, capable de se répliquer chez E. coli seulement, mais portant un marqueur de résistance s'exprimant chez Streptomyces. Ce vecteur, pDH5, a été développé par Hillemann et al. (1991).

### 9.1.1. Construction du plasmide pVRC505.

Cet exemple illustre comment il est possible de construire un plasmide ne se répliquant pas chez S. pristinaespiralis SP92 et qui peut être utilisé pour disrupter par simple recombinaison homologue le gène snaA.

Le plasmide pVRC505 a été construit pour réaliser le mutant chromosomique SP92 disrupté dans le gène snaA à partir du plasmide pXL2045 décrit dans l'exemple 6.3.

Le fragment BamHI de 6 kb, cloné dans le pXL2045 (figure 16), a été coupé par les enzymes de restriction EcoRI et PstI. Après séparation des fragments ainsi générés par électrophorèse sur gel d'agarose à 1 %, un fragment de 0,7 kb, contenant l'extrémité 5' du gène snaA, a été isolé et purifié par Geneclean (Bio101, La Jolla, Californie).

100 ng de vecteur pDH5 linéarisés par une double digestion EcoRI, PstI, ont été ligués avec 100 ng du fragment de 0,7 kb, comme décrit dans l'exemple 3.3. Un clone portant le fragment souhaité a été isolé après transformation de la souche TG1. Le plasmide recombinant a été nommé pVRC505. Le plasmide pVRC505 a été préparé comme décrit dans l'exemple 2.1. Sa carte de restriction est présentée dans la figure 25.

### 9.1.2. Isolement du mutant SP92 disrupté dans le gène snaA par recombinaison homologue.

Cet exemple illustre comment le mutant de S. pristinaespiralis SP92, disrupté dans le gène snaA, a été construit.

Ce mutant a été isolé par transformation de la souche SP92 avec le plasmide suicide pVRC505.

La préparation des protoplastes et leur transformation ont été réalisées comme décrit dans D. Hopwood et al. (1985).

La souche SP92 a été cultivée en milieu YEME, 34 % de sucrose, 5 mM MgCl₂, glycine 0.25 % pendant 40 heures à 30°C. Le mycélium a été protoplastisé en présence de lysozyme et 5X1 µg de pVRC505 ont été utilisés pour la transformation (par la méthode utilisant le PEG) des protoplastes. Après une nuit de régénération des protoplastes sur milieu R2YE (D. Hopwood et al. 1985), les recombinants ont été sélectionnés par étalement de 3 ml de milieu SNA (D. Hopwood et al. 1985) contenant 2.6 µg/ml de thiostrepton.

Sur les 5 transformations réalisées, 3 clones résistants au thiostrepton ont été isolés. Ceci donne un taux de recombinants inférieur à 1 par µg d'ADN. Ces recombinants résultent de l'intégration par simple recombinaison homologue entre le gène snaA porté par le chromosome de la souche SP92 et le fragment de 0,7 kb du plasmide suicide pVRC505. La faible taille du fragment inséré dans pVRC505, 0,7 kb, explique le faible taux de recombinaison.

Les spores des recombinants ont été isolées par étalement et croissance sur milieu R2YE supplémenté par 400 µg/ml de thiostrepton, et réétalées sur le même milieu pour obtenir des colonies isolées.

Afin de vérifier la position de l'intégration du plasmide pVRC505, différents Southerns de l'ADN total de plusieurs clones recombinants digéré par les enzymes de restriction appropriées, ont été réalisés et hybridés avec le vecteur pDH5 et le fragment de 0,7 kb, utilisés successivement comme sondes après marquage par random priming (Random Primed DNA labeling kit, Boehringer Mannheim, France) avec du [α-³²P]dCTP, comme décrit dans Maniatis et al. (1989). Les résultats d'hybridation montrent l'apparition, dans le génome des clones recombinants, d'une bande EcoRI-PstI supplémentaire, de la taille du vecteur pDH5 et hybridant avec celui-ci ainsi que d'une bande supplémentaire EcoRI-EcoRI, hybridant à la fois avec les 2 sondes. Un de ces mutants a été nommé SP92::pVRC505. Ce mutant correspond bien à l'intégration par simple recombinaison homologue du plasmide pVRC505 dans le gène snaA.

### 9.1.3. Production de Pristinamycines par le mutant SP92::pVRC505.

Cet exemple illustre comment il est déterminé que le mutant de S. pristinaespiralis SP92 disrupté dans le gène snaA par l'intégration du plasmide pVRC505, ne produit plus de Pristinamycine IIA, tout en continuant à produire de la Pristinamycine IIB.

Le mutant SP92::pVRC505, ainsi que la souche SP92, en tant que souche témoin, ont été cultivés en milieu de production liquide. La fermentation a été réalisée comme suit : 0.5 ml d'une suspension de spores des souches citées sont ajoutés en conditions stériles à 40 ml de milieu inoculum dans un erlen de 300 ml. Le milieu inoculum est constitué par 10 g/l de Corn Steep, 15 g/l de saccharose, 10 g/l de (NH₄)₂SO₄, 1 g/l de K₂HPO₄, 3 g/l de NaCl, 0.2 g/l de MgSO₄-7H₂O et 1.25 g/l de CaCO₃. Le pH est ajusté à 6.9 par de la soude avant l'introduction du carbonate de calcium. Les erlens sont agités pendant 44 heures à 27°C sur un agitateur rotatif à la vitesse de 325 rpm. 2.5 ml de la culture précédente âgée de 44 heures sont ajoutés stérilement à 30 ml de milieu de production dans un erlen de 300 ml. Le milieu de production est constitué par 25 g/l de farine de soja, 7.5 g/l d'amidon, 22.5 g/l de glucose, 3.5 g/l de levure fourragère, 0.5 g/l de sulfate de zinc et 6 g/l de carbonate de calcium. Le pH est ajusté à 6,0 par de l'acide chlorhydrique avant l'introduction du carbonate de calcium. Les erlens sont agités pendant 24, 28 et 32 heures à 27°C. A chaque temps, 10 g de moût sont pesés dans un erlen lisse , auxquels sont ajoutés 20 ml de phase mobile composée de 62.5 % d'acétonitrile et 37.5 % d'une solution de 0.1 M de KH₂PO₄ (ajusté à pH 3,0 par H₃PO₄) et permettant l'extraction des Pristinamycines. Après agitation sur un agitateur (325 rpm) pendant 20 min à température ambiante, le tout est filtré sur filtre de papier et les Pristinamycines sont dosées par CLHP comme décrit à l'exemple 5.1.1.A.

Les résultats ont montré que, dans les conditions de fermentation réalisées, le mutant SP92::pVRC505 a produit une quantité de Pristinamycine I équivalente à celle du témoin SP92, ceci pour les 3 temps testés. En revanche, alors que le témoin a produit à 24, 28 et 32 heures de fermentation, environ 70 % de Pristinamycine IIA et 30 % de Pristinamycine IIB, le mutant SP92 :: pVRC505 a produit pour ces mêmes temps, 100 % de Pristinamycine IIB, en quantités équivalentes à la somme des Pcistinamycine IIA + Pristinamycine IIB produites par la souche SP92. Le mutant est donc bien bloqué dans une étape de biosynthèse de la Pristinamycine II qui correspond à l'oxydation de la liaison 2-3 de la D-proline de l'intermédiaire Pristinamycine IIB. Ce mutant accumule donc la Pristinamycine IIB. Ceci montre bien l'implication fonctionnelle du gène snaA dans la conversion Pristinamycine IIB en Pristinamycine IIA.

Cet exemple montre qu'il est possible, à partir des gènes de biosynthèse clonés, de construire des souches mutées dans les étapes de biosynthèse des Pristinamycines. Ceci a été montré pour les Pristinamycines II mais les mêmes résultats peuvent être obtenus pour les Pristinamycines I et par extension, pour les différents composants des Streptogramines. Des souches produisant différents intermédiaires peuvent être ainsi obtenues. Ces souches peuvent être utilisées pour produire des molécules nouvelles, par modification(s) chimique (s), biochimique(s), enzymatique(s), etc. des dits intermédiaires. Un blocage dans une étape précoce de la voie de biosynthèse de l'un ou l'autre des composants des Streptogramines peut également conduire à des souches mutées ne produisant plus que l'un ou l'autre des composants. 9.2. Construction d'un mutant de S. pristinaespiralis SP92 discupté dans le gène samS.

Cet exemple illlustre comment il est possible par disruption du gène samS de construire une souche de S. pristinaespiralis SP92 qui produit 35 % de moins de PIA et environ 10 fois plus de PIB (les structures chimiques sont représentées sur la figure 2) par rapport à la souche sauvage SP92. Ce mutant a été construit dans le but de confirmer la fonction de SAM synthase présumée pour la protéine codée par le gène samS et d'obtenir une souche synthétisant plus de PIB que la souche sauvage SP92.

### 9.2.1. Construction du plasmide pVRC702.

A partir du plasmide pXL2045 (décrit dans l'exemple 6.3.), on a isolé par coupure enzymatique le fragment BamH1-EcoR1 de 3,2 kb que l'on a purifié après électrophorèse sur gel d'agarose à 1 % par la méthode du kit Geneclean (voir exemple 6.8). Ce fragment porte le gène snaB ainsi que le gène samS (figure 16). Ce fragment a été ensuite cloné dans un plasmide pUC18 de la façon suivante: 50 ng de pUC 18 ont été linéarisés par la double digestion à l'aide des enzymes EcoR1 et BamH1 puis ligués en présence de T4 DNA ligase (Biolabs) avec 300 ng du fragment BamH1-EcoR1 de 3,2 kb. Après transformation de cellules compétentes de la souche E. coli TG1 avec ce mélange de ligation, on a pu isoler un clone recombinant possédant le plasmide pUC18 avec l'insert de 3,2 kb que l'on a nommé pVRC701 (figure 26).

Le plasmide pVRC702 dérive du plasmide pVRC701 par l'introduction entre les deux sites Sst1 situés au milieu du gène samS (figure 27) d'une cassette portant le gène amR codant pour la résistance à l'apramycine et à la géniticine. Pour cela, un fragment BamH1-BamH1 de 2,2 kb portant la cassette ΩamR a d'abord été isolé par digestion BamH1 du plasmide pHP45ΩamR (donné par J.L. Pernodet, Laboratoire de Génétique d'Orsay) en utilisant la même technique que précédemment. 200 ng de ce fragment ont ensuite été ligués avec 50 ng du plasmide pUC1318 (Kay et McPherson, 1987) linéarisés par l'enzyme BamH1 et ce mélange de ligation a été introduit dans des cellules compétentes E. coli TG1. A partir du clone recombinant possédant le plasmide pUC1318 contenant la cassette ΩamR, on a pu réisoler par coupure partielle à l'aide de l'enzyme Sst1, 50 ng d'un fragment Sst1-Sst1 de 2,2 kb contenant la cassette ΩamR que l'on a ligué avec 30 ng du plasmide pVRC701 coupé par Sst1 (figure 26) pour donner après transformation de cellules compétentes E. coli TG1, le plasmide PVRC 702 dont la structure est détaillée sur la figure 27.

Le plasmide pVRC702 ainsi obtenu a été préparé en larges quantités selon la méthode précédemment décrite dans l'exemple 2.1.

### 9.2.2. Construction de la souche ayant le gène chromosomique samS:: ΩamR.

Cette souche a été obtenue par transformation de protoplastes de S. pristinaespiralis avec 1 µg du plasmide suicide pVRC702 ne pouvant pas se répliquer dans une cellule de Streptomyces. Les protocoles de préparation des protoplastes et de transformation sont les mêmes que ci-dessus (exemple 9.1). Les seules modifications apportées par rapport à l'exemple 9.1 concernent l'antibiotique de sélection. Dans le cas présent, les protoplastes recombinants après une régénération de 18 heures à 30°C sur milieu R2YE sont sélectionnés en présence de 50 µg/ml final de généticine (Sigma Chemical Co.). Ainsi à chaque boîte de R2YE, on ajoute une surcouche composée de 3ml de SNA contenant 383- µg/ml de géniticine.

On a ainsi pu isoler 500 clones recombinants résistants à la généticine qui peuvent résulter soit d'une intégration dans le chromosome du plasmide pVRC702 à la suite d'une recombinaison homologue simple entre les gènes chromosomique et plasmidique samS (cas de simple crossing-over), soit d'un échange entre le gène chromosomique samS et le gène plasmidique samS::ΩamR à la suite d'un double événement de recombinaison homologue (cas de double crossing-over). Dans ces deux cas de figure, la cassette ΩamR se trouve transférée sur le chromosome de la souche et lui confère une résistance amR stable au cours des générations.

Les clones recombinants ont été isolés par étalement et croissance sur milieu R2YE contenant 50 µg/ml final de généticine puis analysés par la technique d'hybridation sur colonies. L'hybridation des clones avec une première sonde obtenue comme il est décrit dans l'exemple 9.1 à partir du fragment BamH1-EcoRI de 2,7 kb issu de pVRC702 et correspondant au pUC18 ainsi qu'avec une deuxième sonde correspondant au fragment BamHI de 2,2 kb portant la cassette ΩamR, a permis de distinguer les cas de simple crossing-over (hybridant avec les deux sondes) des cas de double crossing-over (n'hybridant qu'avec la deuxième sonde). Les 3 clones résultant d'un double crossing-over ainsi sélectionnés ont été purifiés par étalement et croissance sur milieu R2YE contenant 50 µg/ml final de généticine et des stocks de spores ont été obtenus.

Afin de vérifier la structure génomique des 3 doubles recombinants, différents Southerns de l'ADN chromosomique de ces clones digéré par les enzymes EcoRI et BamHI ont été réalisés et hybridés avec les trois sondes suivantes : la sonde correspondant à la cassette ΩamR obtenue à partir du fragment BamHI de 2,2 kb de pVRC702, la sonde correspondant à pUC18 obtenue à partir du fragment BamHI-EcoRI de 2,7 kb de pVRC701 et enfin une sonde obtenue à partir du fragment EcoRI-SstI de 1,3 kb de pVRC701 portant le gène snaB et le début de samS. Ces hybridations ont permis de vérifier que les trois clones testés résultaient bien d'un double évenement de recombinaison homologue ayant permis le remplacement du gène chromosomique intact samS par le gène muté samS interrompu par la cassette ΩamR.

Un de ces trois clones mutants a été nommé SP92 samS::ΩamR.

### 9.2.3. Production de pristinamycines par la souche mutante samS::ΩamR.

Cet exemple illustre comment il est déterminé que le mutant SP92 samS::ΩamR ayant le gène samS disrupté produit 35 % de moins de Pristinamycine IA et 10 fois plus de Pristinamycine IB que la souche sauvage SP92.

Le mutant SP92 samS::ΩamR ainsi que la souche témoin SP92 ont été cultivées en milieu de production liquide et leurs productions de Pristinamycine II et de Pristinamycine I dosées comme décrit dans l'exemple 9.1.

Les résultats ont montré que, dans les conditions de fermentation réalisées, le mutant SP92 samS::ΩamR produit une quantité de Pristinamycines II équivalente à celle du témoin SP92 pour les trois temps testés. En revanche, le mutant SP92 samS::ΩamR produit aux trois temps testés environ 35 % de moins de Pristinamycine IA et 10 fois plus de Pristinamycine IB que la souche témoin. La forme IB des Pristinamycines représente ainsi 20 % de l'ensemble des Pristinamycines du type I totales produites par le mutant SP92 samS::ΩamR alors que la souche témoin ne synthétise que de l'ordre de 1 % de PIB. La forme IB des Pristinamycines diffère de la forme IA par le fait que le cinquième résidu est la p-méthylaminophénylalanine au lieu de la p-diméthylaminophénylalanine pour la Pristinamycine IA. Le fait que le mutant SP92 samS::ΩamR produise plus de Pristinamycine IB et moins de Pristinamycine IA montre que la disruption du gène samS provoque une diminution du degré de méthylation du cinquième résidu des Pristinamycines I et donc que le gène samS est probablement impliqué dans la biosynthèse du donneur de méthyle la SAM c'est-à-dire qu'il code pour une SAM synthase.

### 9.3. Construction d'un mutant de S. pristinaespiralis SP92 disrupté dans le gène papA.

Cet exemple illustre comment il est possible par disruption du gène papA, de construire une souche de S. pristinaespiralis SP92 qui ne produit plus de PI. Ce mutant a été construit dans le but de confirmer la fonctionnalité de la proteine PapA. Sa construction a été réalisée à l'aide du vecteur suicide, pDH5, décrit dans l'exemple 9.1.

### 9.3.1. Construction du plasmide pVRC508.

Cet exemple illustre comment il est possible de construire un plasmide ne se répliquant pas chez S.pristinaespiralis SP92, qui peut être utilisé pour disrupter par simple recombinaison homologue le gène papA.

Le plasmide pVRC508 a été construit pour réaliser le mutant chromosomique SP92 disrupté dans le gène papA, à partir du plasmide pVRC903, décrit dans l'exemple 7.7.

Dans l'exemple 7.7., il a été décrit le clonage du fragment PvuII-EcoRI de 1,4 kb dans M13mp18 à partir du plasmide pVRC903, en vue du séquençage du gène papA (ce fragment correspond au fragment PvuII-XhoI de 1,4 kb présenté dans le vecteur pVRC903, figure 23).

Cette construction dans M13mp18 a été digérée par les enzymes de restriction HindIII et EcoRI. Après séparation par électrophorèse sur gel d'agarose à 0.8 % du vecteur M13mp18 et du fragment de 1,4 kb, contenant une partie du gène papA, celui ci a été isolé et purifié par Geneclean (Bio101, La Jolla, Californie). La localisation du fragment dans le gène papA est présentée figure 23.

100 ng de vecteur pDH5 linéarisés par une double digestion avec les enzymes de restriction HindIII et EcoRI, ont été ligués avec 200 ng du fragment de 1,4 kb, comme décrit dans l'exemple 3.3. Un clone portant le fragment souhaité a été isolé après transformation de la souche TG1. Le plasmide recombinant a été nommé pVRC508. Le plasmide pVRC508 a été préparé comme décrit dans l'exemple 2.1. Sa carte de restriction est présentée dans la figure 28.

### 9.3.2. Isolement du mutant SP92 disrupté dans le gène papA par recombinaison homologue.

Cet exemple illustre comment le mutant de S. pristinaespiralis SP92, disrupté dans le gène papA, a été construit. Ce mutant a été isolé par transformation de la souche SP92 avec le plasmide suicide pVRC508. La préparation des protoplastes et leur transformation ont été réalisées comme décrit dans l'exemple 9.1. Après transformation des protoplastes de la souche SP92, les recombinants ont été sélectionnés par étalement de 3 ml de milieu SNA contenant 2.6 mg/ml de thiostrepton. Sur les 5 transformations réalisées avec 5 fois 1 µg du plasmide pVRC508, une dizaine de clones résistants au thiostrepton ont été isolés. Ceci donne un taux de recombinants d'environ 2 par µg d'ADN. Ces recombinants résultent de l'intégration par simple recombinaison homologue entre le gène papA porté par le chromosome de la souche SP92 et le fragment de 1.4 kb du plasmide suicide pVRC508.

Les spores des recombinants ont été isolées par étalement et croissance sur milieu R2YE contenant 400 µg/ml de thiostrepton, et réétalées sur le même milieu pour obtenir des colonies isolées.

Afin de vérifier la position de l'intégration du plasmide pVRC508, différents Southerns de l'ADN total de plusieurs clones recombinants, purifiés comme décrit ci-dessus, ont été réalisés et hybridés avec le vecteur pDH5 et le fragment de 1,4 kb utilisés successivement comme sondes après marquage par random priming avec du [α-³²P]dCTP comme décrit dans l'exemple 9.1. Les résultats d'hybridation montrent la disparition, dans le génome des clones recombinants digérés par l'enzyme de restriction EcoRI (site encadrant le fragment de 1,4 kb) de la bande EcoRI de 6,8 kb et l'apparition de deux bandes supplémentaires par rapport à la souche témoin SP92, l'une de 2,4 kb hybridant avec le fragment de 1,4 kb et l'autre de 10,5 kb hybridant à la fois avec pDH5 et avec le fragment de 1,4 kb. La digestion des clones recombinants par l'enzyme de restriction PstI montre l'apparition, de deux bandes supplémentaires par rapport à la souche témoin SP92, l'une de 1,0 kb hybridant avec le fragment de 1,4 kb et l'autre de 5,1 kb hybridant à la fois avec pDH5 et avec le fragment de 1,4 kb. Un de ces mutants a été nommé SP92::pVRC508.

### 9.3.3. Production de Pristinamycines par le mutant SP92::pVRC508.

Cet exemple illustre comment il est déterminé que le mutant de S. pristinaespiralis SP92 disrupté dans le gène papA par l'intégration du plasmide pVRC508, ne produit plus de Pristinamycine I.

Le mutant SP92::pVRC508, ainsi que la souche SP92, en tant que souche témoin, ont été cultivés en milieu de production liquide. La fermentation, ainsi que le dosage des Pristinamycines I et II, ont été réalisés comme décrit dans l'exemple 9.1.

Les résultats ont montré que dans les conditions de fermentation réalisées, alors que la souche témoin SP92 produit une quantité standard de Pristinamycines I, aucune trace de Pristinamycines de type I n'a été détectée dans le moût de fermentation du mutant SP92::pVRC508. Par ailleurs la production de Pristinamycines II par le mutant SP92::pVRC508 est équivalente à celle du témoin SP92. Le mutant SP92::pVRC508 ne produit donc plus que des Pristinamycines II. Ces résultats montrent bien que le gène papA code pour une protéine impliquée dans la biosynthèse des Pristinamycines I.

Par ailleurs pour s'assurer que la disruption réalisée ne touche que le gène ciblé papA. le mutant SP92::pVRC508 a été fermenté en présence de p-diméthylamino phenylalanine. Le mutant SP92::pVRC508, a été fermenté comme décrit plus haut avec ajout, à 17 heures de fermentation, de 100 mg/l de p-diméthylaminophénylalanine. Dans ces conditions de complémentation le mutant SP92::pVRC508 produit une quantité de Pristinamycines 1 équivalente à celle produite par la souche SP92. La production de Pristinamycines II est équivalente dans les deux souches. Ceci nous permet de conclure que le mutant SP92::pVRC508 ne produit pas de Pristinamycines I parce qu'il est bien disrupté dans un gène intervenant dans la biosynthèse de la p-diméthylaminophenylalanine (le gène papA). La complémentation de ce mutant par la p-diméthylaminophenylalanine restaure sa capacité à produire des Pristinamycines I, prouvant que la mutation dans le gène papA n'a pas d'effet polaire sur la synthèse des autres précurseurs des Pristinamycines I ou sur la formation de la chaine peptidique à partir de ces précurseurs.

Cet exemple montre qu'il est possible, à partir de gènes de biosynthèse clonés, de construire des souches mutées dans les étapes de biosynthèse des Pristinamycines et en particulier des Pristinamycines I. Cet exemple montre également qu'il est possible par cette approche de construire des souches de S. pristinaespiralis produisant spécifiquement des Pristinamycines II et par extension des souches produisant spécifiquement des Pristinamycines I. Cette même approche pourrait également être utilisée pour les autres souches d'actinomycètes producteurs de streptogramines.

### 9.4. Construction d'un mutant de S.pristinaespiralis SP92 disrupté dans le gène snbA.

Cet exemple illustre comment il est possible par disruption du gène snbA, de construire une souche de S. pristinaespiralis SP92 qui ne produit plus de Pristinamycines I. Ce mutant a été construit dans le but de confirmer la fonctionnalité de la proteine SnbA. Sa construction a été réalisée à l'aide du vecteur suicide, pDH5, décrit dans l'exemple 9.1.

### 9.4.1. Construction du plasmide pVRC404.

Cet exemple illustre comment il est possible de construire un plasmide ne se répliquant pas chez S. pristinaespiralis SP92, qui peut être utilisé pour disrupter par simple recombinaison homologue le gène snbA.

Le plasmide pVRC404 a été construit à partir du plasmide pVRC402 décrit dans l'exemple 6.1., pour réaliser le mutant chromosomique SP92 disrupté dans le gène snbA. Le plasmide pVRC402 a été digéré par les enzymes de restriction XhoI et HindIII. Après séparation des fragments ainsi générés par électrophorèse sur gel d'agarose à 0.8 %, un fragment de 1170 pb, contenant une partie interne du gène snbA, a été isolé et purifié par Geneclean (Bio101, La Jolla, Californie). La localisation du fragment dans le gène snbA est présentée figure 15A.

100 ng de vecteur pDH5 linéarisés par une digestion SmaI, ont été ligués avec 200 ng du fragment de 1170 pb, comme décrit dans l'exemple 3.3. Un clone portant le fragment souhaité a été isolé après transformation de la souche TG1. Le plasmide recombinant a été nommé pVRC404. Le plasmide pVRC404 a été préparé comme décrit dans l'exemple 2.1. Sa carte de restriction est présentée dans la figure 29.

### 9.4.2. Isolement du mutant SP92 disrupté dans le gène snbA par recombinaison homologue.

Cet exemple illustre comment le mutant de S. pristinaespiralis SP92, disrupté dans le gène snbA, a été construit.

Ce mutant a été isolé par transformation de la souche SP92 avec le plasmide suicide pVRC404. La préparation des protoplastes et leur transformation ont été réalisées comme décrit dans l'exemple 9.1. Après transformation des protoplastes de la souche SP92, les recombinants ont été sélectionnés par étalement de 3 ml de milieu SNA contenant 2.6 mg/ml de thiostrepton. Sur les 5 transformations réalisées avec 5 fois 1 µg du plasmide pVRC404, une trentaine de clones résistants au thiostrepton ont été isolés. Ceci donne un taux de recombinants d'environ 5 par µg d'ADN. Ces recombinants résultent de l'intégration par simple recombinaison homologue entre le gène snbA porté par le chromosome de la souche SP92 et le fragment de 1170 pb du plasmide suicide pVRC404. Les spores des recombinants ont été isolées par étalement et croissance sur milieu R2YE + 400 µg/ml de thiostrepton, et réétalées sur le même milieu pour obtenir des colonies isolées. Afin de vérifier la position de l'intégration du plasmide pVRC404, différents Southerns de l'ADN total de plusieurs clones recombinants, purifiés comme décrit ci-dessus, ont été réalisés et hybridés avec le vecteur pDH5 et le fragment de 1170 kb utilisés successivement comme sondes après marquage par random priming avec du [α-³²P]dCTP comme décrit dans l'exemple 9.1. Les résultats d'hybridation montrent l'apparition, dans le génome des clones recombinants digérés par les enzymes de restriction XhoI et HindIII, d'une bande XhoI-HindIII supplémentaire de 4,7 kb (vecteur pDH5 + 1,17 kb), par rapport à la souche témoin SP92, hybridant à la fois avec pDH5 et avec le fragment de 1170 pb. La digestion des clones recombinants par l'enzyme de restriction Pf1MI (sites encadrant le fragment XhoI-HindIII de 1170 pb) montre la disparition de la bande Pf1MI-Pf1MI de 3,1 kb et l'apparition d'une bande à 8,8 kb hybridant avec les deux sondes. Ces résultats indiquent que la structure génomique des clones analysés est bien celle que l'on attend après un événement de recombinaison homologue entre pVRC404 et le gène chromosomique snbA. Un de ces mutants a été nommé SP92::pVRC404.

### 9.4.3. Production de pristinamycines par le mutant SP92::pVRC404.

Cet exemple illustre comment il est déterminé que le mutant de S. pristinaespiralis SP92 disrupté dans le gène snbA par l'intégration du plasmide pYRC404, ne produit plus de Pristinamycine I.

Le mutant SP92::pVRC404, ainsi que la souche SP92, en tant que souche témoin, ont été cultivés en milieu de production liquide. La fermentation, ainsi que le dosage des Pristinamycines I et II, ont été réalisés comme décrit dans l'exemple 9.1. Les résultats ont montré que dans les conditions de fermentation réalisées, alors que la souche témoin SP92 produit une quantité standard de Pristinamycines I, aucune trace de Pristinamycines I n'a été détectée dans le moût de fermentation du mutant SP92::pVRC404. Par ailleurs la production de Pristinamycines II par le mutant SP92::pVRC404 est équivalente à celle du témoin SP92. Le mutant SP92::pVRC404 ne produit plus que des Pristinamycines II. Ceci montre bien que le gène snbA code pour une protéine SnbA impliquée dans la biosynthèse des Pristinamycines I, comme il avait été montré lors de la purification dans l'exemple 5.2.

Cet exemple montre, comme dans l'exemple précédent, qu'il est possible, à partir de gènes de biosynthèse clonés, de construire des souches mutées dans les étapes de biosynthèse des Pristinamycines et en particulier des Pristinamycines L Cet exemple montre également qu'il est possible par cette approche de produire des souches de S. pristinaespiralis produisant spécifiquement des Pristinamycines II et par extension des souches produisant spécifiquement des Pristinamycines I, comme décrit dans l'exemple suivant: 9.5. Cette même approche pourrait également être utilisée pour les autres souches d'actinomycètes producteurs de streptogramines.

### 9.5. Construction d'un mutant de S. pristinaespiralis SP92 disrupté dans le gène snaD codant probablement pour une peptide synthase impliquée dans la biosynthèse des Pristinamycines II.

Cet exemple illustre comment il est possible par disruption du gène snaD codant probablement pour une peptide synthase impliquée dans la biosynthèse des Pristinamycines II, de construire une souche de S. pristinaespiralis SP92 qui ne produit plus de Pristinamycines II.

Ce mutant a été construit dans le but de confirmer la fonctionnalité du gène snaD et d'obtenir une souche dérivée de SP92 ne synthétisant plus que des Pristinamycines I.

Sa construction a été réalisée à l'aide du plasmide PVRC1000 décrit dans l'exemple 6.8. dérivé du vecteur suicide pDH5, capable de se répliquer chez E. coli seulement et portant un marqueur de résistance s'exprimant chez Streptomyces (cf exemple 9.1.).

### 9.5.1. Construction du plasmide pVRC1000.

Cet exemple illustre comment il est possible de construire un plasmide ne se répliquant pas chez S. pristinaespiralis SP92, qui peut être utilisé pour disrupter par simple recombinaison homologue le gène snaD. La construction du plasmide pVRC1000 portant une partie du gène snaD est décrit dans l'exemple 6.8.

### 9.5.2. Isolement du mutant SP92 disrupté dans le gène snaD par recombinaison homologue.

Cet exemple illustre comment le mutant de S. pristinaespiralis SP92, disrupté dans le gène snaD, a été construit. Ce mutant a été isolé par transformation de la souche SP92 avec le plasmide suicide pVRC1000. La préparation des protoplastes et leur transformation ont été réalisées comme décrit dans l'exemple 9.1. Sur les 5 transformations réalisées avec 1 µg de pVRC1000 environ 1500 clones résistants au thiostrepton ont été isolés. Ceci donne un taux de recombinants d'environ 375 par µg d'ADN. Ces recombinants résultent de l'intégration par simple recombinaison homologue entre le gène snaD porté par le chromosome de la souche SP92 et le fragment BamHI-SstI de 1,5 kb du plasmide suicide pVRC1000. Une vingtaine de recombinants a été repiquée sur milieu R2YE contenant 400 µg/ml de thiostrepton et les spores de ces recombinants ont été isolées par réétalement et croissance sur milieu R2YE contenant 400 µg/ml de thiostrepton.

Afin de vérifier la position de l'intégration du plasmide pVRC1000, différents Southerns de l'ADN total de 7 clones recombinants, purifiés comme décrit ci-dessus, ont été réalisés et hybridés avec le vecteur pDH5 et le fragment BamHI-SstI de 1,5 kb contenu dans PVRC1000 utilisés successivement comme sondes après marquage avec du [α-³²P]dCTP comme décrit dans l'exemple 9.1. Les résultats d'hybridation montrent l'apparition, dans le génome des 7 clones recombinants, d'une bande EcoRI de 13,8 kb et d'une bande BglII de 17 kb environ hybridant avec les 2 sondes ainsi que d'une bande EcoRI de 3,7 kb hybridant avec la sonde BamHI-SstI de 1,5 kb. Un de ces mutants a été nommé SP92::pVRC1000 et correspond bien à l'intégration par simple recombinaison homologue du plasmide pVRC1000 dans le gène snaD.

### 9.5.3. Production de pristinamycines par le mutant SP92::pVRC1000.

Cet exemple illustre comment il est déterminé que le mutant de S. pristinaespiralis SP92 disrupté dans le gène snaD par l'intégration du plasmide pVRC1000, ne produit plus de Pristinamycines II mais seulement des Pristinamycines I. Le mutant SP92::pVRC1000 ainsi que la souche témoin SP92 ont été cultivées en milieu de production liquide et leurs productions de Pristinamycines II et I dosées comme décrit dans l'exemple 9.1.

Les résultats ont montré que, dans les conditions de fermentation réalisées et pour les trois temps testés, le mutant SP92::pVRC1000 produit 0 mg/l de Pristinamycines II et une quantité de Pristinamycines I équivalente à celle du témoin SP92. Ce mutant est donc bien bloqué dans une étape de biosynthèse des Pristinamycines II, ce qui montre que le gène snaD code pour une enzyme impliquée dans la biosynthèse des Pristinamycines II, et très probablement pour une peptide synthase.

Cet exemple montre, comme dans l'exemple précédent, qu'il est possible, à partir de gènes de biosynthèse clonés, de construire des souches mutées dans les étapes de biosynthèse des Pristinamycines et en particulier des Pristinamycines II. Cet exemple montre également qu'il est possible par cette approche de produire des souches de S. pristinaespiralis produisant spécifiquement des Pristinamycines I et par extension des souches produisant spécifiquement des Pristinamycines II. Cette même approche pourrait également être utilisée pour les autres souches d'actinomycètes producteurs de streptogramines.

### EXEMPLE 10 : Complémentation d'un mutant non producteur de la souche SP92.

Cet exemple montre comment il est possible d'exprimer des gènes de biosynthèse des Pristinamycines. Cette expression a été plus particulièrement réalisée pour les gènes snaA et snaB portés par le cosmide pIBV1 dans une souche mutante dérivée de SP92 : SP120. Ce mutant ne produit pas de Pristinamycine IIA. Il accumule le dernier intermédiaire de la voie de biosynthèse de la Pristinamycine II : Pristinamycine IIB.

### 10.1. Clonage des gènes snaA et snaB dans le vecteur navette pIJ903.

Cet exemple illustre comment un sous-fragment du cosmide pIBV1, contenant les gènes snaA et snaB, a été cloné dans un vecteur capable de se répliquer à la fois chez E. coli et chez Streptomyces.

Le vecteur pIJ903 (Lydiate D. J. et al., 1985) est un vecteur navette à faible nombre de copies (1 à 3 par cellule), capable de se répliquer à la fois chez E.coli grâce à son origine de réplication de pBR322 et chez Streptomyces grâce à son origine de réplication de SCP2*. Le gène de résistance à l'ampicilline permet la sélection dans E. coli et le gène de résistance au thiostrepton permet la sélection dans Streptomyces.

Le cosmide pIBV1 a été digéré par l'enzyme de restriction SstI. Un large fragment d'ADN de 7,6 kb portant les gènes snaA et snaB a été isolé par électrophorèse sur gel d'agarose à 0.8 % et électroélué. 500 ng de ce fragment ont été ligués avec 100 ng du vecteur pUC1813 (Kay et McPherson, 1987) linéarisé par SstI. Après transformation de la souche E.coli DH5α (supE44 ΔlacU169 (f80lacZΔM15) hsdR17 recA1 endA1 gyrA96 thi-1 relA1) et sélection des transformants sur milieu LB solide contenant 150 µg/ml d'ampicilline et 20 µg/ml de X-gal, un clone portant le fragment de 7,6 kb a été isolé. Le plasmide a été nommé pVRC506. Une préparation de ce plasmide recombinant a été réalisée comme décrit dans l'exemple 2.1.

Le clonage dans le vecteur pIJ903 a été réalisé au site HindIII. Le plasmide pVRC506 a été coupé par HindIII et le fragment de 7,6 kb portant les gènes snaA et snaB a été isolé par électrophorèse sur gel d'agarose à 0.8 % et électroélué. 500 ng de ce fragment ont été ligués avec 500 ng du vecteur pIJ903 linéarisés par HindIII. Après transformation de la souche E. coli DH5α et sélection des transformants sur LB solide contenant 150 µg/ml d'ampicilline, un clone portant le fragment de 7,6 kb a été isolé. Le plasmide a été nommé pVRC507. Une préparation de ce plasmide recombinant a été réalisé comme décrit dans l'exemple 2.1. Sa cartographie est présentée figure 30.

### 10.2. Expression des gène snaA et snaB dans le mutant SP120.

Cet exemple illustre comment il est possible de produire les protéines SnaA et SnaB chez S. pristinaespiralis SP92, par introduction dans cette souche, d'un plasmide portant les gènes de structure correspondants. L'expression des gènes snaA et snaB a été réalisée après transformation de la souche mutante SP120 avec 500 ng de plasmide pVRC507. La transformation des protoplastes de SP120 et la sélection des transformants au thiostrepton a été réalisée comme décrit dans l'exemple 9.1.2.

De nombreux transformants ont été ainsi obtenus et 3 d'entre eux ont été choisis pour les tests de production en milieu liquide. La souche SP120 portant le plasmide pIJ903 a été choisie comme témoin. Les fermentations ainsi que l'extraction des produits de biosynthèse ont été réalisés comme décrit en l'exemple 9.1.3.

Les résultats ont montré que dans les conditions de fermentation réalisées, alors que le témoin (SP120 portant le plasmide pIJ903) a produit à 24, 28 et 32 heures de fermentation, 100 % de P IIB et 0 % de P IIA, les 3 clones de la souche SP120 transformés par le plasmide pVRC507, ont produit pour ces mêmes temps, environ 85 à 80 % de Pristinamycine IIB et 15 à 20 % de Pristinamycine IIA, dont la somme est équivalente en quantité à la production de Pristinamycine IIB de la souche témoin (SP120 portant le plasmide pIJ903). Les clones portant pVRC507 ont bien été partiellement complémentés pour l'étape de biosynthèse des Pristinamycines II correspondant à l'oxydation de la liaison 2-3 de la D-proline de l'intermédiaire Pristinamycine IIB. Ceci a été confirmé par dosage enzymatique de l'activité Pristinamycine IIA synthase, comme décrit dans l'exemple 5.1.1.A., pour les souches SP120 portant pVRC507 et SP120 portant pIJ903. Alors que la souche témoin SP120 portant pIJ903, ne présente aucune activité Pristinamycine IIA synthase, la souche SP120 portant pVRC507, présente une activité PIIA synthase.

Cet exemple montre qu'il est possible d'exprimer des gènes de biosynthèse des Streptogramines. Cette expression a été étudiée plus particulièrement pour les gènes codant pour la Pristinamycine IIA synthase mais les autres gènes de biosynthèse des Pristinamycines II et des Pristinamycines I et ainsi que ceux impliqués dans la biosynthèse des composants des différentes Streptogramines peuvent être ainsi exprimés. Cette expression peut être réalisée dans des souches mutantes comme c'est le cas dans l'exemple 10, mais également dans des souches productrices afin d'augmenter les niveaux de production des Streptogramines. L'expression peut être modifiée par clonage des gènes dans un vecteur à nombre de copies différent (faible ou élévé) ou dans un vecteur intégratif, par dérégulation de ces gènes, par clonage de ces gènes sous un promoteur homologue ou hétérologue (promoteur fort ou régulé spécifiquement). L'expression des différents gènes de biosynthèse des Streptogramines peut également être réalisée dans des souches hétérologues à partir de vecteurs d'expression adaptés, afin de produire des antibiotiques hybrides.

### EXEMPLE 11 : Expression du gène papM de S. pristinaespiralis dans E. coli

Cet exemple illlustre comment il est possible d'exprimer un gène de S. pristinaespiralis dans E. coli de manière à pouvoir identifier, purifier et étudier la protéine codée par ce gène.

### 11.1. Construction du plasmide pVRC706.

L'expression du gène papM dans E. coli est obtenue en plaçant ce gène en aval du promoteur et du site de fixation des ribosomes du gène lacZ de E. coli. Le fragment MluI-StuI de 1,7 kb a été isolé du plasmide pVRC409 décrit dans l'exemple 7.8. puis cloné dans le plasmide pMTL23 (Chambers et al, 1988) coupé au site BamHI rempli ultérieurement à l'aide de l'enzyme Klenow (Maniatis et al, 1989) et au site MluI pour donner le plasmide pVRC 706 représenté sur la figure 31. Le clonage au site MluI permet d'obtenir une fusion en phase entre les 32 premiers acides aminés de la β-galactosidase codée par le gène lacZ du plasmide pMTL23 et les onze derniers acides aminés du gène situé juste en amont de papM. ce qui permet de préserver le couplage traductionnel qui semble exister entre le gène papM et ce gène amont au vu de la séquence nucléotidique donnée dans l'exemple 7.8. Ainsi l'expression du gène hybride entre lacZ et le gène amont de papM et celle du gène papM est sous le contrôle des signaux d'expression du gène lacZ.

### 11.2. Expression dans la souche E. coli DH5α du produit du gène papM.

Les plasmides pVRC706 et pMTL23 on été introduits par transformation dans la souche de E. coli DH5α et l'expression de leurs gènes étudiée dans les conditions où le promoteur du gène lacZ est induit comme cela est déjà décrit (Maniatis et al, 1989). Les souche de E. coli portant le plasmide pVRC706 ou le plasmide contrôle pMTL23 ont été cultivées dans 500 ml de milieu riche LB contenant 100 µg/ml d'ampicilline et 1 mM d'IPTG permettant l'induction du promoteur du gène lacZ. On prélève ces cultures lorsqu'elles ont atteint une densité optique à 600 nm voisine de 1 et on prépare les extraits protéiques comme il est décrit ci-dessous.

### 11.3. Dosage de l'activité du produit du gène papM exprimé dans E. coli.

L'activité correspondant à la protéine codée par le gène papM est dosée pour les deux extraits préparés à partir des cultures de E. coli portant le plasmide pVRC706 ou le plasmide pMTL23 exemple 5.7). Il a été montré que l'extrait préparé à partir de la souche E. coli::pVRC706 catalyse la méthylation de la p-aminophénylalanine en p-diméthylaminophénylalanine avec une activité de 235 unités/mg, alors que cette activité est absente dans les extraits de la souche contrôle E. coli::pMTL23 (voir exemple 5.7.1.C.). Ces résultats indiquent qu'il est possible d'exprimer le gène papM de S.pristinaespiralis dans E.coli et que la protéine correspondante est bien l'enzyme catalysant la méthylation de la p-aminophénylalanine en p-diméthylaminophénylalanine. Cet exemple montre qu'il est possible d'exprimer des gènes de biosynthèse des Streptogramines dans des souches hétérologues (telles que E.coli mais aussi dans d'autres microorganismes) à partir de vecteurs d'expression adaptés afin de produire des précurseurs d'antibiotiques ou même des antibiotiques naturels ou hybrides.

### EXEMPLE 12 : Mise en évidence de l'homologie de gènes impliqués dans la biosynthèse des streptogramines, chez différents Streptomyces.

Cet exemple illustre comment il est possible de mettre en évidence, par hybridation sur des ADN totaux, l'homologie existant entre différents gènes impliqués dans la biosynthèse des Streptogramines chez différentes souches de Streptomyces productrices de Streptogramines.

### 12.1. Extraction de l'ADN total de différents Streptomyces producteurs de Streptogramines.

Cet exemple illustre comment l'ADN des différentes souches productrices de Streptogramines a été purifié. Ces souches de Streptomyces ont été choisies parmis celles décrites dans le tableau 1 :
Streptomyces loïdensis
Streptomyces olivaceus
Streptomyces ostréogriseus
Streptomyces virginiae

Une souche non productrice de streptogramines : Streptomyces hygroscopicus a été choisie comme témoin négatif.

Les extractions des différents ADN totaux ont été réalisées à partir de cultures en milieu YEME, comme décrit dans l'exemple 1.

### 12.2. Hybridation des ADNs totaux de souches productrices de Streptogramines par des fragments d'ADN contenant des gènes impliqués dans la biosynthèse des Pristinamycines et isolés de la souche S. pristinaespiralis SP92.

Cet exemple illustre comment il est possible à partir de gènes impliqués dans la biosynthèse des Pristinamycines et isolés de la souche SP92 comme décrit dans les exemples précédents, de mettre en évidence des gènes homologues par hybridation des ADN totaux d'autres souches productrices de Streptogramines. Les fragment d'ADN utilisés comme sonde ont été :
Le fragment XhoI-XhoI de 3,6 kb isolé à partir du pVRC1106 décrit dans l'exemple 6.5. et dont la carte de restriction est présentée figure 18. Ce fragment contient une partie du gène codant pour la Pristinamycine I synthase IL
Le Fragment BamHI-BamHI de 6 kb isolé à partir du plasmide pXL2045, décrit dans l'exemple 6.3. et dont la carte est présentée dans la figure 16. Ce fragment contient les gènes de structure des deux sous-unités de la PIIA synthase.

Les ADN totaux des quatre souches productrices de Streptogramines, de la souche S. hygroscopicus ainsi que de la souche SP92 ont été digérés par les enzymes de restriction BamHI et XhoI. Les fragments d'ADN ainsi obtenus ont été séparés sur gel d'agarose 0.7 % et l'ADN a été transféré sur membrane de nylon comme décrit par Maniatis et al. (1989). Le marquage des fragments XhoI-XhoI de 3,6 kb et BamHI-BamHI de 6 kb a été réalisé par marquage par random priming, comme décrit dans l'exemple 9.1.2. Les hybridations des membranes ont été réalisées en présence de 50 % de formamide à 42°C, comme décrit dans Maniatis et al. (1989). Les lavages des membranes après hybridation, ont été réalisés à 50 et 60°C dans une solution contenant du SSC (Maniatis et al. 1989) dilué 10 fois et du SDS 0.1 %.

Ces hybridations mettent en évidence les résultats suivants :
La souche S. hygroscopicus ne présente aucune hybridation avec les deux sondes utilisées.
Les ADN totaux (digérés par XhoI et BamHI) des souches S. ostreogriseus, S. olivaceus, S. loïdensis et S. virginiae présentent tous des signaux d'hybridation d'intensité comparable à ceux observés sur l'ADN total de la souche SP92 avec les deux sondes utilisées.

Cet exemple montre que différentes souches de Streptomyces produisant des Streptogramines, comportent des gènes hybridant avec des gènes isolés chez S. pristinaespiralis SP92 et impliqués dans la biosynthèse des Streptogramines, comme décrit dans les exemples précédents. Ces hybridations mettent ainsi en évidence l'homologie existant entre les gènes impliqués dans la biosynthèse des Streptogramines de la souche SP92 et ceux impliqués dans la biosynthèse des Streptogramines des autres souches productrices de Streptogramines.

Cet exemple montre donc qu'il est possible à partir des gènes isolés de SP92 et impliqués dans la biosynthèse des Streptogramines d'isoler par hybridation et clonage, les gènes homologues présents chez les autres souches productrices de Streptogramines.

### EXEMPLE 13 : Etude de la liaison physique des différents gènes de S. pristinaespiralis SP92 impliqués dans la biosynthèse des Pristinamycines I et des pristinamycines II.

Cet exemple illustre comment il est possible d'étudier la liaison physique des gènes de S. pristinaespiralis SP92 impliqués dans la biosynthèse des Pristinamycines I et II. Cette étude a été menée dans le but de montrer que tous ces gènes sont groupés sur le chromosome en un cluster et qu'il est donc possible en marchant sur le chromosome à partir des gènes déjà identifiés d'isoler d'autres gènes impliqués dans la biosynthèse des Pristinamycines **I** et II. Une telle démarche peut être envisagée pour les gènes impliqués dans la biosynthèse d'autres Streptogramines.

### 13.1. Enzymes de restriction utilisée pour l'électrophorèse à champs pulsé.

Le génome de S. pristinaespiralis SP92 est composé de 70 % à 75 % de nucléotides contenant les bases G et C. Pour couper son génome en un faible nombre de grands fragments, nous avons utilisé des enzymes qui reconnaissent une séquence riche en AT telles que AseI (AT/TAAT), SspI (AAT/ATT) mais aussi HindIII (A/AGCTT), EcoRI (G/AATTC), NdeI (CA/TATG) et ClaI (AT/CGAT).

### 13.2. Souches de S. pristinaespiralis utilisées pour l'électrophorèse à champs pulsé.

Nous avons utilisé l'ADN chromosomique de plusieurs souches pour étudier par électrophorèse à champ pulsé la liaison physique des gènes impliqués dans la biosynthèse des Pristinamycines I et des Pristinamycines II. Nous avons préparé des inserts comme il est décrit dans l'exemple 4.1. de l'ADN chromosomique de la souche S. pristinaespiralis SP92, mais aussi de l'ADN chromosomique des souches dérivées de SP92 dont la construction est décrite dans les exemples 9.1. et 9.4. Il s'agit de la souche SP92::pVRC505 dont le gène snaA a été disrupté par intégration du plasmide pVRC505 (exemple 9.1) et de la souche SP92::pVRC404 dont le gène snbA a été disrupté par intégration du plasmide pVRC404 (exemple 9.4). Ces deux dernières souches ont été incluses dans cette étude car elles ont permis de positionner précisement les gènes snaA et snbA sur la carte chromosomique en exploitant la présence de sites coupant rarement l'ADN chromosomique AseI, SspI, HindIII, EcoRI, NdeI et ClaI dans les plasmides pVRC505 et pVRC404.

### 13.3. Sondes d'ADN utilisées pour les hybridations des fragments isolés par électrophorèse à champs pulsé.

Nous avons utilisé différents fragments d'ADN pour obtenir des sondes marquées radioactivement comme il est décrit dans l'exemple 9.1. que nous avons hybridées aux fragments séparés par électrophorèse à champs pulsé après coupure enzymatique des inserts d'ADN chromosomique des trois souches présentées ci-dessus (exemple 4.2). Les sondes sont les suivantes : le fragment EcoRI-BamHI de 3,2 kb isolé du plasmide pVRC701 portant les gènes snaB et samS (cf exemple 9.2), le fragment BamH1-Sst1 de 1,5 kb isolé du plasmide pVRC 1000 portant une partie du gène snaD (cf exemple 6.8.), le fragment XhoI-HindIII de 1,1 kb isolé du plasmide pVRC402 portant le gène snbA (cf exemple 6.1), le fragment Pst1-Pst1 de 2,4 kb isolé du plasmide pVRC900 portant le gène papA (cf exemples 6.7 et 8.8) et le fragment XhoI-PstI de 1,5 kb isolé du plasmide pVRC509 portant le gène snaC (cf exemple 6.9.).

### 13.4. Localisation sur le chromosome des différents gènes impliqués dans la biosynthèse des Pristinamycines I et II et étude de leur liaison physique.

Les hybridations avec les différentes sondes décrites précédemment des ADN chromosomiques des souches S. pristinaespiralis SP92, SP92::pVRC404 et SP92::pVRC505 coupés par simples digestions et doubles digestions à l'aide des six enzymes mentionnées ci-dessus ont conduit à la cartographie générale représentée sur la figure 32 : la position de sites importants a été indiquée, ainsi que la position et le sens de transcription des gènes impliqués dans la biosynthèse des pristinamycines PI et PII. On a ainsi pu calculer la distance séparant les 3 régions chromosomiques contenant les gènes identifiés : celle des gènes snbA, snbR, papA et papM (cosmide pIBV2, exemple 5.2) celle des gènes snaA, snaB, samS, snaD snbC, snbD, snbE (cosmides pIBV1 et 3, exemple 5.1), et enfin celle du gène snaC (cosmide pIBV4, exemple 5.6). Par exemple, la distance entre les gènes snaA et snbA a été évaluée à environ 160-170 kb. Ceci montre que les gènes déjà identifiés sont tous contenus dans une région couvrant seulement 200 kb du chromosome de la souche S. pristinaespiralis soit moins de 3 % de la longueur totale du génome que nous avons pu estimer à 7500 kb par la technique d'électrophorèse à champs pulsé.

Ces résultats montrent que les gènes impliqués dans la biosynthèse des Pristinamycines I et II sont groupés sur le chromosome en un cluster et qu'il est donc possible en marchant sur le chromosome à partir des gènes déjà identifiés d'isoler d'autres gènes impliqués dans la biosynthèse des Pristinamycines I et des Pristinamycines II. Plus généralement, il est possible en marchant sur le chromosome, à partir de tout gène impliqué dans la biosynthèse des Streptogramines, d'identifier les autres gènes impliqués dans cette biosynthèse.

**TABLEAU 1**

| **MICROORGANISMES** | **ANTIBIOTIQUES** |
|---|---|
| **CHAMPIGNONS** | |
| Micromonospora sp. | Vernamycines |
| **STREPTOMYCES** | |
| S. alborectus | Virginiamycines |
| S. conganensis (ATCC13528) | F1370 A, B |
| S. diastaticus | Plauracines, Streptogramines |
| S. graminofasciens | Streptogramines |
| S. griseus (NRRL2426) | Viridogriséine (Etamycine) |
| S. griseoviridus | Griseoviridine |
| S. griseoviridus (FERMP3562) | Néoviridogriséines |
| S. lavendulae | Etamycines |
| S. loïdensis (ATCC11415) | Vemamycines |
| S. mitakaensis (ATCC15297) | Mikamycines |
| S. olivaceus (ATCC12019) | Synergistines (PA114 A, B) |
| S. ostréogriseus (ATCC27455) | Ostréogrycines |
| S. pristinaespiralis (ATCC25486) | Pristinamycines |
| S. virginiae (ATCC13161) | Virginiamycines (Staphylomycines) |

| **ACTINOMYCETES** | |
|---|---|
| A. auranticolor(ATCC31011) | Plauracines |
| A. azureus (ATCC31157) | Plauracines |
| A. daghestanicus | Etamycine |
| A. philppinensis | A-2315 A, B, C |
| Actinoplanes sp. (ATCC33002) | A15104 |
| Actinoplanes sp. | A17002 A, B, C, F |
| Actinomadura falva | Madumycines |

### Abréviations utilisées :

- ADN :: acide déoxyribonucléique
- AMP :: adénosine 5'-monophosphate
- ATP :: adénosine 5'-triphosphate
- BET :: bromure d'éthidium
- bis-tris :: (bis[2-hydroxyethyl]imino-tris[hydroxymethyl]methane
- bis-tris propane :: (1,3-bis[tris(hydroxymethyl)-methylamino]propane)
- BSA :: bovine sérum albumine
- CLHP :: chromatographie liquide haute performance
- DO :: Densité optique
- DTE :: dithioerythritol
- DTT :: dithiothréitol
- E64 :: trans-epoxysuccinyl-L-leucylamido-(4-guanidino)butane
- EDTA :: acide éthylènediaminetétraacétique
- EGTA :: acide éthylèneglycol bis (β-aminoéthyl) tétraacétique
- FMN :: flavine mononucleotide
- FMNH2 :: flavine mononucleotide reduite
- Hepes :: (N-[2-hydroxyethyl]piperazine-N'-[2-ethanesulfonic acid])
- IPTG :: isopropyl β-D-thiogalactopyranoside
- kDa :: kilodalton
- kb :: kilobase
- LB :: Luria broth (milieu de croissance riche pour E. coli)
- NAD :: nicotinamide dinucleotide
- NADH :: nicotinamide dinucleotide reduit
- PAGE :: electrophorese sur gel polyacrylamide
- pb :: paire de base
- PMSF :: phenylmethylsulfonyl fluoride
- PPi :: pyrophosphate
- rpm :: révolution/min
- S.A.:: sulfate d'ammonium
- SAM :: S-adénosylméthionine
- SDS :: sodium dodecyl sulfate
- STI :: inhibiteur de trypsine du soja
- TE :: tampon 10 mM Tris-HCl, 1 mM EDTA, pH 7,5
- Tris :: amino-2-hydroxylméthyl-2 propanediol-1,3
- U.V. :: rayons ultra-violets
- Xgal :: 5-bromo-4-chloro-3-indoyl-b-D-galactoside
- YEME :: yeast extract-malt extract medium (milieu de croissance riche pour Streptomyces)
- PEG :: Polyéthylène glycol
- LMP :: Low melting point
- PM :: Poids moléculaire

### Bibliographie :

- Anzai H., Murakami T., Imai A., Satoh A., Nagaoka K. et Thompson C. J. (1987) *J. Bacteriol.,* **169 :** 3482-3488.
- Bancroft I. et WolK C. P. (1989) *J. Bacteriol.,* **171 :** 5949-5954.
- Bibb M. J., Findlay P. R. et Johnson M. W. (1984) *Gene,* **30 :** 157-166.
- Birnboim H. C. et Doly J. (1979) *Nucleic Acids Res.,* **7 :** 1513-1523.
- Blattner F. R., Williams B. G., Blechl A. E., Denniston-Thompson K., Faber H. E., Furlong L. A., Grunwald D. J., Kiefer D. O., Moore D.D., Schumm J.W., Sheldon E. L. et Smithies O. (1977) *Science,* **196 :** 161-169.
- Bolivar F., Rodriguez R. L., Greene P. J., Betlach M. C., Heynecker H. L., Boyer H. W., Crosa J. H. et Falkow S. (1977) *Gene,* **2** ***:*** 95-113.
- Boyer H. W. et Roulland-Dussoix D. (1969) *J. Mol. Biol.,* **41 :** 459.
- Chater K. F. (1990) *Bio*/*Technology,* **8 :** 115-121.
- Cocito C. G. (1979) *Microbiol. Rev.,* **43 :** 145-198.
- Cocito C. G. (1983) *In Antibiotics,* **6 :** (Ed. F. E. Hahn), 296-332.
- Dessen P. C., Fondrat C., Valencien C. et Mugnier C. (1990) *Comp. Appl. in Biosciences,* **6 :** 355-356.
- Di Giambattista M., Chinali G. et Cocito C. G. (1989) *J. Antim. Chemother.,* **24 :** 485-507.
- Fernandez-Moreno M. A., Caballero J. L., Hopwood D. A. et Malpartida F. (1991) *Cell,* **66 :** 769-780.
- Gibson T. J. (1984) *Ph.D. thesis,* Cambridge University, England.
- Hallam S. E., Malpartida F. et Hopwood D. A. (1988) *Gene,* **74:** 305-320.
- Hames B. D. et Higgins S. J. (1985) *IRL Press Ltd., Oxford, U. K.,*
- Hanahan D. (1983) *J. Mol. Biol.,* **166:** 557
- Hillemann D., Pülher A. et Wohlleben W. (1991) *Nucl. Acids Res.,* **19:** 727-731.
- Hohn B. et Collins J. F. (1980) *Gene,* **11 :** 291-298.
- Hook J. D. et Vining L. C. (1973) *J.C.S. Chem. Comm.,* 185-186.
- Hopwood D. A., Bibb M. J., Chater K. F., Kieser T., Bruton C. J., Kieser H. M., Lydiate D. J., Smith C. P., Ward J. M. et Scrempf H. (1985) *A laboratory manual.,* The John Innes Fondation, Norwich, England.
- Hopwood D. A., Bibb M. J., Chater K. F., Janssen G. R., Malpartida F. et Smith C. (1986b) *In Regulation ofgene expression* - *25 years on (ed. I; A Booth C; F. Higgins),* 251-276.
- Hopwood D. A., Malpartida F., Kieser H. M., Ikeda H., Duncan J., Fujii I., Rudd A. M., Floss H. G. et Omura S. (1985a) *Nature,* **314:** 642-644.
- Hopwood D. A., Malpartida F., Kieser H. M., Ikeda H. et Omura S. (1985b) *In Microbiology (ed S. Silver). American Society for Microbiology, Washington D. C.*, 409-413.
- Hopwood D. A., Malpartida F. et Chater K. F. (1986a) *In Regulation of secondary metabolite formation. (eds. H. Kleinkauf, H. von Hohren, H. Dornauer G. Nesemann),* 22-33.
- Hoshino T., Ikeda T., Tomizuka N. et Furukawa K. (1985) *Gene,* **37 :** 131-138.
- Hutchinson C. R., Borell C. W., Otten S. L., Stutzman-Engwall K. J. et Wang Y. (1989) *J. Med Chem.,* **32 :** 929-937.
- Ish-Horowitz D. et Burk J. F. (1981) *Nucleic Acids Res.,* **9 :** 2989-298.
- Kanehisa M. I. (1984) *Nucleic Acids Res.,* **12:** 203-215.
- Kay R. et McPherson J. (1987) *Nucleic Acids Res.,* 15 (6) : 2778.
- Khan S. A. et Novick R. (1983) *Plasmid,* **10 :** 251-259.
- Kingston D. G. I., Kolpak M. X, Lefevre W. et Borup-Grochtmann L B. (1983) *J. Am. Chem. Soc.,* **105 :** 5106-5110.
- Kyte J. et Doolittle R. (1982) *J. Biol. Mol.,* **157:** 105-135.
- Low B. (1968) *Proc. Nalt. Acad. Sci.,* **60 :** 160.
- Lydiate D. J., Malpartida F. et Hopwood D. A. (1985) *Gene,* **35 :** 223-235.
- Maniatis T., Fritsh E. F. et Sambrook J. (1989) Molecular cloning : a laboratory manual. *Cold Spring Harbor, N. Y.,*
- Meinkoth J. et Wahl G. (1984) *Anal. Biochem.,* **138 :** 267-284.
- Messing J., Crea R. et Seeburg P. H. (1981) *Nucleic Acids Res.,* **9 :** 309.
- Neal R. J. et Chater K. F. (1987) *Gene,* **58 :** 229-241.
- Ohnuki T., Imanaka T. et Aiba S. (1985) *J. Bacteriol.,* **164 :** 85-94.
- Sawadogo M. et Van Dyke M. W. (1991) *Nucl. Acids Res.,* **19 :** 674.
- Staad J. F., Elkins M. F. et Earhart C. F. (1989) *FEMS Microbial. Lett.,* **59 :** 15.
- Staden R. et McLachlan A. D. (1982) *Nucleic Acids Res.,* 1O : 141-156.
- Videau D. (1982) *Path. Biol.,* **30 :** 529-534.
- Chambers S. P., Prior S. E., Barstow D. A. et Minton N. P. (1988) Gène, **68:** 139.
- Gutierrez S., Diez B., Montenegro E. et Martin J.F. (1991) Journal of bacteriology. 173: 2354-2365.
- Hori K., Yantamoto Y., Minetoki T., Kurotsu T., Kanda M., Miura S., Okamura K., Kuruyama J. et Saito Y. (1989) J. Biochem., 106: 639-645.
- Horikawa S., Ishikawa M., Ozaka H. et Tsukada K. (1989) Eur. J. Biochem., 184: 497-501.
- Kaplan J., Merkel W. et Nichols B. (1985) J. Mol. Biol., 183: 327-340.
- Markham G.D., DeParasis J. et Gatmaitan J. (1984) J. Biol. Chem., 259: 14505-14507.
- Sharka B., Westlake D. W. S. et Vining L. C. (1970) Chem. Zvesti, 24: 66-72.
- Thomas D., Rothstein R., Rosenberg N. et Surdin-Kerjan Y. (1988) Mol. Cell. Biol., 8: 5132-5139.
- Turgay K. et al (1992) Molecular Microbiology, 6(4) : 546.
- Weckermann R., FYrbab R. et Marahiel M.A. (1988) Nucl. Acids Res., 16: 11841
- Yanisch-Perron C., Vieira J. et Messing J. (1985) Gene, 33: 103-119.
- Zimmer W., Aparicio C. et Elmerich C. (1991) Mol. Gen. Genet., 229: 41-51.
- Reed et al. J.Nat.Prod 49 (1986) 626
- Molinero et al., J.Nat.Prod 52 (1989) 99
- Reed et al. J.Org.Chem. 54 (1989) 1161
- Watanabe et al., Mol.Cell.Biochem. 44 (1982) 181
- Jablonski et al., Biochemistry 16 (1977) 2832
- Duane et al., Mol.Cell.Biochem. 6 (1975) 53.

## Revendications

1. Séquence nucléotidique codant pour un polypeptide impliqué dans la biosynthèse des Streptogramines **caractérisée en ce qu'**elle est choisie parmi :
(a) les gènes snaA (SEQ ID n° 2), snaB (SEQ ID n° 3), snaC (SEQ ID n° 7), snaD (SEQ ID n° 8), papA (SEQ ID n° 9), papM (SEQ ID n° 10), samS (SEQ ID n° 4), snbA (SEQ ID n° 5), snbC (SEQ ID n° 11 et 12), snbD (SEQ ID n° 13 et 14), snbE (SEQ ID n° 15 et 16) et snbR (SEQ ID n° 6),
(b) les séquences hybridant avec les gènes (a) et codant pour un polypeptide impliqué dans la biosynthèse des Streptogramines, et,
(c) les séquences dérivées des séquences (a) en raison de la dégénérescence du code génétique.

2. Séquence nucléotidique selon la revendication 1 **caractérisée en ce qu'**elle est choisie parmi les gènes snaA, snaB, snaC, snaD, papA, papM, samS, snbA, snbC, snbD, snbE et snbR.

3. ADN recombinant comprenant un gène de biosynthèse des Streptogramines selon la revendication 2.

4. Vecteur d'expression à réplication autonome et/ou intégratif **caractérisé en ce qu'**il comprend une séquence nucléotidique selon l'une des revendications 1 ou 2 ou un ADN recombinant selon la revendication 3.

5. Cellule recombinante contenant un vecteur d'expression selon la revendication 4.

6. Procédé de production d'un polypeptide impliqué dans la biosynthèse des Streptogramines **caractérisé en ce que** l'on cultive une cellule recombinante selon la revendication 5 et on récupère le polypeptide produit.

7. Utilisation d'une cellule recombinante selon la revendication 5 exprimant un polypeptide au moins impliqué dans la biosynthèse des Streptogramines, dans une réaction de bioconversion.

8. Utilisation d'une séquence nucléotidique selon l'une des revendications 1 à 2 ou d'un ADN recombinant selon la revendication 3 ou un vecteur selon la revendication 4 pour amplifier la production de Streptogramines.

9. Procédé de production de Streptogramines **caractérisé en ce que** :
- on introduit et/ou on amplifie dans une cellule productrice de Streptogramines ou potentiellement productrice de Streptogramines une ou plusieurs séquences et/ou vecteurs selon l'une des revendications 1 à 4,
- on cultive ladite cellule dans des conditions de production des Streptogramines, et,
- on récupère les Streptogramines produites.

10. Procédé selon la revendication 9 pour la production de Pristinamycines, Mikamycines ou Virginiamycines.

11. Procédé de préparation de cellules bloquées dans une étape de la voie de biosynthèse des Streptogramines **caractérisé en ce que** l'on effectue, sur une cellule productrice de Streptogramine, une mutagénèse sur au moins un gène de la voie de biosynthèse ledit gène étant choisi parmi les gènes snaA (SEQ ID n°2), snaD (SEQ ID n° 8), papA (SEQ ID n°9), samS (SEQ ID n°4), et snbA (SEQ ID n°5).

12. Procédé selon la revendication 11 **caractérisé en ce que** la mutagénèse est effectuée in vitro ou in situ, par suppression, substitution, délétion et/ou addition d'une ou plusieurs bases dans le gène considéré, ou par disruption génique.

13. Mutant d'un microorganisme producteur de Streptogramines **caractérisé en ce qu'**il possède une disruption au moins dans un gène impliqué dans la biosynthèse des Streptogramines ledit gène étant choisi parmi les gènes snaA (SEQ ID n°2), snaD (SEQ ID n° 8), papA (SEQ ID n°9), samS (SEQ ID n°4), et snbA (SEQ ID n°5).

14. Procédé de préparation d'un intermédiaire de biosynthèse des Streptogramines **caractérisé en ce que** :
- on prépare une cellule bloquée dans une étape de la voie de biosynthèse des Streptogramines selon la revendication 11,
- on cultive ladite cellule, et
- on récupère l'intermédiaire accumulé.

15. Procédé de préparation d'une molécule dérivée des Streptogramines **caractérisé en ce que** :
- on prépare une cellule bloquée dans une étape de la voie de biosynthèse des Streptogramines selon la revendication 11,
- on cultive ladite cellule, et,
- on modifie l'intermédiaire accumulé par cette cellule, éventuellement après séparation du milieu de culture.

16. Procédé de préparation de Streptogramines comprenant la culture d'une cellule selon la revendication 13 contenant en outre une séquence nucléotidique et/ou un ADN recombinant et/ou un vecteur d'expression selon l'une des revendications 1 à 4.

17. Utilisation d'une séquence et/ou d'un vecteur selon l'une des revendications 1 à 4 pour la préparation d'antibiotiques hybrides.

18. Polypeptide résultant de l'expression d'une séquence selon l'une des revendications 1 à 2.

19. Polypeptide choisi parmi les polypeptides SnaA (SEQ ID n° 2), SnaB (SEQ ID n° 3), SnaC (SEQ ID n° 7), SnaD (SEQ ID n° 8), PapA (SEQ ID n° 9), PapM (SEQ ID n° 10), SamS (SEQ ID n° 4), SnbA (SEQ 1D n° 5), SnbC (SEQ ID n° 11 et 12), SnbD (SEQ ID n° 13 et 14), SnbE (SEQ ID n° 15 et 16) et SnbR (SEQ ID n° 6).

## Patentansprüche

1. Nukleotidsequenz, die für ein an der Biosynthese der Streptogramine beteiligtes Polypeptid codiert, **dadurch gekennzeichnet, dass** sie ausgewählt ist aus:
(a) den Genen snaA (SEQ ID NO: 2), snaB (SEQ ID NO: 3), snaC (SEQ ID NO: 7), snaD (SEQ ID NO: 8), papA (SEQ ID NO: 9), papM (SEQ ID NO: 10), samS (SEQ ID NO: 4), snbA (SEQ ID: 5), snbC (SEQ ID NO: 11 und 12), snbD (SEQ ID NO: 13 und 14), snbE (SEQ ID NO: 15 und 16) und snbR (SEQ ID NO: 6),
b) den Sequenzen, die mit den Genen (a) hybridisieren und für ein an der Biosynthese der Streptogramine beteiligtes Peptid codieren, und
c) den Sequenzen, die von den Sequenzen (a) durch Degeneriertheit des genetischen Codes abgeleitet sind.

2. Nukleotidsequenz nach Anspruch 1, **dadurch gekennzeichnet, dass** sie aus den Genen snaA, snaB, snaC, snaD, papA, papM, samS, snbA, snbC, snbD, snbE und snbR ausgewählt ist.

3. Rekombinante DNA, die ein Gen der Biosynthese der Streptogramine nach Anspruch 2 umfasst.

4. Expressionsvektor zur autonomen und/oder integrativen Replikation, **dadurch gekennzeichnet, dass** er eine Nukleotidsequenz nach einem der Ansprüche 1 oder 2 oder eine rekombinante DNA nach Anspruch 3 umfasst.

5. Rekombinante Zelle, die einen Expressionsvektor nach Anspruch 4 enthält.

6. Verfahren zur Herstellung eines an der Biosynthese der Streptogramine beteiligten Peptids, **dadurch gekennzeichnet, dass** man eine rekombinante Zelle nach Anspruch 5 kultiviert und das Polypeptidprodukt gewinnt.

7. Verwendung einer rekombinanten Zelle nach Anspruch 5, die ein Polypeptid, das wenigstens an der Biosynthese der Streptogramine beteiligt ist, exprimiert, in einer Biokonversionsreaktion.

8. Verwendung einer Nukleotidsequenz nach einem der Ansprüche 1 bis 2 oder einer rekombinanten DNA gemäß Anspruch 3 oder eines Vektors gemäß Anspruch 4 zur Verstärkung der Produktion von Streptograminen.

9. Verfahren zur Herstellung von Streptograminen, **dadurch gekennzeichnet, dass**:
- man in eine Streptogramine produzierende Zelle oder eine potentiell Streptogramine produzierende Zelle eine Sequenz oder mehrere Sequenzen und/oder Vektoren gemäß den Ansprüchen 1 bis 4 einführt und/oder amplifiziert,
- man die Zelle unter den Produktionsbedingungen für Streptogramine kultiviert und
- man die produzierten Streptogramine isoliert.

10. Verfahren nach Anspruch 9 für die Produktion von Pristinamycinen, Mikamycinen oder Virginamycinen.

11. Verfahren zur Herstellung von Zellen, die in einer Stufe des Biosynthesewegs der Streptogramine blockiert sind, **dadurch gekennzeichnet, dass** man an einer Streptogramin produzierenden Zelle eine Mutagenese an wenigstens einem Gen des Biosynthesewegs durchführt, wobei das Gen unter den Genen snaA (SEQ ID NO: 2), snaD (SEQ ID NO: 8), papA (SEQ ID NO: 9), samS (SEQ ID NO: 4) und snbA (SEQ ID NO: 5) ausgewählt wird.

12. Verfahren nach Anspruch 11, **dadurch gekennzeichnet, dass** die Mutagenese in vitro oder in situ durch Suppression, Substitution, Deletion und/oder Addition einer Base oder mehrerer Basen in dem betrachteten Gen oder durch Gendurchtrennung durchgeführt wird.

13. Mutante eines Streptogramine produzierenden Mikroorganismus, **dadurch gekennzeichnet, dass** er eine Durchtrennung wenigstens in einem an der Biosynthese der Streptogramine beteiligten Gen besitzt, wobei das Gen unter den Genen snaA (SEQ ID NO: 2), snaD (SEQ ID NO: 8), papA (SEQ ID NO: 9), samS (SEQ ID NO: 4) und snbA (SEQ ID NO: 5) ausgewählt ist.

14. Verfahren zur Herstellung eines Zwischenproduktes der Biosynthese der Streptogramine, **dadurch gekennzeichnet, dass**:
- man eine Zelle, die in einer Stufe des Biosynthesewegs der Streptogramine blockiert ist, gemäß Anspruch 11 herstellt,
- man die Zelle kultiviert und
- man das akkumulierte Zwischenprodukt isoliert.

15. Verfahren zur Herstellung eines von Streptograminen abgeleiteten Moleküls, **dadurch gekennzeichnet, dass**:
- man eine Zelle, die in einer Stufe des Biosynthesewegs der Streptogramine blockiert ist, gemäß Anspruch 11 herstellt,
- man die Zelle kultiviert und
- man das durch diese Zelle akkumulierte Zwischenprodukt, gegebenenfalls nach Abtrennung des Kulturmediums, modifiziert.

16. Verfahren zur Herstellung von Streptograminen, umfassend die Kultur einer Zelle gemäß Anspruch 13, die außerdem eine Nukleotidsequenz und/oder eine rekombinante DNA und/oder einen Expressionsvektor nach einem der Ansprüche 1 bis 4 enthält.

17. Verwendung einer Sequenz und/oder eines Vektors nach einem der Ansprüche 1 bis 4 zur Herstellung von Antibiotikahybriden.

18. Polypeptid, das aus der Expression einer Sequenz nach einem der Ansprüche 1 bis 2 resultiert.

19. Polypeptid, ausgewählt aus den Polypeptiden SnaA (SEQ ID NO: 2), SnaB (SEQ ID NO: 3), SnaC (SEQ ID NO: 7), SnaD (SEQ ID NO: 8), PapA (SEQ ID NO : 9), PapM (SEQ ID NO: 10), SamS (SEQ ID NO: 4), SnbA (SEQ ID: 5), SnbC (SEQ ID NO: 11 und 12), SnbD (SEQ ID NO: 13 und 14), SnbE (SEQ ID NO: 15 und 16) und SnbR (SEQ ID NO: 6).

## Claims

1. Nucleotide sequence coding for a polypeptide involved in the biosynthesis of streptogramins, **characterized in that** it is chosen from:
(a) the snaA (SEQ ID no. 2), snaB (SEQ ID no. 3), snaC (SEQ ID no. 7), snaD (SED ID no. 8), papA (SEQ ID no. 9), papM (SEQ ID no. 10), samS (SEQ ID no. 4), snbA (SEQ ID no. 5), snbC (SEQ ID nos. 11 and 12), snbD (SEQ ID nos. 13 and 14), snbE (SEQ ID nos. 15 and 16) and snbR (SEQ ID no. 6) genes,
(b) the sequences which hybridize with the genes (a) and which code for a polypeptide involved in the biosynthesis of streptogramins, and
(c) the sequences derived from the sequences (a) owing to the degeneracy of the genetic code.

2. Nucleotide sequence according to Claim 1, **characterized in that** it is chosen from the snaA, snaB, snaC, snaD, papA, papM, samS, snbA, snbC, snbD, snbE and snbR genes.

3. Recombinant DNA comprising a gene for the biosynthesis of streptogramins according to Claim 2.

4. Autonomously replicating and/or integrative expression vector, **characterized in that** it comprises a nucleotide sequence according to either of Claims 1 and 2 or a recombinant DNA according to Claim 3.

5. Recombinant cell containing an expression vector according to Claim 4

6. Method for producing a polypeptide involved in the biosynthesis of streptogramins, **characterized in that** a recombinant cell according to Claim 5 is cultured and the polypeptide produced is recovered.

7. Use of a recombinant cell according to Claim 5, expressing at least one polypeptide involved in the biosynthesis of streptogramins, in a bioconversion reaction.

8. Use of a nucleotide sequence according to either of Claims 1 and 2 or a recombinant DNA according to Claim 3 or a vector according to Claim 4 for amplifying streptogramin production.

9. Method for producing streptogramins, **characterized in that**:
- one or more sequences and/or vectors according to one of Claims 1 to 4 is/are introduced and/or amplified in a cell producing streptogramins or which is potentially a producer of streptogramins,
- the said cell is cultured under conditions of streptogramin production, and
- the streptogramins produced are recovered.

10. Method according to Claim 9, for producing pristinamycins, mikamycins or virginiamycins.

11. Method for preparing cells blocked in a step of the pathway of biosynthesis of streptogramins, **characterized in that** a mutagenesis is performed on at least one gene of the biosynthesis pathway, on a cell producing streptogramin, the said gene being chosen from the snaA (SEQ ID no. 2), snaD (SED IQ no. 8), papa (SEQ ID no. 9), samS (SEQ ID no. 4) and snbA (SEQ ID no.5) genes.

12. Method according to Claim 11, **characterized in that** the mutagenesis is performed in vitro or in situ, by suppression, substitution, deletion and/or addition of one or more bases in the gene in question, or by gene disruption.

13. Mutant of a microorganism producing streptogramins, **characterized in that** it possesses at least one disruption in a gene involved in the biosynthesis of streptogramins, the said gene being chosen from the snaA (SEQ ID no. 2), snaD (SED IQ no. 8), papa (SEQ ID no. 9), samS (SEQ ID no. 4) and snbA (SEQ ID no.5) genes.

14. Method for preparing an intermediate of the biosynthesis of streptogramins, **characterized in that**:
- a cell blocked in a step of the pathway of biosynthesis of streptogramins is prepared according to Claim 11,
- the said cell is cultured, and
- the accumulated intermediate is recovered.

15. Method for preparing a molecule derived from streptogramins, **characterized in that**:
- a cell blocked in a step of the pathway of biosynthesis of streptogramins is prepared according to Claim 11,
- the said cell is cultured, and
- the intermediate accumulated by this cell is modified, where appropriate after separation from the culture medium.

16. Method for preparing streptogramins, comprising the culturing of a cell according to Claim 13 containing, in addition, a nucleotide sequence and/or a recombinant DNA and/or an expression vector according to one of Claims 1 to 4.

17. Use of a sequence and/or a vector according to one of Claims 1 to 4 for the preparation of hybrid antibiotics.

18. Polypeptide resulting from the expression of a sequence according to either of Claims 1 and 2.

19. Polypeptide chosen from the polypeptides SnaA (SEQ ID no. 2), SnaB (SEQ ID no. 3), SnaC (SEQ ID no. 7), SnaD (SEQ ID no. 8), PapA (SEQ ID no. 9), PapM (SEQ ID no. 10), SamS (SEQ ID no. 4), SnbA (SEQ ID no. 5), SnbC (SEQ ID nos. 11 and 12), SnbD (SEQ ID nos. 13 and 14), SnbE (SEQ ID nos. 15 and 16) and SnbR (SEQ ID no. 6).
